# EUROPEAN PATENT APPLICATION

(11) **EP 2 208 728 A1**
(43) Date of publication of application: **21.07.2010**
(21) Application number: 08831464.6
(22) Date of filing: 17.09.2008
(51) Int. Cl.: C07D 265/24, A61K 31/536, A61K 31/54, A61K 31/5415, A61K 31/547, A61K 31/55, A61P 3/04, A61P 3/06, A61P 3/10, A61P 9/10, A61P 43/00, C07D 279/06, C07D 279/08, C07D 417/04, C07D 417/12

(54) **HETEROCYCLIC DERIVATIVE HAVING INHIBITORY ACTIVITY ON TYPE-I 11 -HYDROXYSTEROID DEHYDROGENASE**

(30) Priority: 19.09.2007 JP 2007241976
(71) Applicant: Institute Of Medicinal Molecular Design, Inc., Tokyo 113-0033 (JP); Shionogi&Co., Ltd., Osaka-shi, Osaka 5410045 (JP)
(72) Inventor: ITAI, Akiko, Tokyo 113-0033 (JP); MUTO, Susumu, Tokyo 113-0033 (JP); TOKUYAMA, Ryuko, Tokyo 113-0033 (JP); FUKASAWA, Hiroshi, Tokyo 113-0033 (JP); YANASE, Takeshi, Tokyo 113-0033 (JP)
(74) Representative: HOFFMANN EITLE
(86) International application number: PCT/JP2008/066708
(87) International publication number: WO 2009/038064

(57) **Abstract**

Disclosed is a compound which is useful as an 11β-hydroxysteroid dehydrogenase type 1 inhibitor.

A compound represented by the formula: 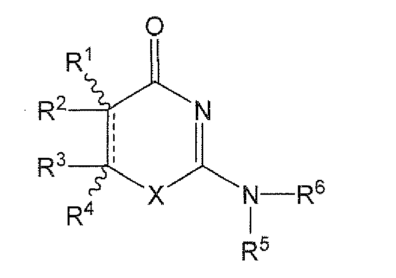 its pharmaceutically acceptable salt, or a solvate thereof, wherein X is O or S, a broken line and a wavy line represent the presence or the absence of a bond,
(i) when a broken line represents the presence of a bond, a wavy line represents the absence of a bond,
R² and R³ are each independently hydrogen, halogen, cyano, hydroxy, carboxy, optionally substituted alkyl, optionally substituted alkenyl, optionally substituted alkynyl or the like,
(ii) when a broken line represents the absence of a bond, a wavy line represents the presence of a bond,
R¹ and R⁴ are each independently hydrogen, halogen or the like,
R² and R³ are each independently hydrogen, halogen, cyano, hydroxy, carboxy, optionally substituted alkyl, optionally substituted alkenyl, optionally substituted alkynyl or the like, and
R⁵ and R⁶ are each independently hydrogen, optionally substituted alkyl, optionally substituted alkenyl, optionally substituted alkynyl or the like.

## Description

### Field of the Invention

The present invention relates to a pharmaceutically useful compound having inhibitory activity on 11β-hydroxysteroid dehydrogenase type I (hereinafter, referred to as 11β-HSD-1).

### Background art

11β-HSD-1 is an enzyme that converts 11β-dehydrosteroid, which is an inactive steroid, into an active steroid, and is considered to have great significance in biological basal metabolism (Non-patent document 1). Also, an 11β-HSD-1 knockout mouse has resistance to hyperglycemia induced by obesity, stress and the like (Non-patent document 2). Also in human, a similar phenomenon was observed when carbenoxolone which is an 11β-HSD-1 inhibitor was administered (Non-patent document 3).
These facts suggest the possibility of a selective inhibitor of this enzyme as a therapeutic agent in insulin independent diabetes and obesity (Non-patent document 4).
Patent document 1 discloses a benzothiazinone derivative useful as a fungicidal agent.
Patent document 2 discloses a benzothiazinone derivative useful as an anti-inflammatory agent, a central nervous system inhibitor, an analgesic agent, or a diuretic agent.
Patent document 3 discloses a benzothiazinone derivative useful as a bronchodilator.
Patent documents 4 and 5 disclose a benzoxazinone derivative useful as an antibacterial agent.
Patent documents 6 to 9 disclose a benzoxazinone derivative useful as an anti-atherosclerosis agent.
Patent document 10 discloses a benzoxazinone derivative useful as an analgesic agent.
Patent document 11 discloses a thiazinone derivative useful as a central nervous system inhibitor.
Patent documents 12 and 13 and Non-patent document 8 disclose a process for producing an oxazinone derivative.
Non-patent document 5 discloses a benzothiazinone derivative useful as an anti-cancer agent.
Non-patent document 6 discloses a benzothiazinone derivative useful as a tuberculosis therapeutic agent.
Non-patent document 7 discloses a thiazinone derivative useful as an antibacterial agent.

[Non-patent document 1] Clin. Endocrinol., 1996, vol.44, pp.493
[Non-patent document 2] Proc. Nat. Acad. Sci. USA, 1997, vol.94, pp.14924
[Non-patent document 3] J. Clin. Endocrinol. Matab., 1995, vol.80, pp.3155
[Non-patent document 4] Lancet, 1997, vol.349, pp.1210
[Non-patent document 5] Anticancer Research, 1999, vol.19(1A), pp.119
[Non-patent document 6] Rev. Chim., Acad. Rep. Populaire Roumaine, 1956, 1(No.1), pp.97
[Non-patent document 7] Collection of Czechoslovak Chemical Communications, 1998, vol.63, No.1, pp.94
[Non-patent document 8] Chemical & Pharmaceutical Bulletin, 1984, vol.32, No.1, pp.106
[Patent document 1] EP245902
[Patent document 2] US3470168
[Patent document 3] DE1807165
[Patent document 4] WO2002094797
[Patent document 5] WO2000051992
[Patent document 6] WO9119707
[Patent document 7] WO9006921
[Patent document 8] WO9002129
[Patent document 9] US4412071
[Patent document 10] FRM3318
[Patent document 11] US2585064
[Patent document 12] DD286171
[Patent document 13] J60021985

### Disclosure of invention

### Problems to be solved by the invention

The present invention provides a useful 11β-hydroxysteroid dehydrogenase type 1 inhibitor.

### Means to solve the problems

The present invention provides:
(1) A pharmaceutical composition having inhibitory activity on 11β-hydroxysteroid dehydrogenase type I comprising a compound represented by the formula (I): 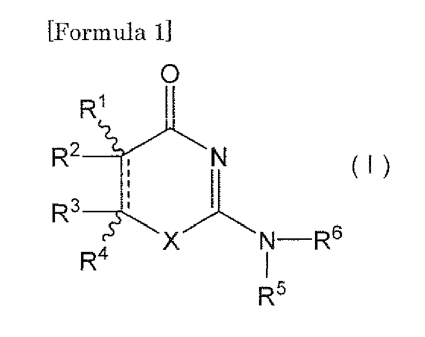 , its pharmaceutically acceptable salt, or a solvate thereof,
   wherein
   X is O or S,
   a broken line and a wavy line represent the presence or the absence of a bond,
   (i) when a broken line represents the presence of a bond, a wavy line represents the absence of a bond,
      R² and R³ are each independently hydrogen, halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R² and R³ may be taken together with the adjacent carbon atom to which they are attached to form an optionally substituted nonaromatic hydrocarbon ring or an optionally substituted aromatic hydrocarbon ring
   (ii) when a broken line represents the absence of a bond, a wavy line represents the presence of a bond,
      R¹ and R⁴ are each independently hydrogen, halogen, hydroxy, optionally substituted alkyloxy, optionally substituted alkyl, optionally substituted cycloalkyl or optionally substituted aryl,
      R² and R³ are each independently hydrogen, halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl, or optionally substituted arylsulfonyl, or
      R¹ and R², R² and R³ or R³ and R⁴ may be taken together with the adjacent carbon atom to which they are attached to form an optionally substituted nonaromatic hydrocarbon ring,
      R⁵ and R⁶ are each independently hydrogen, optionally substituted alkyl, optionally substituted alkenyl, optionally substituted alkynyl, optionally substituted aryl, optionally substituted arylalkyl, optionally substituted cycloalkyl, optionally substituted cycloalkylalkyl, optionally substituted cycloalkenyl, optionally substituted cycloalkenylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkylsulfonyl or optionally substituted arylsulfonyl, or R⁵ and R⁶ may be taken together with the adjacent nitrogen atom to which they are attached to form an optionally substituted ring.
(2) The pharmaceutical composition having inhibitory activity on 11β-hydroxysteroid dehydrogenase type I comprising the compound according to the above (1), its pharmaceutically acceptable salt, or a solvate thereof, wherein
   (i) a broken line represents the presence of a bond, a wavy line represents the absence of a bond, and R² and R³ are taken together with the adjacent carbon atom to which they are attached to form an optionally substituted nonaromatic hydrocarbon ring or an optionally substituted aromatic hydrocarbon ring, or
   (ii) a broken line represents the absence of a bond, a wavy line represents the presence of a bond, and R² and R³ are taken together with the adjacent carbon atom to which they are attached to form an optionally substituted nonaromatic hydrocarbon ring.
(3) The pharmaceutical composition having inhibitory activity on 11β-hydroxysteroid dehydrogenase type I comprising the compound according to the above (2), its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted nonaromatic hydrocarbon ring.
(4) The pharmaceutical composition having inhibitory activity on 11β-hydroxysteroid dehydrogenase type I comprising the compound according to the above (2), its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted aromatic hydrocarbon ring.
(5) The pharmaceutical composition having inhibitory activity on 11β-hydroxysteroid dehydrogenase type I comprising the compound according to the above (2), its pharmaceutically acceptable salt, or a solvate thereof, wherein a broken line represents the absence of a bond, a wavy line represents the presence of a bond, and R² and R³ are taken together with the adjacent carbon atom to which they are attached to form an optionally substituted nonaromatic hydrocarbon ring.
(6) The pharmaceutical composition having inhibitory activity on 11β-hydroxysteroid dehydrogenase type I comprising the compound according to any one of the above (2) to (5), its pharmaceutically acceptable salt, or a solvate thereof, wherein X is S.
(7) The pharmaceutical composition having inhibitory activity on 11β-hydroxysteroid dehydrogenase type I comprising the compound according to the above (I), its pharmaceutically acceptable salt, or a solvate thereof, wherein a broken line represents the absence of a bond, a wavy line represents the presence of a bond, R¹ and R² are each hydrogen, R³ and R⁴ are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R³ and R⁴ may be taken together with the adjacent carbon atom to which they are attached to form an optionally substituted nonaromatic hydrocarbon ring, or
   a broken line represents the absence of a bond, a wavy line represents the presence of a bond, R³ and R⁴ are each hydrogen, R¹ and R² are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R¹ and R² may be taken together with the adjacent carbon atom to which they are attached to form an optionally substituted nonaromatic hydrocarbon ring.
(8) The pharmaceutical composition having inhibitory activity on 11β-hydroxysteroid dehydrogenase type I comprising the compound according to the above (7), its pharmaceutically acceptable salt, or a solvate thereof, wherein a broken line represents the absence of a bond, a wavy line represents the presence of a bond, R¹ and R² are each hydrogen, R³ and R⁴ are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylakyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R³ and R⁴ may be taken together with the adjacent carbon atom to which they are attached to form an optionally substituted nonaromatic hydrocarbon ring.
(9) A compound represented by the formula (I): 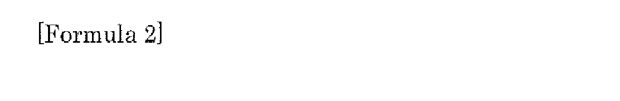 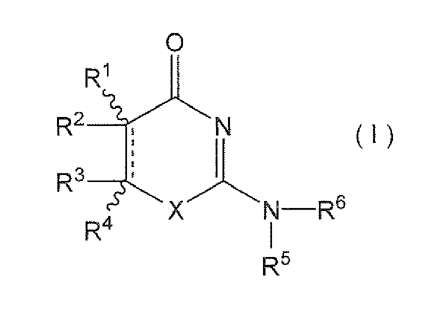 , its pharmaceutically acceptable salt, or a solvate thereof,
   wherein
   X is O or S,
   a broken line and a wavy line represent the presence or the absence of a bond,
   (i) when a broken line represents the presence of a bond, a wavy line represents the absence of a bond,
      R² and R³ are taken together with the adjacent carbon atom to which they are attached to form an optionally substituted nonaromatic hydrocarbon ring,
   (ii) when a broken line represents the absence of a bond, a wavy line represents the presence of a bond,
      R¹ and R⁴ are each independently hydrogen, halogen, hydroxy, optionally substituted alkyloxy or optionally substituted alkyl, R² and R³ are taken together with the adjacent carbon atom to which they are attached to form an optionally substituted nonaromatic hydrocarbon ring,
      R⁵ and R⁶ are each independently hydrogen, optionally substituted alkyl, optionally substituted alkenyl, optionally substituted alkynyl, optionally substituted aryl, optionally substituted arylalkyl, optionally substituted cycloalkyl, optionally substituted cycloalkylalkyl, optionally substituted cycloalkenyl, optionally substituted cycloalkenylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkylsulfonyl or optionally substituted arylsulfonyl, or R⁵ and R⁶ may be taken together with the adjacent nitrogen atom to which they are attached to form an optionally substituted ring,
   provided that, the following compounds are excluded: 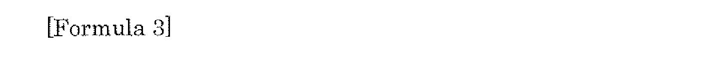 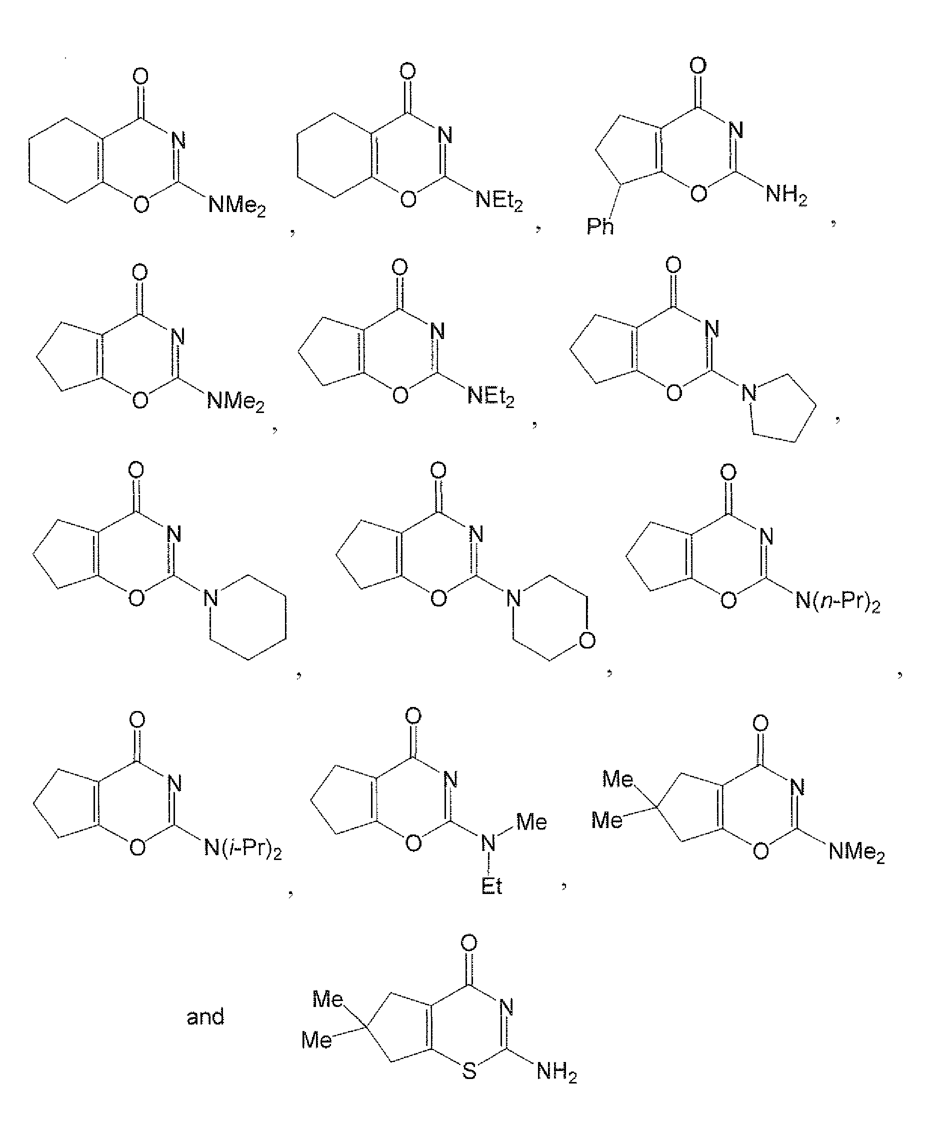
(10) The compound according to the above (9), its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted nonaromatic hydrocarbon ring.
(11) The compound according to the above (9), its pharmaceutically acceptable salt, or a solvate thereof, wherein a broken line represents the absence of a bond, a wavy line represents the presence of a bond, and R² and R³ are taken together with the adjacent carbon atom to which they are attached to form an optionally substituted nonaromatic hydrocarbon ring.
(12) The compound according to the above (9) or (11), its pharmaceutically acceptable salt, or a solvate thereof, wherein R¹ and R⁴ are each hydrogen.
(13) The compound according to any one of the above (9) to (12), its pharmaceutically acceptable salt, or a solvate thereof, wherein X is S.
(14) The compound according to any one of the above (9) to (13), its pharmaceutically acceptable salt, or a solvate thereof, wherein R⁵ and R⁶ are each independently optionally substituted alkyl, optionally substituted aryl, optionally substituted cycloalkyl, optionally substituted heterocycle, optionally substituted amino, or R⁵ and R⁶ may be taken together with the adjacent nitrogen atom to which they are attached to form an optionally substituted ring.
(15) A pharmaceutical composition comprising the compound according to any one of the above (9) to (14), its pharmaceutically acceptable salt, or a solvate thereof.
(16) The pharmaceutical composition according to any one of the above (1) to (8), or (15), for treatment and/or prevention of diabetes.
   Further, the present invention includes:
(17) A method for preventing or treating diabetes, comprising administering the compound according to any one of the above (1) to (16), its pharmaceutically acceptable salt, or a solvate thereof, and
(18) A use of the compound according to any one of the above (1) to (16), its pharmaceutically acceptable salt, or a solvate thereof for manufacturing a medicament of treatment and/or prevention of diabetes.

### Effect of the invention

Since the present compound has inhibitory activity on 11β-hydroxysteroid dehydrogenase type I, pharmaceutical compositions comprising the present compound are very useful as medicaments, especially, as medicaments for treatment and/or prevention of hyperlipidemia, diabetes, obesity, arteriosclerosis, atherosclerosis, hyperglycemia, and/or syndrome X. Moreover, the present compound selectively inhibits 11β-hydroxysteroid dehydrogenase type I, and is a compound having other utility as a medicament. Here, the utility as a medicament includes excellent metabolic stability, a weak drug-metabolizing enzyme induction, a weak inhibition of drug metabolizing enzyme that metabolizes other drug, a high oral absorption, a low clearance, a long half-life period enough to exhibit drug efficacy and so on.

### Best mode for carrying out the invention

In the following, meanings of terms used in the present specification will be explained. Each term has the same meaning when used alone or in combination with other term in this description.
"Halogen" includes fluorine, chlorine, bromine or iodine. Particularly, fluorine, chlorine and bromine are preferable.

"Alkyl" means a C1, to C10 straight or branched alkyl group, and example includes methyl, ethyl, n-propyl, isopropyl, n-butyl, isobutyl, see-butyl, tert-butyl, n-pentyl, isopentyl, neopentyl, n-hexyl, isohexyl, n-heptyl, n-octyl, n-nonyl, n-decyl or the like. Preferable is C1 to C6 or C1 to C4 alkyl, and example includes methyl, ethyl, n-propyl, isopropyl, n-butyl, isobutyl, sec-butyl, tert-butyl, n-pentyl, isopentyl, neopentyl, n-hexyl or isohexyl.

"Alkenyl" means C2 to C8 straight or branched alkenyl having one or more double bond(s) in the above "alkyl", and example includes vinyl, 1-propenyl, 2-propenyl, 1-butenyl, 2-butenyl, 3-butenyl, 1,3-butadienyl, 3-methyl-2-butenyl or the like.

"Alkynyl" means C2 to C8 straight or branched alkynyl having one or more triple bond(s) in the above "alkyl", and example includes ethynyl, propynyl, butynyl or the like.

"cycloalkyl" means a C3 to C15 cyclic saturated hydrocarbon group, and example includes cyclopropyl, cyclobutyl, cyclopentyl, cyclohexyl, cycloheptyl, cyclooctyl, bridged cyclic hydrocarbon group, spiro hydrocarbon group or the like. Preferable is cyclopropyl, cyclobutyl, cyclopentyl, cyclohexyl or bridged cyclic hydrocarbon group.

"Bridged cyclic hydrocarbon group" includes a group which is derived by excluding one hydrogen from a C5 to C8 aliphatic cycle which consists of two or more rings that share two or more atoms. Example includes bicyclo[2.1.0]pentyl, bicyclo[2.2.1]heptyl, bicyclo[2.2.2]octyl, bicyclo[3.2.1]octyl, tricyclo[2.2.1.0]heptyl or the like.

"Spiro hydrocarbon group" includes a group which is derived by excluding one hydrogen from a cycle which consists of two hydrocarbon rings that share one carbon atom. Example includes spiro[3.4]octyl or the like.

"Cycloalkenyl" means a C3 to C7 cyclic unsaturated aliphatic hydrocarbon group, and example includes cyclopropenyl, cyclobutenyl, cyclopentenyl, cyclohexenyl or cycloheptenyl. Preferable is cyclopropenyl, cyclobutenyl, cylopentenyl or cyclohexenyl. Cycloalkenyl includes a bridged cyclic hydrocarbon group and a spiro hydrocarbon group having an unsaturated bond in a ring.

"Aryl" means a monocyclic aromatic hydrocarbon group (e.g.: phenyl) and a polycyclic aromatic hydrocarbon group (e.g.: 1-naphthyl, 2-naphthyl, 1-anthryl, 2-anthryl, 9-anthryl, 1-phenanthryl, 2-phenanthryl, 3-phenanthryl, 4-phenanthryl or 9-phenanthryl). Preferable is phenyl or naphthyl (1-naphthyl or 2-naphthyl).

"Heteroaryl" means a monocyclic aromatic heterocyclic group or a fused aromatic heterocyclic group. The monocyclic aromatic heterocyclic group means a group derived from a 5- to 8-membered aromatic ring which may contain 1 to 4 oxygen, sulfur and/or nitrogen atom(s) in the ring, and may have a bond at a substitutable arbitrary position. The fused aromatic heterocyclic group means a group in which a 5- to 8-membered aromatic ring optionally containing 1 to 4 oxygen, sulfur and/or nitrogen atom(s) in the ring is fused with 1 to 4 of 5- to 8-membered aromatic carbocycle(s) or other 5- to 8-membered aromatic heterocycle(s), and which may have a bond at a substitutable arbitrary position.

Example of the "heteroaryl" includes furyl (e.g.: 2-furyl or 3-furyl), thienyl (e.g.: 2-thienyl or 8-thienyl), pyrrolyl (e.g.: 1-pyrrolyl, 2-pyrrolyl or 3-pyrrolyl), imidazolyl (e.g.: 1-imidazolyl, 2-imidazolyl or 4-imidazolyl), pyrazolyl (e.g.: 1-pyrazolyl, 3-pyrazolyl or 4-pyrazolyl), triazolyl (e.g.: 1,2,4-triazol-1-yl, 1,2,4-triazol-3-yl or 1,2,4-triazol-4-yl), tetrazolyl (e.g.: 1-tetrazolyl, 2-tetrazolyl or 5-tetrazolyl), oxazolyl (e.g.: 2-oxazolyl, 4-oxazolyl or 5-oxazolyl), isoxazolyl (e.g.: 3-isoxazolyl, 4-isoxazolyl or 5-isoxazolyl), thiazolyl (e.g.: 2-thiazolyl, 4-thiazolyl or 5-thiazolyl), thiadiazolyl, isothiazolyl (e.g.: 3-isothiazolyl, 4-isothiazolyl or 5-isothiazolyl), pyridyl (e.g.: 2-pyridyl, 3-pyridyl or 4-pyridyl), pyridazinyl (e.g.: 3-pyridazinyl or 4-pyridazinyl), pyrimidinyl (e.g.: 2- pyrimidinyl, 4- pyrimidinyl or 5-pyrimidinyl), furazanyl (e.g.: 3-furazanyl), pyrazinyl (e.g.: 2-pyrazinyl), oxadiazolyl (e.g.: 1,3,4-oxadiazol-2-yl), benzofuryl (e.g.: 2-benzo[b]furyl, 3-benzo[b]furyl, 4-benzo[b]furyl, 5-benzo[b]furyl, 6-benzo[b]furyl or 7-benzo[b]furyl), benzothienyl (e.g.: 2-benzo[b]thienyl, 3-benzo[b]thienyl, 4-benzol[b]thienyl, 5-benzo[b]thienyl, 6-benzo[b]thienyl or 7-benzo[b]thienyl), benzimidazolyl (e.g.: 1-benzimidazolyl, 2-benzimidazolyl, 4-benzimidazolyl or 5-benzimidazolyl), dibenzofuryl, benzoxazolyl, benzothiazolyl, quinoxalinyl (e.g.: 2-quinoxalinyl, 5-quinoxalinyl or 6-quinoxalinyl), cinnolinyl (e.g.: 3-cinnolinyl, 4-cinnolinyl, 5-cinnolinyl, 6-cinnolinyl, 7-cinnolinyl or 8-cinnolinyl), quinazolinyl (e.g.: 2-quinazolinyl, 4-quinazolinyl, 5-quinazolinyl, 6-quinazolinyl, 7-quinazolinyl or 8-quinazolinyl), quinolyl (e.g.: 2-quinolyl, 3-quinolyl, 4-quinolyl, 5-quinolyl, 6-quinolyl, 7-quinolyl or 8-quinolyl), phthalazinyl (e.g.: 1-phthalazinyl, 5-phthalazinyl or 6-phthalazinyl), isoquinolyl (e.g.: 1-isoquinolyl, 3-isoquinolyl, 4-isoquinolyl, 5-isoquinolyl, 6-isoquinolyl, 7-isoquinolyl or 8-isoquinolyl), puryl, pteridinyl (e.g.: 2-pteridinyl, 4-pteridinyl, 6-pteridinyl or 7-pteridinyl), carbazolyl, phenanthridinyl, acridinyl (e.g.: 1-acridinyl, 2-acridinyl, 3-acridinyl, 4-acridinyl or 9-acridinyl), indolyl (e.g.: 1-indolyl, 2-indolyl, 3-indolyl, 4-indolyl, 5-indolyl, 6-indolyl or 7-indolyl), isoindolyl, phenazinyl (e.g.: 1-phenazinyl or 2-phenazinyl), phenothiazinyl (e.g.: 1-phenothiazinyl, 2-phenothiazinyl, 3-phenothiazinyl or 4-phenothiazinyl) or the like.

"Heterocycle" means a nonaromatic heterocyclic group which may contain 1 to 4 oxygen, sulfur and/or nitrogen atom(s) in the ring, and may have a bond at a substitutable arbitrary position. Moreover, the nonaromatic heterocyclic group can be bridged with a C1 to C4 alkyl chain, or can be fused with cycloalkane (5- to 6-membered ring is preferable) or benzene ring. Heterocycle can be saturated or unsaturated as long as it is nonaromatic. Preferable is a 5- to 8-membered ring. Example includes 1-pyrrolinyl, 2-pyrrolinyl, 3-pyrrolinyl, 1-pyrrolidinyl, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidinyl, 3-piperidinyl, 4-piperidinyl, 1-piperazinyl, 2-piperazinyl, 2-morpholinyl, 3-morpholinyl, morpholino, tetrahydropyranyl or the like.

"Acyl" means formyl, optionally substituted alkylcarbonyl, optionally substituted alkenylcarbonyl, optionally substituted cycloalkylcarbonyl, optionally substituted cycloalkenylcarbonyl, optionally substituted arylcarbonyl, optionally substituted heteroarylcarbonyl, or optionally substituted heterocyclecarbonyl.

An alkyl part of the "optionally substituted alkoxy", the "optionally substituted alkoxycarbonyl", the "optionally substituted alkylthio", the "optionally substituted alkylsulfonyl", the "optionally substituted arylalkyl", the "optionally substituted cycloalkylalkyl", the "optionally substituted cycloalkenylalkyl", the "optionally substituted heteroarylalkyl", and the "optionally substituted heterocyclealkyl" means the above "alkyl".

An aryl part of the "optionally substituted arylthio" and the "optionally substituted arylsulfonyl" means the above "aryl".

As an optionally substituted nonaromatic hydrocarbon ring in which a broken line represents the presence of a bond and a wavy line represents the absence of a bond, and which is formed by R² and R³ together with the adjacent carbon atom to which they are attached, the following rings are exemplified. The ring is not limited to the following rings.

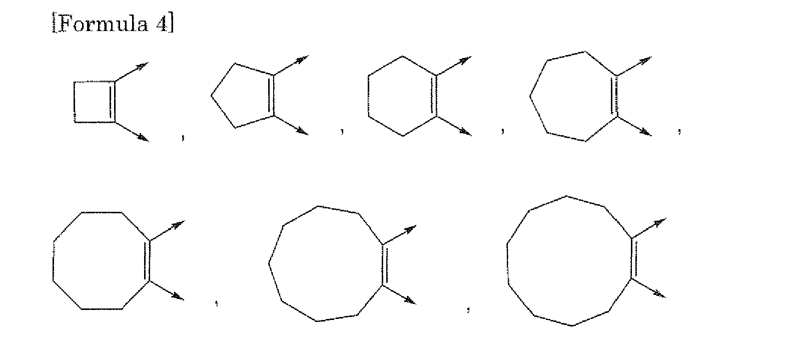

The ring may have a bridged structure as exemplified below. 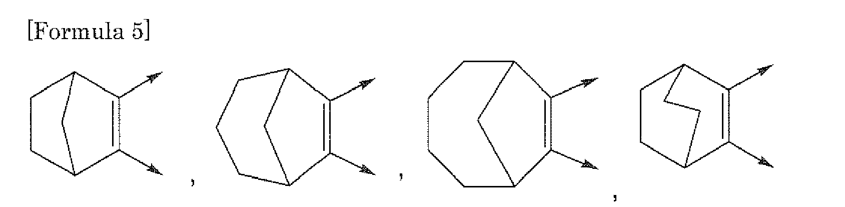 Furthermore, as exemplified below, the ring may be fused with an aromatic hydrocarbon ring, a nonaromatic hydrocarbon ring, an aromatic heterocycle or a nonaromatic heterocycle. 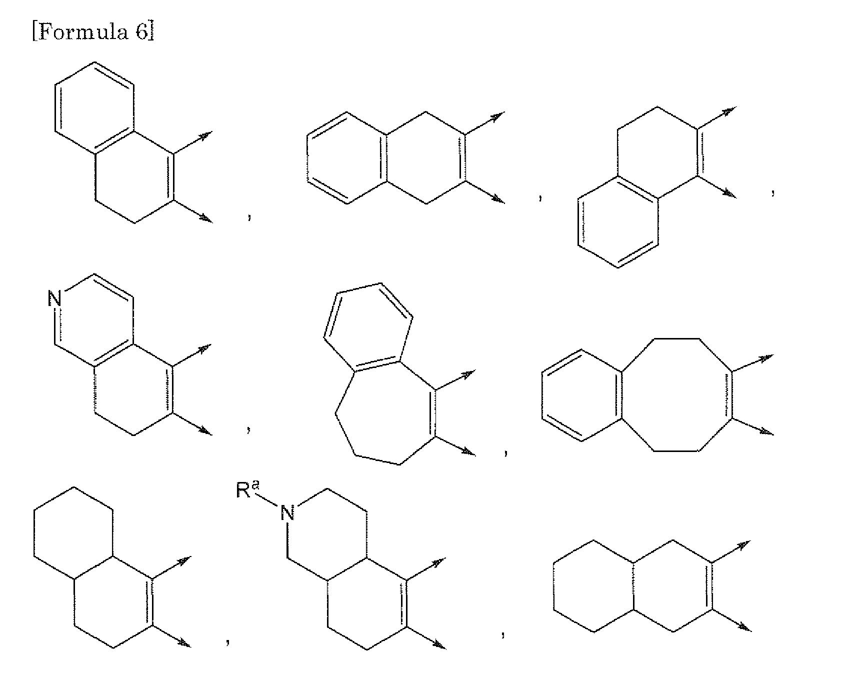 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

As an optionally substituted nonaromatic hydrocarbon ring in which a broken line represents the presence of a bond and a wavy line represents the absence of a bond, and which is formed by R² and R³ together with the adjacent carbon atom to which they are attached, preferably, the following rings are exemplified. The ring is not limited to the following rings.

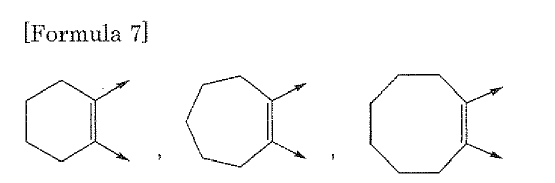

As an optionally substituted aromatic hydrocarbon ring in which a broken line represents the presence of a bond and a wavy line represents the absence of a bond, and which is formed by R² and R³ together with the adjacent carbon atom to which they are attached, the following rings are exemplified.

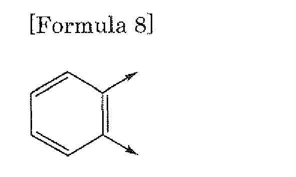

As exemplified below, the ring may be fused with an aromatic hydrocarbon ring, a nonaromatic hydrocarbon ring, an aromatic heterocycle or a nonaromatic heterocycle. 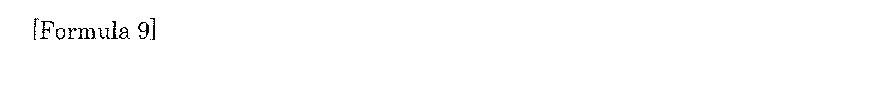 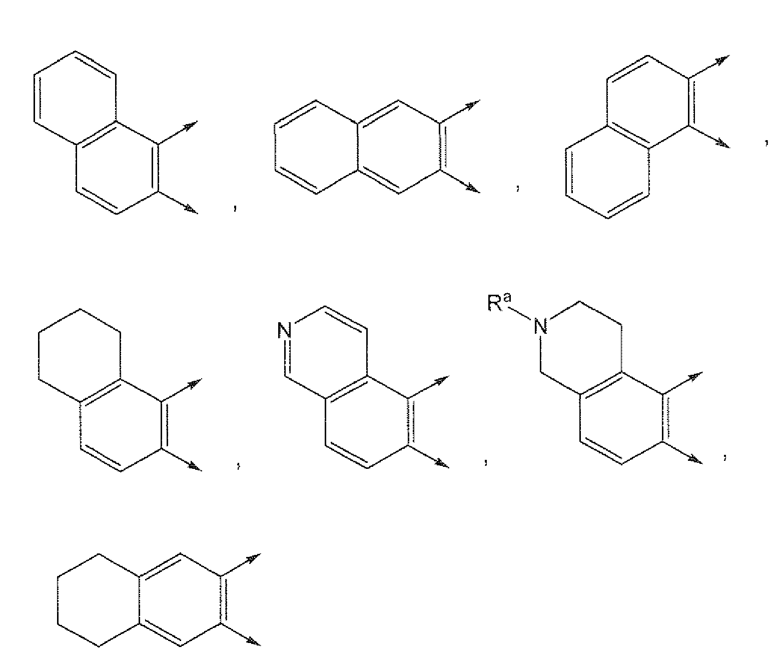 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

As an optionally substituted aromatic hydrocarbon ring in which a broken line represents the presence of a bond and a wavy line represents the absence of a bond, and which is formed by R² and R³ together with the adjacent carbon atom to which they are attached, preferably, the following rings are exemplified.

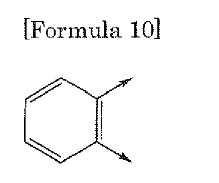

As an optionally substituted nonaromatic hydrocarbon ring in which a broken line represents the absence of a bond and a wavy line represents the presence of a bond, and which is formed by R¹ and R² together with the adjacent carbon atom to which they are attached, the following rings are exemplified. The ring is not limited to the following rings.

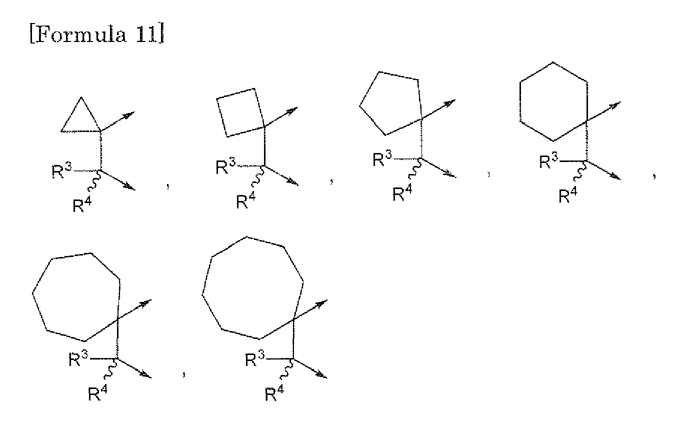

As exemplified below, the ring may have a bridged structure. 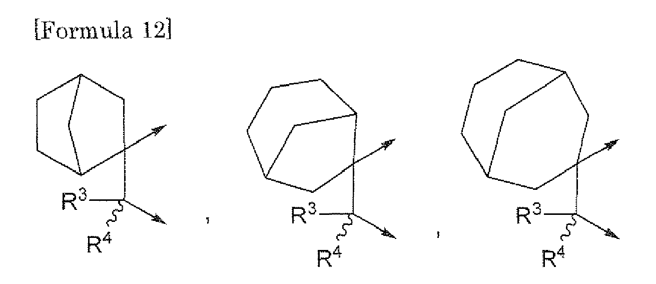 Furthermore, as exemplified below, the ring may be fused with an aromatic hydrocarbon ring, a nonaromatic hydrocarbon ring, an aromatic heterocycle or a nonaromatic heterocycle. 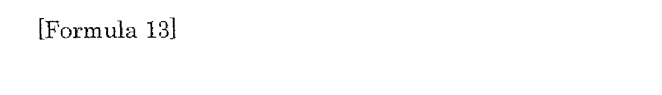 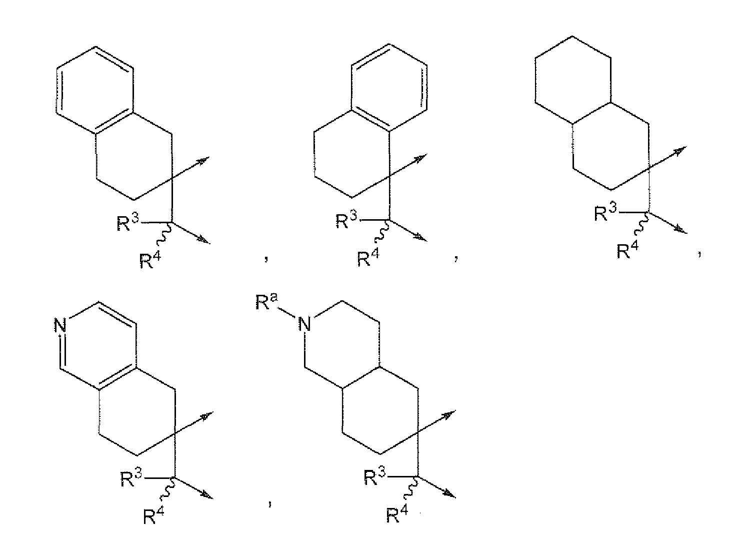 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

As an optionally substituted nonaromatic hydrocarbon ring in which a broken line represents the absence of a bond and a wavy line represents the presence of a bond, and which is formed by R¹ and R² together with the adjacent carbon atom to which they are attached, preferably, the following rings are exemplified.

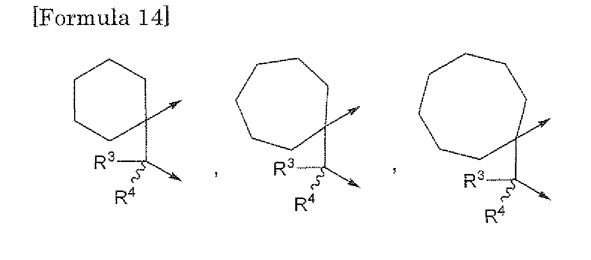

As an optionally substituted nonaromatic hydrocarbon ring in which a broken line represents the absence of a bond and a wavy line represents the presence of a bond, and which is formed by R² and R³ together with the adjacent carbon atom to which they are attached, the following rings are exemplified. The ring is not limited to the following rings.

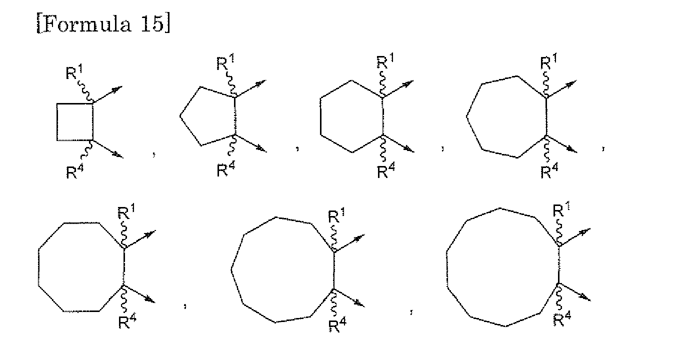

As exemplified below, the ring may have a bridged structure. 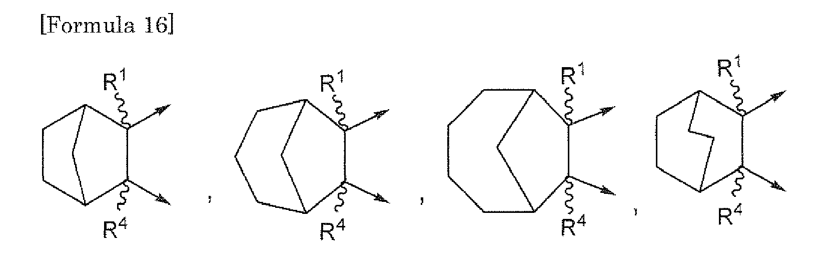 Furthermore, as exemplified below, the ring may be fused with an aromatic hydrocarbon ring, a nonaromatic hydrocarbon ring, an aromatic heterocycle or a nonaromatic heterocycle. 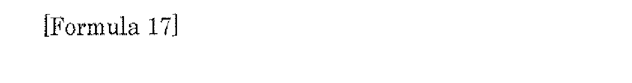 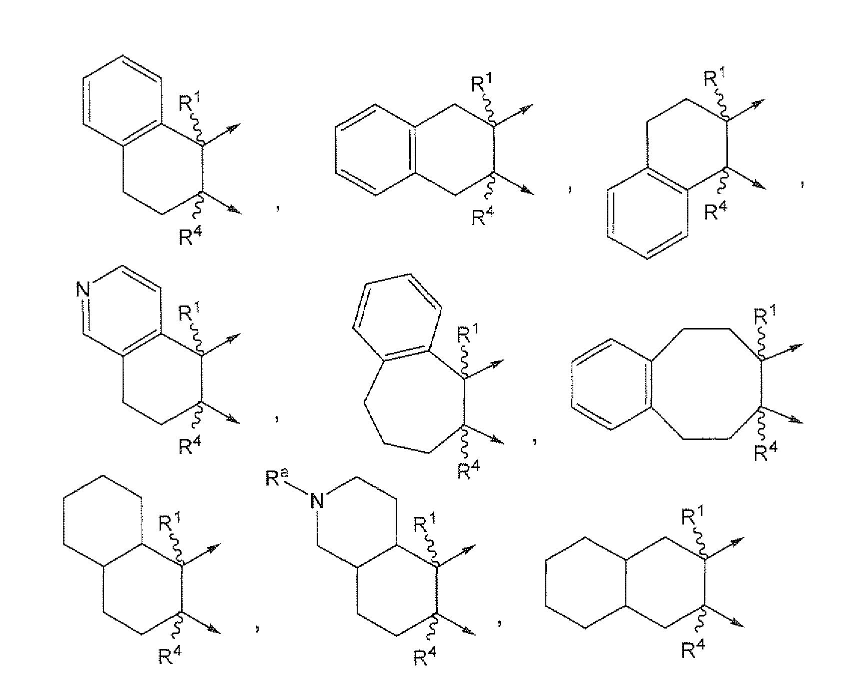 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

As an optionally substituted nonaromatic hydrocarbon ring in which a broken line represents the absence of a bond and a wavy line represents the presence of a bond, and which is formed by R² and R³ together with the adjacent carbon atom to which they are attached, preferably, the following rings are exemplified.

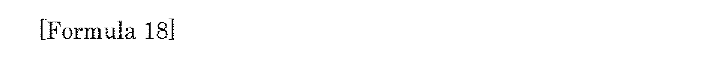

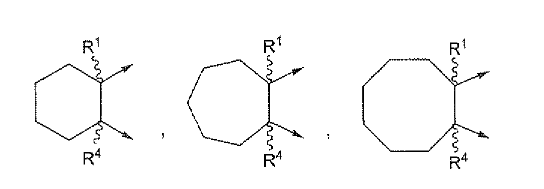

As an optionally substituted nonaromatic hydrocarbon ring in which a broken line represents the absence of a bond and a wavy line represents the presence of a bond, and which is formed by R³ and R⁴ together with the adjacent carbon atom to which they are attached, the following rings are exemplified. The ring is not limited to the following rings.

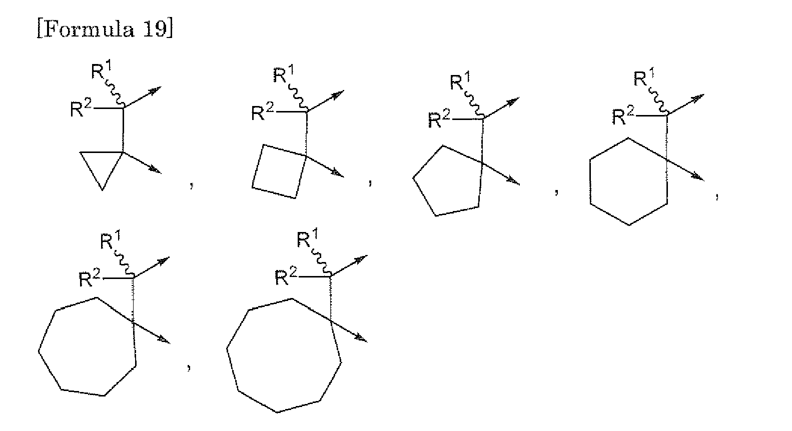

As exemplified below, the ring may have a bridged structure. 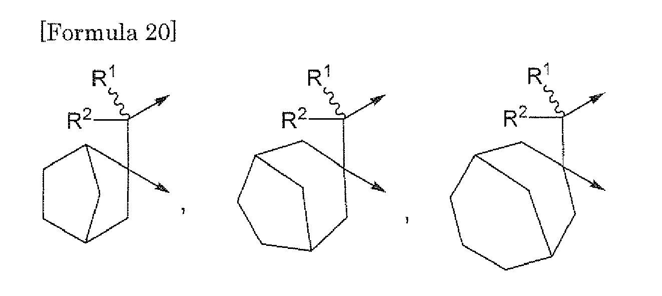 Furthermore, as exemplified below, the ring may be fused with an aromatic hydrocarbon ring, a nonaromatic hydrocarbon ring, an aromatic heterocycle or a nonaromatic heterocycle. 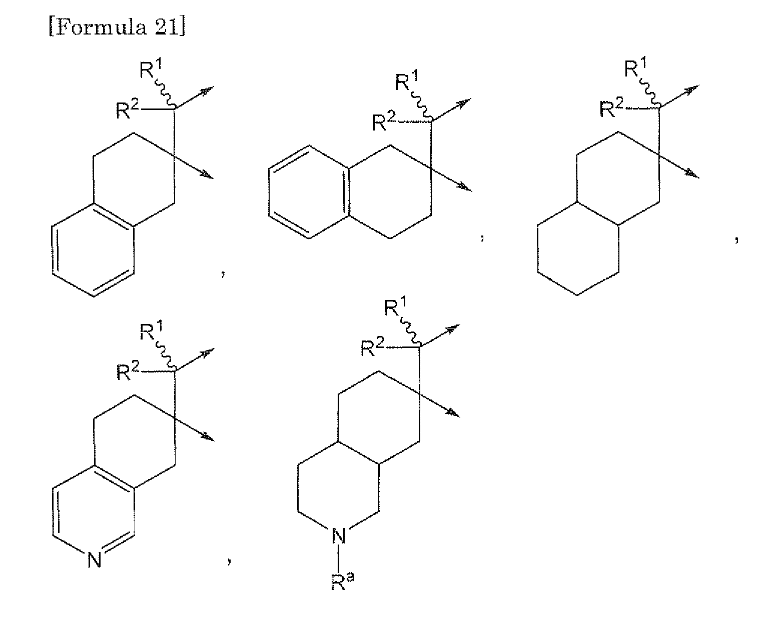 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

As an optionally substituted nonaromatic hydrocarbon ring in which a broken line represents the absence of a bond and a wavy line represents the presence of a bond, and which is formed by R³ and R⁴ together with the adjacent carbon atom to which they are attached, preferably, the following rings are exemplified.

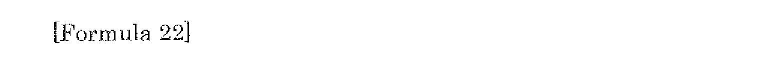

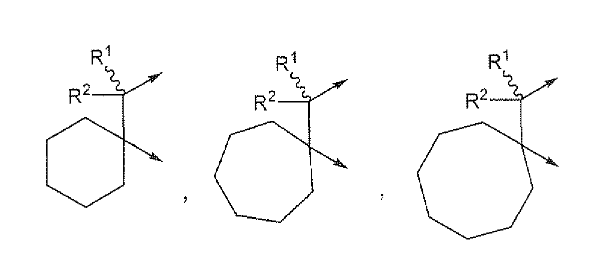

As a ring formed by R⁵ and R⁶ together with the adjacent nitrogen atom to which they are attached, the following rings are exemplified. 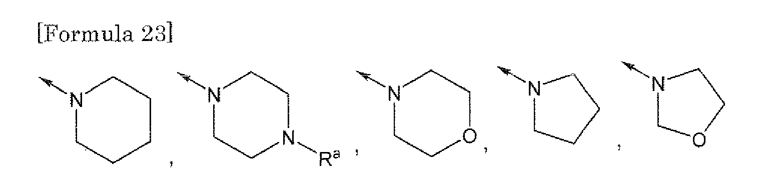 (wherein R^{a} is hydrogen, optionally substituted alkyl, optionally substituted cycloalkyl, optionally substituted aryl, optionally substituted heteroaryl, optionally substituted heterocycle, optionally substituted acyl, optionally substituted alkylsulfonyl, or optionally substituted arylsulfonyl)

Examples of a substituent of the "optionally substituted amino", the "optionally substituted carbamoyl", and the "optionally substituted sulfamoyl" include a substituent group α: hydroxy, carboxy, alkyl (e.g.: methyl, ethyl, isopropyl or tert-butyl), alkenyl (e.g.: vinyl), alkynyl (e.g.: ethynyl), cycloalkyl, (e.g.: cyclopropyl or adamantyl), cycloalkylalkyl (e.g.: cyclohexylmethyl or adamantylmethyl), cycloalkenyl, (e.g.: cyclohexenyl), cycloalkenylalkyl (e.g.: cyclohexenylmethyl), aryl (e.g.: phenyl or naphthyl), arylalkyl (e.g.: benzyl or phenethyl), heteroaryl (e.g.: pyridyl or furyl), heteroarylalkyl (e.g.: pyridylmethyl), heterocycle (e.g.: piperidyl), heterocyclealkyl (e.g.: morpholylmethyl), alkoxy (e.g.: methoxy, ethoxy, propoxy or butoxy), cycloalkyloxy (e.g.: cyclohexyloxy), aryloxy (e.g.: phenyloxy), arylalkoxy (e.g.: benzyloxy), heteroaryloxy (e.g.: pyridyloxy), heterocycleoxy (e.g.: piperidyloxy), halogenated alkoxy (e.g.: -OCF₃), alkenyloxy (e.g.: vinyloxy or allyloxy), alkoxycarbonyl (e.g.: methoxycarbonyl, ethoxycarbonyl or tert-butoxycarbonyl), cycloalkyloxycarbonyl (e.g: cyclohexyloxycarbonyl), aryloxycarbonyl (e.g.: phenyloxycarbonyl), heteroaryloxycarbonyl (e.g.: pyridyloxycarbonyl), heterocycleoxycarbonyl (e.g.: piperidyloxycarbonyl), acyl (e.g.: formyl, alkylcarbonyl (e.g.: acetyl), cycloalkylcarbonyl (e.g.: cyclohexylcarbonyl), arylcarbonyl (e.g.: benzoyl), heteroarylcarbonyl (e.g.: pyridylcarbonyl), heterocyclecarbonyl (e.g.: piperidylcarbonyl)), sulfamoyl, carbamoyl, alkylsulfonyl (e.g.: methanesulfonyl), cycloalkylsulfonyl (e.g.: cyclohexylsulfonyl), arylsulfonyl (e.g.: benzenesulfonyl or tosyl), heteroarylsulfonyl (e.g.: pyridylsulfonyl), heterocyclesulfonyl (e.g.: piperidylsulfonyl), alkylsulfinyl (e.g.: methylsulfinyl), cycloalkylsulfinyl (e.g.: cyclohexylsulfinyl), arylsulfinyl (e.g.: benzenesulfinyl), heteroarylsulfinyl (e.g.: pyridylsulfinyl), heterocyclesulfinyl (e.g.: piperidylsulfinyl) or amino (e.g.: alkylamino (e.g.: methylamino, ethylamino or dimethylamino), acylamino (e.g.: acetylamino or benzoylamino), arylamino (e.g.: anilino), arylalkylamino (e.g.: benzylamino or tritylamino), hydroxyamino or sulfoamino)). They may be substituted with 1 to 2 of the substituent(s).

A substituent in the "optionally substituted alkyl", the "optionally substituted alkenyl", the "optionally substituted alkynyl", the "optionally substituted cycloalkyl", the "optionally substituted cycloalkenyl", the "optionally substituted aryl", the "optionally substituted heteroaryl", the "optionally substituted heterocycle", the "optionally substituted acyl", the "optionally substituted alkoxy", the "optionally substituted alkoxycarbonyl", the "optionally substituted alkylthio", the "optionally substituted arylthio", the "optionally substituted alkylsulfonyl", the "optionally substituted arylsulfonyl", the "optionally substituted arylalkyl", the "optionally substituted cycloalkylalkyl", the "optionally substituted cycloalkenylalkyl", the "optionally substituted heteroarylalkyl", and the "optionally substituted heterocyclealkyl" is selected, for example, from the group consisting of a substituent group α, halogen (e.g.: F, Cl, Br, I), halogenated alkyl (e.g.: CF₃, CH₂CF₃ or CH₂CCl₃), nitro, nitroso, cyano, isocyano, isocyanato, thiocyanato, isothiocyanato, imino, alkylthio (e.g.: methylthio), cycloalkylthio (e.g.: cyclohexylthio), arylthio (e.g.: phenylthio), heteroarylthio (e.g.: pyridylthio), heterocyclethio (e.g.: piperidylthio), mercapto, sulfo, sulfino and the like. They may be substituted with 1 to 4 of the substituent(s).

A substituent on a ring of the "optionally substituted nonaromatic hydrocarbon ring in which a broken line represents the presence of a bond and a wavy line represents the absence of a bond, and which is formed by R² and R³ together with the adjacent carbon atom to which they are attached", the "optionally substituted aromatic hydrocarbon ring in which a broken line represents the presence of a bond and a wavy line represents the absence of a bond, and which is formed by R² and R³ together with the adjacent carbon atom to which they are attached", the "optionally substituted nonaromatic hydrocarbon ring in which a broken line represents the absence of a bond and a wavy line represents the presence of a bond, and which is formed by R¹ and R² together with the adjacent carbon atom to which they are attached", the "optionally substituted nonaromatic hydrocarbon ring in which a broken line represents the absence of a bond and a wavy line represents the presence of a bond, and which is formed by R² and R³ together with the adjacent carbon atom to which they are attached", and the "optionally substituted nonaromatic hydrocarbon ring in which a broken line represents the absence of a bond and a wavy line represents the presence of a bond, and which is formed by R³ and R⁴ together with the adjacent carbon atom to which they are attached" is selected from the group consisting of hydroxy, carboxy, halogen (e.g.: F, Cl, Br, I), halogenated alkyl (e.g.: CF₃, CH₂CF₃ or CH₂CCl₃), halogenated alkoxy (e.g.: -OCF₃), nitro, cyano, alkyl (e.g.: methyl, ethyl, isopropyl or tert-butyl), cycloalkyl (e.g.: cyclopropyl or adamantyl), aryl (e.g.: phenyl or naphthyl), heteroaryl (e.g.: pyridiyl or furyl), heterocycle (e.g.: piperidyl), alkoxy (e.g.: methoxy, ethoxy, propoxy or butoxy) and the like. In the case of the aromatic hydrocarbon ring, it may be substituted with 1 to 4 of the substituent(s). In the case of the nonaromatic hydrocarbon ring, it may be substituted with 1 to 12 of the substituent(s).
Preferable is halogen (e.g.: F, Cl, Br, I), alkyl (e.g.: methyl, ethyl, isopropyl or tert-butyl), or alkoxy (e.g.: methoxy, ethoxy, propoxy or butoxy).

An alkyl part and a halogen part of the "halogenated alkyl" and the "halogenated alkoxy" are as defined above.

An alkenyl part of the "alkenyloxy" means the above "alkenyl".

An alkyl part of the "alkylcarbonyl", the "alkylsulfinyl", the "alkylamino", and the "arylalkylamino" means the above "alkyl".

A cycloalkyl part of the "cycloalkyloxy", the "cycloalkyloxycarbonyl", the "cycloalkylcarbonyl", the "cycloalkylsulfonyl", the "cycloalkylsulfinyl", and the "cycloalkylthio" means the above "cycloalkyl".

An aryl part of the "aryloxy", the "aryloxycarbonyl", the "arylcarbonyl", the "arylsulfinyl", and the "arylalkylamino" means the above "aryl".

A heteroaryl part of the "heteroaryloxy", the "heteroaryloxycarbonyl", the "heteroarylcarbonyl", the "heteroarylsulfonyl", the "heteroarylsulfinyl", and the "heteroarylthio" means the above "heteroaryl".

A heterocycle part of the "heterocycleoxy", the "heterocycleoxycarbonyl", the "heterocyclecarbonyl", the "heterocyclesulfonyl", the "heterocyclesulfinyl", and the "heterocyclethio" means the above "heterocycle".

When a broken line represents the presence of a bond, a wavy line represents the absence of a bond,
R² and R³ are each independently hydrogen, halogen, cyano, hydroxyl, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R² and R³ may be taken together with the adjacent carbon atom to which they are attached to form an optionally substituted nonaromatic hydrocarbon ring or an optionally substituted aromatic hydrocarbon ring.
Preferably, R² and R³ are each independently hydrogen, optionally substituted alkyl, optionally substituted cycloalkyl, or optionally substituted cycloalkylalkyl, or R² and R³ are taken together with the adjacent carbon atom to which they are attached to form an optionally substituted nonaromatic hydrocarbon ring, or an optionally substituted aromatic hydrocarbon ring.
More preferably, R² and R³ are taken together with the adjacent carbon atom to which they are attached to form an optionally substituted nonaromatic hydrocarbon ring, or an optionally substituted aromatic hydrocarbon ring.

When a broken line represents the absence of a bond, a wavy line represents the presence of a bond,
R¹ and R⁴ are each independently hydrogen, halogen, hydroxy, optionally substituted alkyloxy, optionally substituted alkyl, optionally substituted cycloalkyl or optionally substituted aryl,
R² and R³ are each independently hydrogen, halogen, cyano, hydroxyl, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or
R¹ and R², R² and R³ or R³ and R⁴ are taken together with the adjacent carbon atom to which they are attached to form an optionally substituted nonaromatic hydrocarbon ring.
Preferably, R¹, R² and R⁴ are each independently hydrogen, and R³ is optionally substituted alkyl, optionally substituted cycloalkyl, or optionally substituted aryl, or
R¹, R³ and R⁴ are each independently hydrogen, and R² is optionally substituted alkyl, optionally substituted cycloalkyl, optionally substituted cycloalkylalkyl, optionally substituted aryl, or optionally substituted arylalkyl, or
R¹ and R² are each independently hydrogen, and R³ and R⁴ are taken together with the adjacent carbon atom to which they are attached to form an optionally substituted nonaromatic hydrocarbon ring, or
R¹ and R⁴ are each independently hydrogen, and R² and R³ are taken together with the adjacent carbon atom to which they are attached to form an optionally substituted nonaromatic hydrocarbon ring.
More preferably, R¹ and R⁴ are each independently hydrogen, and R² and R³ are taken together with the adjacent carbon atom to which they are attached to form an optionally substituted nonaromatic hydrocarbon ring.

R⁵ and R⁶ are each independently hydrogen, optionally substituted alkyl, optionally substituted alkenyl, optionally substituted alkynyl, optionally substituted aryl, optionally substituted arylalkyl, optionally substituted cycloalkyl, optionally substituted cycloalkylalkyl, optionally substituted cycloalkenyl, optionally substituted cycloalkenylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted sulfamoyl, optionally substituted alkylsulfonyl or optionally substituted arylsulfonyl, or R⁵ and R⁶ are taken together with the adjacent nitrogen atom to which they are attached to form an optionally substituted ring.
Preferably, R⁵ and R⁶ are each independently hydrogen, optionally substituted alkyl, optionally substituted aryl, optionally substituted arylalkyl, optionally substituted cycloalkyl, optionally substituted heteroaryl, optionally substituted heterocycle, optionally substituted amino, or optionally substituted arylsulfonyl, or R⁵ and R⁶ are taken together with the adjacent nitrogen atom to which they are attached to form an optionally substituted ring.

X is O or S. Preferable is S.

As a salt of the present compound, a pharmaceutically acceptable salt is preferable. As a pharmaceutically acceptable salt, the following salts can be included.
As a basic salt, example includes alkali metal salt such as sodium salt or potassium salt; alkaline earth metal salt such as calcium salt or magnesium salt; ammonium salt; aliphatic amine salt such as trimethylamine salt, triethylamine salt, dicyclohexylamine salt, ethanolamine salt, diethanolamine salt, triethanolamine salt, procaine salt, meglumine salt, diethanolamine salt or ethylenediamine salt; aralkylamine salt such as N,N-dibenzylethylenediamine salt or benethamine salt; heterocyclic aromatic amine salt such as pyridine salt, picoline salt, quinoline salt, or isoquinoline salt; quaternary ammonium salt such as tetramethylammonium salt, tetraethylammonium salt, benzyltrimethylammonium salt, benzyltriethylammonium salt, benzyltributylammonium salt, methyltrioctylammonium salt, or tetrabutylammonium salt; basic amino acid salt such as arginine salt or lysine salt or the like.
As an acidic salt, example includes inorganic acid salt such as hydrochloride, sulfate, nitrate, phosphate, carbonate, hydrogencarbonate, or perchlorate; organic acid salt such as acetate, propionate, lactate, maleate, fumarate, tartrate, malate, citrate or ascorbate; sulfonate such as methanesulfonate, isethionate, benzenesulfonate or p-toluenesulfonate; acidic amino acid salt such as aspartate or glutamate or the like.

The term "solvate" means a solvate of a compound of the present invention or a pharmaceutically acceptable salt thereof, and example includes alcohol (e.g.: ethanol) solvate, hydrate or the like. Example of hydrate includes monohydrate, dihydrate or the like.

A general process for producing the present compound is exemplified below. As extraction, purification or the like, treatment which is performed in a usual organic chemical experiment may be conducted.
(Compounds wherein X is S, a broken line represents the presence of a bond, a wavy line represents the absence of a bond, and R² and R³ are taken together with the adjacent carbon atom to which they are attached to form an optionally substituted aromatic hydrocarbon ring) 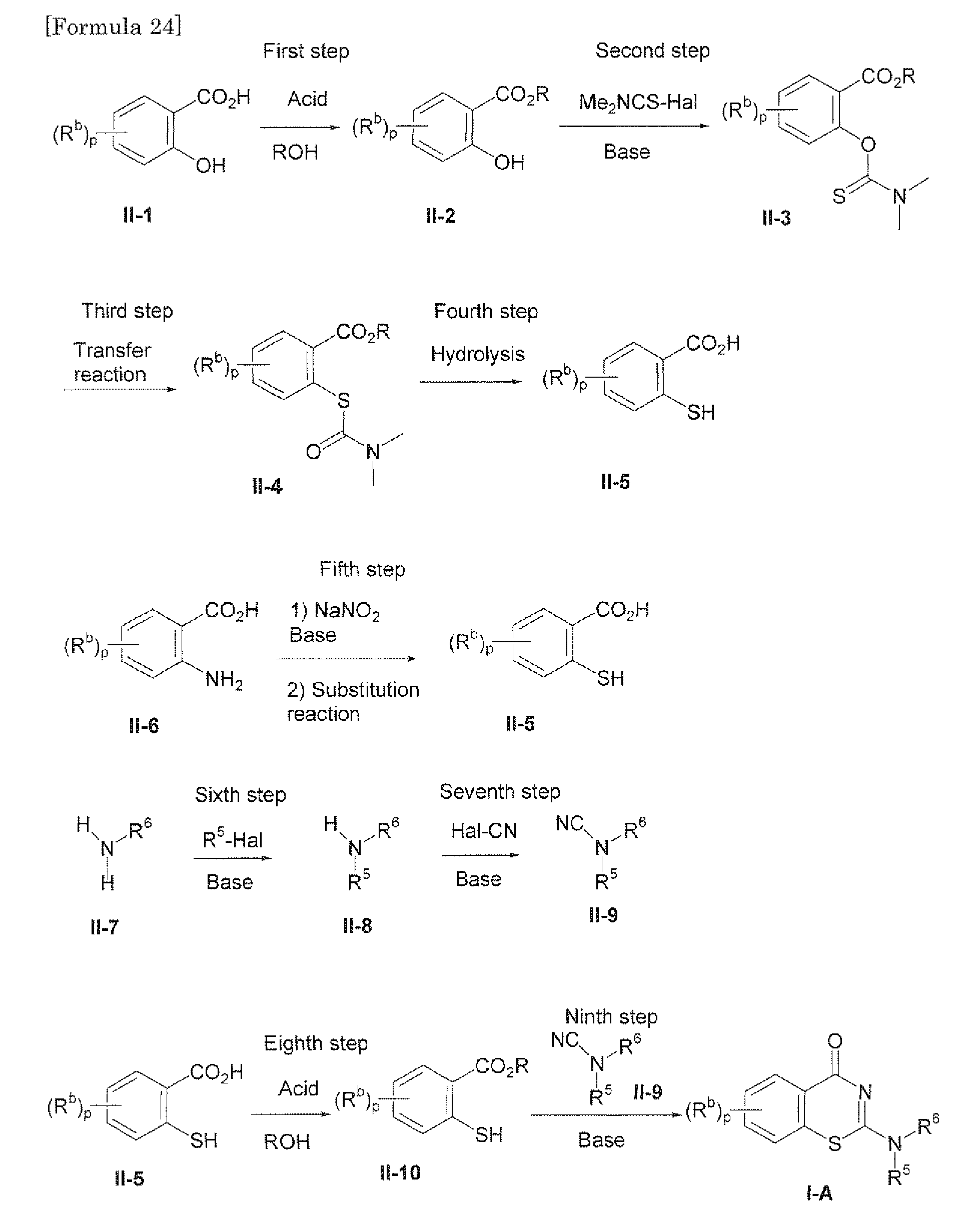 (wherein each symbol is as defined above and, as a compound represented by the formula (II-1), the formula (II-6) and the formula (II-7), a known compound may be used, or a compound derived from a known compound by a usual method may be used. R is alkyl, Hal is halogen, and p is an integer of 0 to 4. R^{b} means a substituent on a ring of "an optionally substituted aromatic hydrocarbon ring in which a broken line represents the presence of a bond and a wavy line represents the absence of a bond, and which is formed by R² and R³ together with the adjacent carbon atom to which they are attached")

### First step

First step is a step of esterifying carboxylic acid represented by the formula (II-1) to produce a compound represented by the formula (II-2).
A reaction may be performed using alcohols (e.g.: methanol, ethanol or the like) as a solvent in the presence of an acid such as hydrochloric acid, sulfuric acid, nitric acid, thionyl chloride and oxalyl chloride.
As the esterification reaction, a method which is generally well-known such as a method of reacting carboxylic acid and diazomethane may be used.
An esterification reaction using an acid and an alcohol may be performed at -20°C to a temperature at which a solvent used is refluxed for 0.5 to 24 hours.

### Second step

Second step is a step of reacting a compound represented by the formula (II-2) and N,N-dimethylthiocarbamoyl halide in the presence of a base to produce a compound represented by the formula (II-3).
As a solvent, example includes N-dimethylformamide, dimethyl sulfoxide, aromatic hydrocarbons (e.g., toluene, benzene, xylene or the like), saturated hydrocarbons (e.g., cyclohexane, hexane or the like), halogenated hydrocarbons (e.g., dichloromethane, chloroform, 1,2-dichloroethane, carbon tetrachloride or the like), ethers (e.g., tetrahydrofuran, diethyl ether, dioxane, 1,2-dimethoxyethane or the like), esters (e.g., methyl acetate, ethyl acetate or the like), ketones (e.g., acetone, methylethylketone or the like), nitriles (e.g., acetonitrile or the like), alcohols (e.g., methanol, ethanol, t-butanol or the like), water, a mixed solvent thereof or the like.
As a base, example includes metal hydrides (e.g., sodium hydride or the like), metal hydroxides (e.g., sodium hydroxide, potassium hydroxide, lithium hydroxide, barium hydroxide or the like), metal carbonates (e.g., sodium carbonate, calcium carbonate, cesium carbonate, potassium carbonate or the like), metal alkoxides (e.g., sodium methoxide, sodium ethoxide, potassium t-butoxide or the like), sodium hydrogen carbonate, metal sodium, organic amines (e.g., triethylamine, diisopropylethylamine, DBU, 1,4-diazabicyclo[2.2.2]octane or the like), pyridine, alkyl lithiums (n-BuLi, sec-BuLi, tert-BuLi), LDA or the like.
Preferably, as the solvent, N-dimethylformamide and, as the base, 1,4-diazabicyclo[2.2.2]octane may be used. The reaction may be performed at -20 to 50°C for 0.5 to 36 hours.

### Third step

Third step is a step of converting a compound represented by the formula (II-3) into a compound represented by the formula (II-4) by a transfer reaction.
As a solvent, a solvent described in second step may or may not be used. For example, the reaction may be performed at a high temperature of 180 to 200°C for 0.5 to 24 hours.

### Fourth step

Fourth step is a step of hydrolyzing a compound represented by the formula (II-4) to produce a compound represented by the formula (II-5).
A solvent and a base described in second step can be used. Preferably, as a solvent, water and, as a base, metal hydroxide (e.g., sodium hydroxide, potassium hydroxide, lithium hydroxide, barium hydroxide or the like) may be used.
The reaction may be performed at 50 to 110°C for 0.5 to 24 hours.

### Fifth step

Fifth step is another method of synthesizing a compound represented by the formula (II-5). Fifth step is a step of converting a compound represented by the formula (II-6) into a compound represented by the formula (II-5) by a Sandmeyer reaction.
As a solvent, a solvent described in second step can be used. Preferably, a diazonium salt may be produced using water as a solvent, and using hydrochloric acid, sodium nitrite, or sodium hydroxide. After neutralization of the reaction solution, the reaction solution may be added dropwise to a mixture of potassium O-ethyl dithiocarbonate and water to perform a substitution reaction. A step of producing a diazonium salt may be performed at -20 to 0°C for 0.5 to 1 hour, and a substitution reaction may be performed at 50 to 100°C for 0.5 to 1 hour.

### Sixth step

Sixth step is a step of reacting a compound represented by the formula (II-7) with a compound represented by the formula R⁵-Hal to produce a compound represented by the formula (II-8).
A solvent and a base described in second step can be used. Preferably, as a solvent, N-dimethylformamide and, as a base, metal carbonate (e.g., sodium carbonate, calcium carbonate, cesium carbonate, potassium carbonate or the like) may be used.
The reaction may be performed at -20 to 50°C for 0.5 to 24 hours.

### Seventh step

Seventh step is a step of reacting a compound represented by the formula (II-8) with a compound represented by the formula Hal-CN to produce a compound represented by the formula (II-9).
A solvent and a base described in second step can be used. Preferably, as a solvent, ethers (e.g., tetrahydrofuran, diethyl ether, dioxane, 1,2-dimethoxyethane or the like), or ketones (e.g., acetone, methylethylketone or the like) may be used. As a base, organic amine (e.g., triethylamine, diisopropylethylamine, DBU, 1,4-diazabicyclo[2.2.2]octane or the like), or metal carbonate (e.g., sodium carbonate, calcium carbonate, cesium carbonate, potassium carbonate or the like) may be used.
The reaction may be performed at -20 to 50°C for 0.5 to 24 hours.

### Eighth step

Eighth step is a step of esterifying carboxylic acid represented by the formula (II-5) to produce a compound represented by the formula (II-10).
The reaction may be performed under the same conditions as the above first step.

### Ninth step

Ninth step is a step of reacting a compound represented by the formula (II-10) with a compound represented by the formula (II-9) to produce a compound represented by the formula (I-A).
A solvent and a base described in second step can be used. Preferably, as a solvent, aromatic hydrocarbons (e.g., toluene, benzene, xylene or the like) and, as a base, organic amine (e.g., triethylamine, diisopropylethylamine, DBU, 1,4-diazabicyclo[2.2.2]octane or the like) may be used.
The reaction may be performed at 30°C to a temperature at which a solvent used is refluxed for 0.5 to 24 hours.
([Compound: I-C], wherein X is S, a broken line represents the presence of a bond, a wavy line represents the absence of a bond, and R² and R³ are taken together with the adjacent carbon atom to which they are attached to form an optionally substituted nonaromatic hydrocarbon ring, and
[Compound: I-B], wherein X is S, a broken line represents the absence of a bond, a wavy line represents the presence of a bond, R¹ and R⁴ are hydrogen, and R² and R³ are taken together with the adjacent carbon atom to which they are attached to form an optionally substituted nonaromatic hydrocarbon ring) 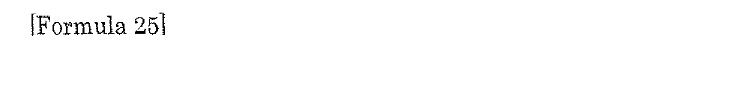 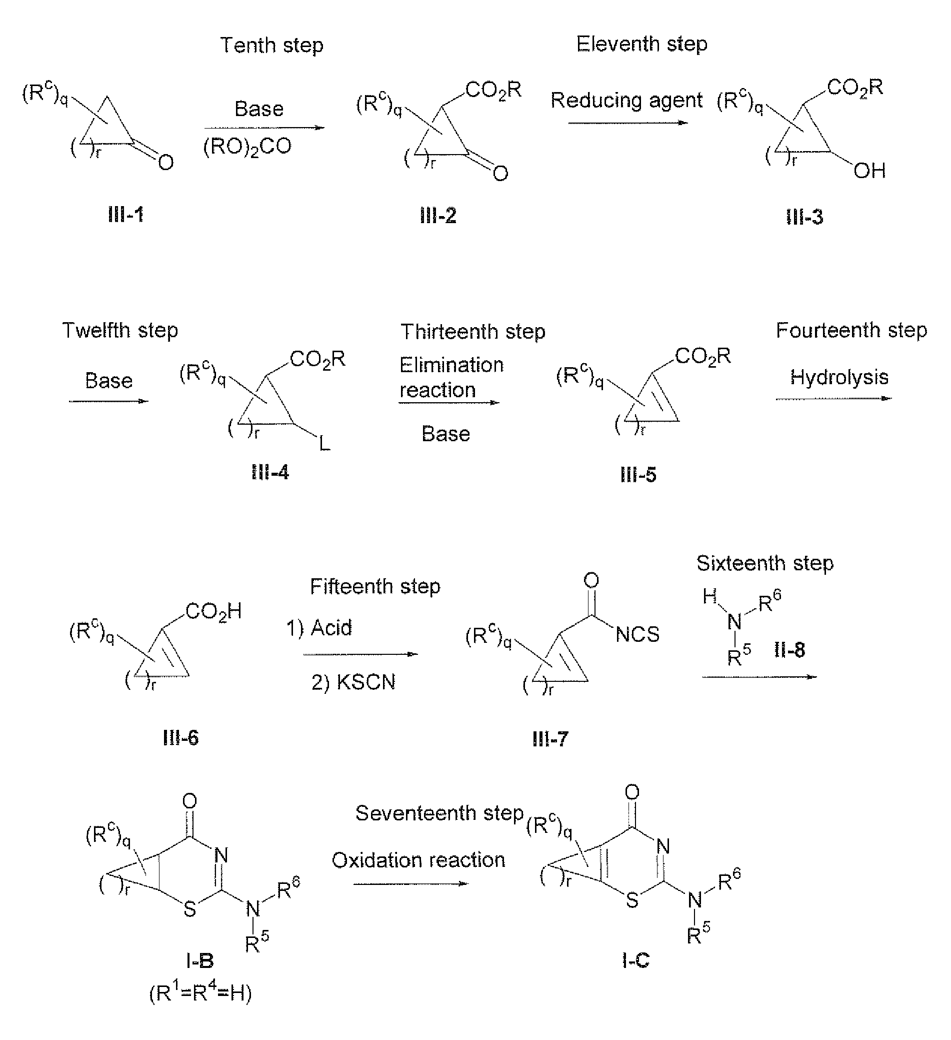 (wherein, each symbol is as defined above and, as a compound represented by the formula (III-1), a known compound may be used, or a compound derived from a known compound by a usual method may be used. R is alkyl, q is an integer of 0 to 12, r is an integer of 1 to 6. And, L means an elimination group. Example of the elimination group includes -OMs, -OTs, -OTf, -ONs or the like. Herein, "Ms" represents a methanesulfonyl group, "Ts" represents a paratoluenesulfonyl group, "Tf" represents a trifluoromethanesulfonyl group, and "Ns" represents an orthonitrobenzenesulfonyl group. R^{c} means a substituent on a ring of "an optionally substituted nonaromatic hydrocarbon ring in which a broken line represents the presence of a bond and a wavy line represents the absence of a bond, and which is formed by R² and R³ together with the adjacent carbon atom to which they are attached", or a substituent on a ring of "an optionally substituted nonaromatic hydrocarbon ring in which a broken line represents the absence of a bond and a wavy line represents the presence of a bond, and which is formed by R² and R³ together with the adjacent carbon atom to which they are attached")

### Tenth step

Tenth step is a step of reacting a compound represented by the formula (III-1) with dialkyl carbonate in the presence of a base to produce a compound represented by the formula (III-2).
A solvent and a base described in second step can be used. Preferably, as a solvent, ethers (e.g., tetrahydrofuran, diethyl ether, dioxane, 1,2-dimethoxyethane or the like) may be used. As a base, metal hydride (e.g., sodium hydride or the like), or LDA may be used.
The reaction may be performed at -78 to 40°C for 0.5 to 24 hours.

### Eleventh step

Eleventh step is a step of reducing a compound represented by the formula (III-2) to produce a compound represented by the formula (III-3).
A solvent described in second step can be used. Preferably, ethers (e.g., tetrahydrofuran, diethyl ether, dioxane, 1,2-dimethoxyethane or the like), or alcohols (e.g., methanol, ethanol or the like) may be used.
As a reducing agent, for example, sodium triacetoxyhydroborate, sodium borohydride, lithium tetrahydroborate, pyridine borane complex, tetrahydrofuran borane complex, 2-picoline borane complex, dimethyl sulfide-borane complex, sodium, sodium cyanoborohydrate, lithium triethylborohydrate, aluminum lithium hydride, Red-Al[bis(2-methoxyethoxy)aluminum sodium hydride], L-Selectride [lithium tri(sec-butyl)borohydride], K-Selectride [potassium tri(sec-butyl)borohydride], DIBAL-H(diisobutylaluminum hydride) or the like can be used. Preferably, sodium borohydride may be used.
The reaction may be performed at -20 to 50°C for 0.5 to 24 hours.

### Twelfth step

Twelfth step is a step of converting a hydroxy group of a compound represented by the formula (III-3) into an elimination group to produce a compound represented by the formula (III-4).
As a solvent, a solvent described in second step can be used. Preferably, ethers (e.g., tetrahydrofuran, diethyl ether, dioxane, 1,2-dimethoxyethane or the like) may be used. As a base, a base described in second step can be used. Preferably, organic amine (e.g., triethylamine, diisopropylethylamine, DBU, 1,4-diazabicyclo[2.2.2]octane or the like), or pyridine may be used.
The reaction may be performed at -20 to 40°C for 0.5 to 24 hours in the presence of mesyl chloride (MsCl), tosyl chloride (TsCl), trifluoromethanesulfonyl chloride (TfCl), trifluoromethanesulfonic acid anhydride (Tf₂O), nosyl chloride (NsCl) or the like.

### Thirteenth step

Thirteenth step is a step of converting a compound represented by the formula (III-4) into a compound represented by the formula (III-5) by an elimination reaction.
A solvent and a base described in second step can be used. Preferably, as a solvent, aromatic hydrocarbons (e.g., toluene, benzene, xylene or the like) and, as a base, DBU may be used.
The reaction may be performed at 40°C to a temperature at which a solvent used is refluxed for 0.5 to 24 hours.

### Fourteenth step

Fourteenth step is a step of converting a compound represented by the formula (III-5) into a compound represented by the formula (III-6) by a hydrolysis reaction.
The reaction may be performed under the same conditions as the above fourth step.

### Fifteenth step

Fifteenth step is a step of converting a compound represented by the formula (III-6) into acid chloride, and reacting this with KSCN to convert into a compound represented by the formula (III-7).
A solvent described in second step can be used. Preferably, the compound(III-6) may be converted into acid chloride using ethers (e.g., tetrahydrofuran, diethyl ether, dioxane, 1,2-dimethoxyethane or the like) in the presence of a few drops of DMF and an acid, followed by a reaction with KSCN. As an acid, oxalyl chloride, or thionyl chloride may be used.
The reaction for converting the compound(III-6) into acid chloride may be performed at -20°C to a temperature at which a solvent used is refluxed for 0.5 to 24 hours. The next substitution reaction may be performed at 0 to 40°C for 0.5 to 24 hours.

### Sixteenth step

Sixteenth step is a step of reacting a compound represented by the formula (III-7) with a compound represented by the formula (II-8) to produce a compound represented by the formula (I-B).
As a solvent, a solvent described in second step can be used. Preferably, ethers (e.g., tetrahydrofuran, diethyl ether, dioxane, 1,2-dimethoxyethane or the like) may be used.
The reaction may be performed at 40°C to a temperature at which a solvent used is refluxed for 0.5 to 24 hours.

### Seventeenth step

Seventeenth step is a step of converting a compound represented by the formula (I-B) into a compound represented by the formula (I-C) by an oxidation reaction.
As a solvent, a solvent described in second step can be used. Preferably, the reaction may be performed using ethers (e.g., tetrahydrofuran, diethyl ether, dioxane, 1,2-dimethoxyethane or the like) in the presence of DDQ.
The reaction may be performed at 40°C to a temperature at which a solvent used is refluxed for 0.5 to 24 hours.

(Compounds wherein X is S, a broken line represents the absence of a bond, a wavy line represents the presence of a bond, R¹ and R⁴ are as defined above [provided that the case where R¹ and R⁴ are hydrogen simultaneously is excluded], and R² and R³ are taken together with the adjacent carbon atom to which they are attached to form an optionally substituted nonaromatic hydrocarbon ring) 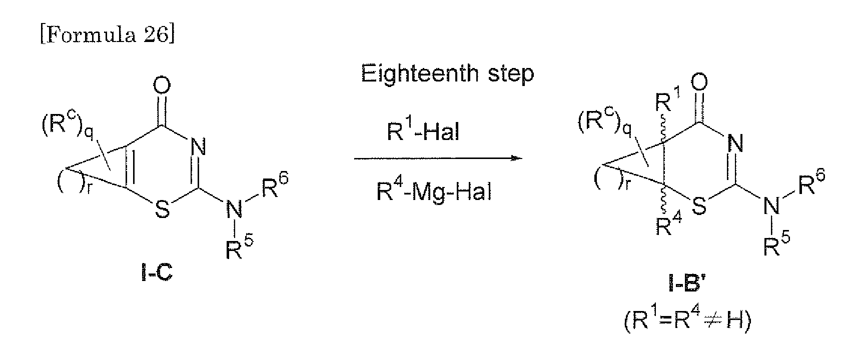 (wherein each symbol is as defined above, Hal is halogen, q is an integer of 0 to 12, and r is an integer of 1 to 6. R^{c} means a substituent on a ring of "an optionally substituted nonaromatic hydrocarbon ring in which a broken line represents the absence of a bond and a wavy line represents the presence of a bond, and which is formed by R² and R³ together with the adjacent carbon atom to which they are attached")

### Eighteenth step

Eighteenth step is a step of converting a compound represented by the formula (I-C) into a compound represented by the formula (I-B').
As a solvent, a solvent described in second step can be used. Preferably, ethers (e.g., tetrahydrofuran, diethyl ether, dioxane, 1,2-dimethoxyethane or the like) may be used. When a substituent is introduced into both of R¹ and R⁴, a reaction of three components of a compound represented by the formula (I-C), R⁴-Mg-Hal and R¹-Hal (or R¹-Mg-Hal and R⁴-Hal) may be performed. When a substituent is introduced into any one of R¹ and R⁴, a Michael addition reaction of a compound represented by the formula (I-C), and R¹-Mg-Hal or R⁴-Mg-Hal may be performed.
([Compound: I-D], wherein a broken line represents the absence of a bond, a wavy line represents the presence of a bond, and R¹ and R² are each hydrogen, and
[Compound: I-E], wherein a broken line represents the presence of a bond, a wavy line represents the absence of a bond, and R¹ and R² are each hydrogen) 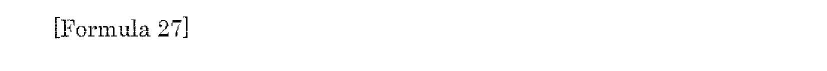 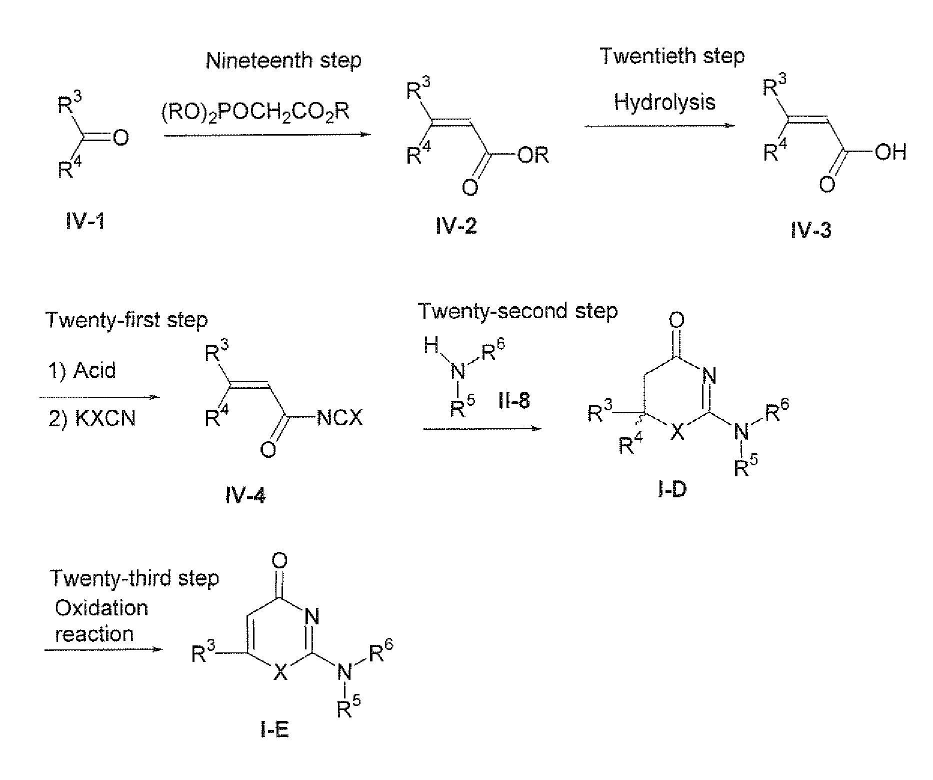 (wherein each symbol is as defined above and, as a compound represented by the formula (IV-1), a known compound may be used, or a compound derived from a known compound by a usual method may be used. R is alkyl)

### Nineteenth step

Nineteenth step is a step of producing a compound represented by the formula (IV-2) from a compound represented by the formula (IV-1) by a Horner-Wadsworth-Emmons reaction.
As a solvent, a solvent described in second step can be used. Preferably, ethers (e.g., tetrahydrofuran, diethyl ether, dioxane, 1,2-dimethoxyethane or the like) may be used.
As a base, a base described in second step can be used. Preferably, metal hydride (e.g., sodium hydride or the like) may be used.
The reaction may be performed at -20 to 40°C for 0.5 to 24 hours.

### Twentieth step

Twentieth step is a step of hydrolyzing a compound represented by the formula (IV-2) to produce a compound represented by the formula (IV-3).
The reaction may be performed under the same conditions as the above fourth step.

### Twenty-first step

Twenty-first step is a step of reacting a compound represented by the formula (IV-3) with a compound represented by KXCN to produce a compound represented by the formula (IV-4).
The reaction may be performed under the same conditions as the above fifteenth step using KSCN or KOCN.

### Twenty-second step

Twenty-second step is a step of reacting a compound represented by the formula (IV-4) with a compound represented by the formula (II-8) to produce a compound represented by the formula (I-D).
The reaction may be performed under the same conditions as the above sixteenth step.

### Twenty-third step

Twenty-third step is a step of converting a compound represented by the formula (I-D) into a compound represented by the formula (I-E) by an oxidation reaction.
The reaction may be performed under the same conditions as the above seventeenth step.

(Compounds wherein X is O, a broken line represents the presence of a bond, a wavy line represents the absence of a bond, and R² and R³ are taken together with the adjacent carbon atom to which they are attached to form an optionally substituted aromatic hydrocarbon ring) 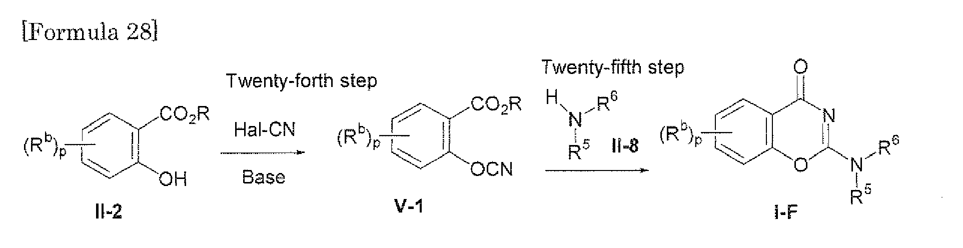 (wherein each symbol is as defined above and, as a compound represented by the formula (II-2), a known compound may be used, or a compound derived from a known compound by a usual method may be used. Hal is halogen, R is alkyl, and p is an integer of 0 to 4. R^{b} means a substituent on a ring of "an optionally substituted aromatic hydrocarbon ring in which a broken line represents the presence of a bond and a wavy line represents the absence of a bond, and which is formed by R² and R³ together with the adjacent carbon atom to which they are attached")

### Twenty-fourth step

Twenty-fourth step is a step of reacting a compound represented by the formula (II-2) with a compound represented by Hal-CN to produce a compound represented by the formula (V-1).
As a solvent, a solvent described in second step can be used. Preferably, ketones (e.g., acetone, methyl ethyl ketone or the like) may be used.
As a base, a base described in second step can be used. Preferably, organic amine (e.g., triethylamine, diisopropylethylamine, DBU, 1,4-diazabicyclo[2.2.2]octane or the like) may be used.
The reaction may be performed at -20 to 40°C for 0.5 to 48 hours.

### Twenty-fifth step

Twenty-fifth step is a step of reacting a compound represented by the formula (V-1) with a compound represented by the formula (II-8) to produce a compound represented by the formula (I-F).
As a solvent, a solvent described in second step can be used. Preferably, ketones (e.g., acetone, methyl ethyl ketone or the like) may be used.
The reaction may be performed at -20 to 40°C for 0.5 to 24 hours in the presence of benzoic acid.
([Compound: I-H], wherein X is O, a broken line represents the presence of a bond, a wavy line represents the absence of a bond, and R² and R³ are taken together with the adjacent carbon atom to which they are attached to form an optionally substituted nonaromatic hydrocarbon ring,
[Compound: I-G], wherein X is O, a broken line represents the absence of a bond, a wavy line represents the presence of a bond, R¹ and R⁴ are hydrogen, and R² and R³ are taken together with the adjacent carbon atom to which they are attached to form an optionally substituted nonaromatic hydrocarbon ring and
[Compound: I-G'], wherein X is O, a broken line represents the absence of a bond, a wavy line represents the presence of a bond, R¹ and R⁴ are as defined above [provided that case where R¹ and R⁴ are hydrogen simultaneously is excluded], and R² and R³ are taken together with the adjacent carbon atom to which they are attached to form an optionally substituted nonaromatic hydrocarbon ring) 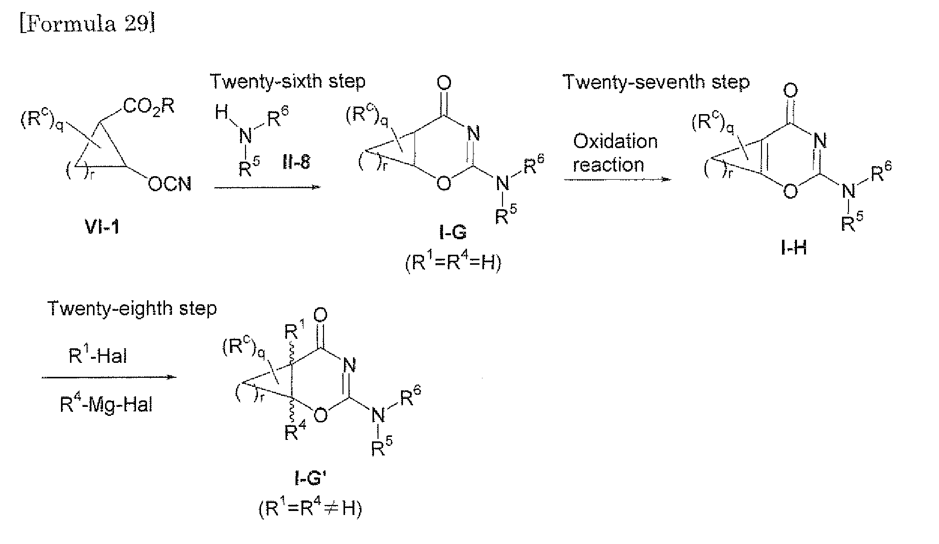 (wherein each symbol is as defined above and, as a compound represented by the formula (VI-1), a known compound may be used, or a compound derived from a known compound by a usual method may be used. R is alkyl, q is an integer of 0 to 12, and r is an integer of 1 to 6. R^{c} means a substituent on a ring of "an optionally substituted nonaromatic hydrocarbon ring in which a broken line represents the presence of a bond and a wavy line represents the absence of a bond, and which is formed by R² and R³ together with the adjacent carbon atom to which they are attached", or a substituent on a ring of "an optionally substituted nonaromatic hydrocarbon ring in which a broken line represents the absence of a bond and a wavy line represents the presence of a bond, and which is formed by R² and R³ together with the adjacent carbon atom to which they are attached".

### Twenty-sixth step

Twenty-sixth step is a step of reacting a compound represented by the formula (VI-1) with a compound represented by the formula (II-8) to produce a compound represented by the formula (I-G).
The reaction may be performed under the same conditions as the above twenty-fifth step.

### Twenty-seventh step

Twenty-seventh step is a step of converting a compound represented by the formula (I-G) into a compound represented by the formula (I-H) by an oxidation reaction.
The reaction may be performed under the same conditions as the above seventeenth step.

### Twenty-eighth step

Twenty-eighth step is a step of converting a compound represented by the formula (I-H) into a compound represented by the formula (I-G').
The reaction may be performed under the same conditions as the above eighteenth step.
([Compound: I-J, I-J'], wherein a broken line represents the absence of a bond, a wavy line represents the presence of a bond, and R³ and R⁴ are each hydrogen and
[Compound: 1-K], wherein a broken line represents the presence of a bond, a wavy line represents the absence of a bond, and R³ and R⁴ are each hydrogen) 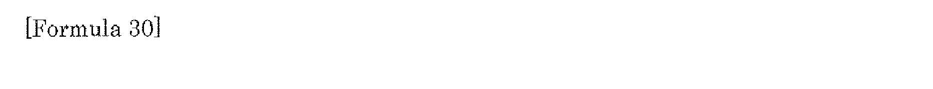 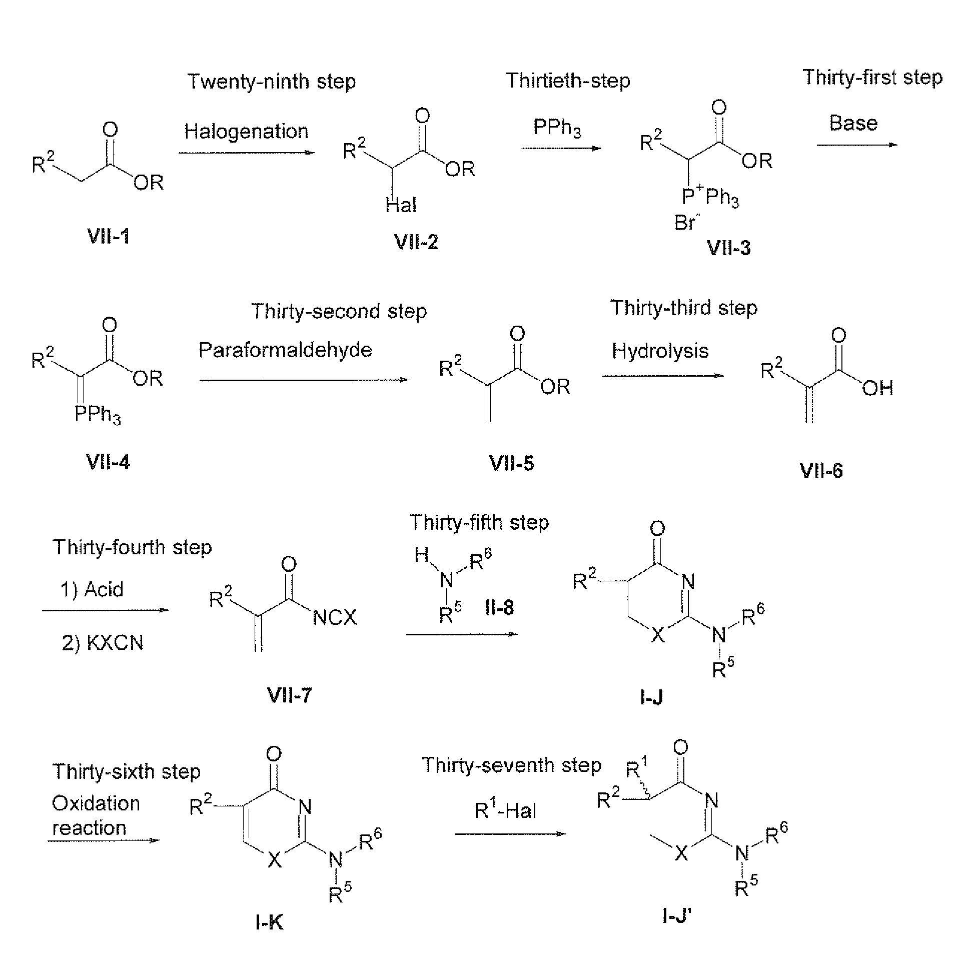 (wherein each symbol is as defined above and, as a compound represented by the formula (VII-1), a known compound may be used, or a compound derived from a known compound by a usual method may be used. R is alkyl, and Hal is halogen)

### Twenty-ninth step

Twenty-ninth step is a step of halogenating a compound represented by the formula (VII-1) to produce a compound represented by the formula (VII-2).
As a solvent, a solvent described in second step can be used. Preferably, halogenated hydrocarbons (e.g., dichloromethane, chloroform, 1,2-dichloroethane, carbon tetrachloride or the like), or ethers (e.g., tetrahydrofuran, diethyl ether, dioxane, 1,2-dimethoxyethane or the like) may be used.
The reaction may be performed at -20 to 50°C for 0.5 to 48 hours, for example, in the presence of bromine. When brominated, a compound(VII-1) may be reacted with NBS in the presence of a catalytic amount of AIBN.

### Thirtieth step

Thirtieth step is a step of reacting a compound represented by the formula (VII-2) with triphenylphosphine to produce a compound represented by the formula (VII-3).
As a solvent, a solvent described in second step can be used. Preferably, nitriles (e.g., acetonitrile or the like), or ethers (e.g., tetrahydrofuran, diethyl ether, dioxane, 1,2-dimethoxyethane or the like) may be used.
The reaction may be performed at 20 to a temperature at which a solvent used is refluxed for 0.5 to 48 hours in the presence of triphenylphosphine.

### Thirty-first step

Thirty-first step is a step of producing a compound represented by the formula (VII-4) from a compound represented by the formula (VII-3).
As a solvent, a solvent described in second step can be used. Preferably, aromatic hydrocarbons (e.g., toluene, benzene, xylene or the like) may be used.
As a base, a base described in second step can be used. Preferably, metal carbonate (e.g., sodium carbonate, calcium carbonate, cesium carbonate, potassium carbonate or the like), or metal hydroxide (e.g., sodium hydroxide, potassium hydroxide, lithium hydroxide, barium hydroxide or the like) may be used.
The reaction may be performed at -20 to 50°C for 0.5 to 24 hours.

### Thirty-second step

Thirty-second step is a step of converting a compound represented by the formula (VII-4) into a compound represented by the formula (VII-5) by a Wittig reaction.
As a solvent, a solvent described in second step can be used. Preferably, ethers (e.g., tetrahydrofuran, diethyl ether, dioxane, 1,2-dimethoxyethane or the like) may be used.
The reaction may be performed at 20°C to a temperature at which a solvent used is refluxed for 0.5 to 24 hours in the presence of paraformaldehyde.

### Thirty-third step

Thirty-third step is a step of hydrolyzing a compound represented by the formula (VII-5) to produce a compound represented by the formula (VII-6).
The reaction may be performed under the same conditions as the above fourth step.

### Thirty-fourth step

Thirty-fourth step is a step of reacting a compound represented by the formula (VII-6) with a compound represented by KXCN to produce a compound represented by the formula (VII-7).
The reaction may be performed under the same conditions as the above fifteenth step using KSCN or KOCN.

### Thirty-fifth step

Thirty-fifth step is a step of reacting a compound represented by the formula (VII-7) with a compound represented by the formula (II-8) to produce a compound represented by the formula (I-J).
The reaction may be performed under the same conditions as the above sixteenth step.

### Thirty-sixth step

Thirty-sixth step is a step of converting a compound represented by the formula (I-J) into a compound represented by the formula (I-K) by an oxidation reaction.
The reaction may be performed under the same conditions as the above seventeenth step.

### Thirty-seventh step

Thirty-seventh step is a step of converting a compound represented by the formula (I-K) into a compound represented by the formula (I-J').
The reaction may be performed under the same conditions as the above eighteenth step.

Various substituents in the present compound can be introduced by referring to (1) Alan R. Katriszly et al., Comprehensive Heterocyclic Chemistry (2) Alan R. Katriszly et al., Comprehensive Heterocyclic Chemistry II (3) RODD'S

### CHEMISTRY OF CARBON COMPOUNDS VOLUME IV HETEROCYCLIC COMPOUNDS or the like.

The present compound has excellent inhibitory activity on 11β-hydroxysteroid dehydrogenase type I. Therefore, it can be used for treatment or prevention of a disease concerning 11β-hydroxysteroid dehydrogenase type I, especially, disease such as hyperlipidemia, diabetes, obesity, arteriosclerosis, atherosclerosis, hyperglycemia, and/or syndrome X. Particularly, it is useful in treatment or prevention of diabetes.

A compound used in the present invention can be orally or parenterally administered. When administered orally, the compound used in the present invention can be used in any dose form including normal formulations, for example, solid formulations such as a tablet, powder, granule, capsule or the like; aqueous formulations oleaginous suspensions; or liquid formulations such as syrup or elixir. When administered parenterally, the compound used in the present invention can be used as an aqueous or oleaginous suspension for injection or nasal solution. In preparation of such formulations, a conventional excipient, binder, lubricant, aqueous solvent, oleaginous solvent, emulsifying agent, suspending agent, preservative, stabilizer and the like can be optionally used. Especially, using in a form of an oral formulation is preferred.
A formulation of the compound used in the present invention can be produced by combining (e.g., mixing) a therapeutically effective amount of the compound used in the present invention with a pharmaceutically acceptable carrier or diluent. Formulation of the compound used in the present invention can be produced by a known method using a well-known easily available ingredient.
A dose of the compound used in the present invention is different depending on an administration method, an age, a weight and the condition of a patient, and a kind of a disease and, in the case of oral administration, usually about 0.05 mg to 3000 mg, preferably about 0.1 mg to 1000 mg per a day for adult person may be administered, if necessary, in divided doses. In addition, in the case of parenteral administration, about 0.01 mg to 1000 mg, preferably about 0.05 mg to 500 mg per a day for adult person may be administered. In administration, it can be used together with other therapeutic agents.

In the following, the present invention will be described in more detail by way of examples which are not intended to limit the scope of the present invention.

### [Example 1]

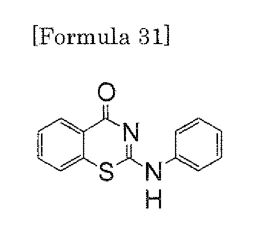

### Compound (I-1);

To a mixture of 2.00 g (11.9 mmole) of methyl thiosalicylate, 2.49 ml (17.8 mmole) of triethylamine and acetone was added dropwise a mixture of 1.26 g (11.9 mmole) of cyanogen bromide and 7 ml of acetone under ice-cooling, and the mixture was stirred at room temperature for 16.5 hours. A solvent of the reaction solution was concentrated under reduced pressure and methylene chloride and water were added to the residue, and extracted. The organic layer was washed with brine, dehydrated with anhydrous sodium sulfate, and filtered. The filtrate was concentrated under reduced pressure, and the residue was purified by silicagel columnchromatography (n-hexane : ethyl acetate = 3 : 1) to give a pale yellow crystal.
A mixture of 100 mg of the crystal, 48.2 mg of aniline, 13.2 mg of benzoic acid and 2 ml of acetone was stirred at room temperature for 17 hours. A solvent of the reaction solution was concentrated under reduced pressure, and the residue was purified by silicagel columnchromatography (n-hexane : ethyl acetate = 3 : 1) to give 132 mg (93%) of a title compound as a colorless crystal. 1H NMR(CDCl3) : δ(ppm)4.54(1H, s(br), NH), 6.70(2H, d, J = 7.5 Hz), 6.77(1H, tt, J = 7.2 Hz, J = 1.1 Hz), 7.13-7.19(3H, m), 7.47(1H, t, J = 7.5 Hz), 7.61(1H, t, J = 7.5 Hz), 8.11(1H, d, J = 7.5 Hz)

### (Reference Example 1)

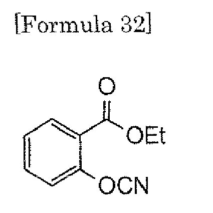

### Compound (I'-1):

To a mixture of 1.5 g (9.03 mmole) of ethyl salicylate, 0.96 g (9.03 mmole) of triethylamine and 30 ml of acetone was added dropwise a solution of 0.96 g of cyanogen bromide in acetone (5 ml) under ice-cooling, and the mixture was stirred at room temperature for 18 hours. The reaction solution was concentrated under reduced pressure, and water and saturated NaHCO₃ were added to the residue, and extracted with ethyl acetate. The organic layer was washed with brine, dehydrated with anhydrous sodium sulfate, and filtered, and the solvent was concentrated under reduced pressure. The residue was purified by silicagel columnchromatography (n-hexane : ethyl acetate = 4 : 1) to give 0.73 g (42%) of a title compound as a yellow liquid.
1H-NR(CDCl3): δ(ppm) 1.42 (3H, t, J=7.0Hz), 4.43 (2H, q, J=7.0Hz), 7.42 (1H, dt, J=7.0, 1.0Hz), 7.57 (1H, dd, J=9.0, 1.0Hz), 7.66 (1H, dt, J=7.5, 2.0Hz), 8.02 (1H, dd, J=7.5, 2.0Hz)

### [Example 2]

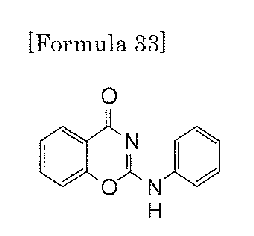

### Compound (I-2):

A mixture of 0.72 g (3.77 mmole) of the compound (I'-1), 0.35 g (3.77 mmole) of aniline, 0.46 g (3.77 mmole) of benzoic acid and 4 ml of acetone was stirred at room temperature for 3.5 hours. The insoluble was removed, and the filtrate was concentrated under reduced pressure, washed with acetone to give 0.62 g (69%) of a title compound as a colorless crystal.
1H-NMR(DMSO-d6): δ(ppm) 7.16 (1H, t, J=7.5Hz), 7.38-7.46 (4H, m), 7.60-7.79 (3H, m), 7.95 (1H, dd, J=7.5, 1.0z).

### [Example 3]

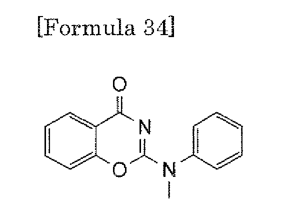

### Compound (I-3):

A mixture of 0.10 g (0.42 mmole) of the compound (1-2), 32 mg (0.47 mmole) of sodium ethoxide, 62 mg (0.44 mmole) of methyl iodide and 4.2 ml of ethanol was stirred at room temperature for 2 days. To the reaction solution were added water and ethyl acetate, this was extracted, and the organic layer was washed with brine. The organic layer was dried with anhydrous sodium sulfate, and filtered, the solvent was concentrated under reduced pressure, and the residue was purified by silicagel columnchromatography (n-hexane : ethyl acetate = 3 : 1) to give 0.01 g (9%) of a title compound as a yellow liquid.
1H-NMR(CDCl3); δ(ppm) 3.59 (3H, s), 7.03-7.15 (3H, m), 7.27 (1H, dt, J=8.0, 1.0Hz), 7.34 (3H, t, J=8.5Hz), 7.54-7.60 (1H, m), 8.07 (1H, dd, J=8.0, 1.5Hz)

### (Reference Example 2)

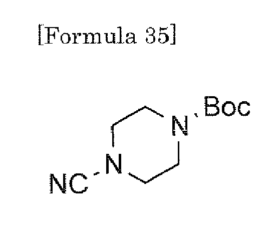

### Compound (I'-2):

A mixture of 0.30 g (1.61 mmole) of 1-tert-butoxycarbonylpiperazine, 0.33 g (2.42 mmole) of potassium carbonate, 0.20 g (1.93 mmole) of cyanogen bromide and 12 ml of acetone was stirred at room temperature for 1 hour. The reaction solution was concentrated under reduced pressure, and water and ethyl acetate were added to the residue, and extracted. The organic layer was washed with brine, dehydrated with anhydrous sodium sulfate, and filtered, and the filtrate was concentrated under reduced pressure to give 0.33 g (100%) of a title compound as a yellow crystal.
1H-NMR(CDCl3): δ(ppm) 1.46 (9H, s), 3.20 (4H, t, J=5.0Hz), 3.52 (4H, t, J=5.5Hz)

The following compounds were synthesized as in Reference Example 2.

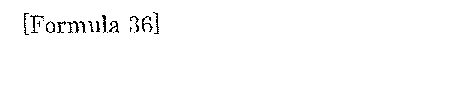

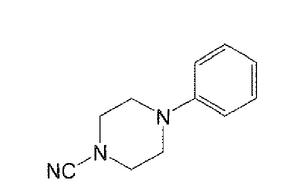

### Compound (I'-3):

Yield: 98%
1H-NMR(CDCl3): δ(ppm) 3.23 (4H, t, J=5.0Hz), 3.39 (4H, t, J=5.0Hz), 6.90-6.97 (3H, m), 7.26-7.33 (2H, m)

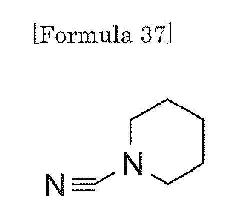

### [Compound I'-4]:

Yield: 54%
1H-NMR(CDCl3): δ(ppm) 1.53-1.69 (6H, m), 3.18 (4H, t, J=5.0Hz)

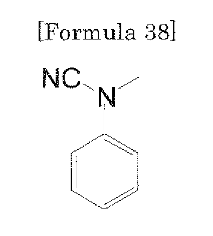

### Compound (I'-5):

Yield: 54%
1H NMR(CDCl3) : δ(ppm) 3.33(3H, s), 7.08-7.13(3H, m), 7.39(2H, t, J = 8.0 Hz)

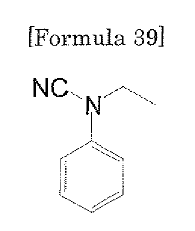

### Compound (I'-6):

Yield: 51%
1H NMR(CDCl3) δ(ppm) 1.46(3H, t, J = 7.2 Hz), 3.64(2H, q, J = 7.3 Hz), 7.07-7.14(3H, m), 7.37(2H, t, J = 8.1 Hz)

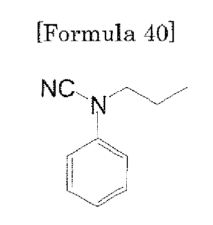

### Compound (I'-7):

Yield: 63%
1H NMR(CDCl3) : δ(ppm) 1.06(3H, t, J = 7.5 Hz), 1.87(2H, sextet, J = 7.5 Hz), 3.56(2H, t, J = 7.5 Hz), 7.07-7.14(3H, m), 7.35-7.40(2H, m)

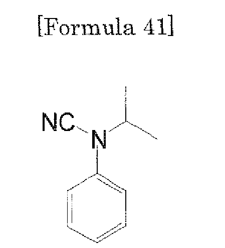

### Compound (I'-8):

Yield: 52%
1H NMR(CDCl3): δ(ppm) 1.43(6H, d, J = 6.3 Hz), 4.20(1H, septet, J = 6.6 Hz), 7.07-7.17(3H, m), 7.37(2H, t, J = 8.1 Hz)

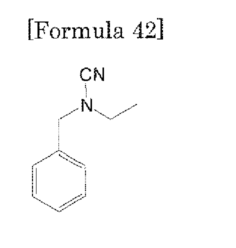

### Compound (I'-9):

Yield: 43%
1H NMR(CDCl3) : δ(ppm) 1.26(3H, t, J = 7.2 Hz), 2.89(2H, q, J = 7.2 Hz), 4.20(2H, s), 7.33-7.38(5H, m).

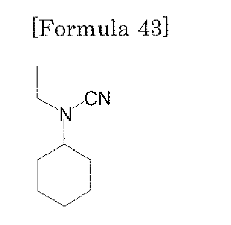

### Compound (I'-10):

Yield: 54%
1H NMR(CDCl3): δ(ppm) 1.13-1.49(7H, m), 1.59-1.66(2H, m), 1.82(2H, d, J = 12.9 Hz), 1.95(2H, d, J = 12.3 Hz), 2.73(1H, tt, J = 11.1 Hz, J = 3.6 Hz), 3.06(2H, q, J = 11.0 Hz)

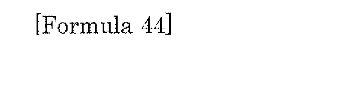

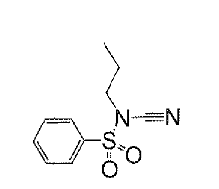

### Compound (I'-11):

Yield: 51%
1H NMR(CDCl3): δ(ppm) 0.93(3H, t, J = 7.5 Hz), 1.70(2H, sextet, J = 7.5 Hz), 3.37(2H, t, J = 7.5 Hz), 7.64(2H, tt, J = 7.2 Hz, J = 1.2 Hz), 7.76(1H, tt, J = 7.2Hz, J = 1.2 Hz), 7.98(2H, d, J = 7.2Hz)

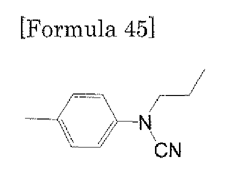

### Compound (I'-12):

Yield: 38%
1H NMR(CDCl3) : δ(ppm) 1.05(3H, t, J = 7.5 Hz), 1.84(2H, sextet, J = 7.5 Hz), 2.32(3H, s), 3.51(2H, t, J = 7.5 Hz), 7.01(2H, d, J = 8.7 Hz), 7.16(2H, d, J = 8.7Hz)

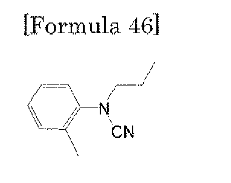

### Compound (I'-13):

Yield: 46%
1H NMR(CDCl3) : δ(ppm) 1.02(3H, t, J = 7.5 Hz), 1.78(2H, sextet, J = 7.5 Hz), 2.43(3H, s), 3.39(2H, t, J = 7.5 Hz), 7.21-7.26(4H, m)

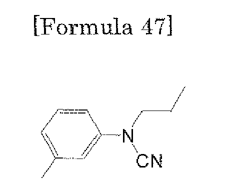

### Compound (I'-14):

Yield: 47%
1H NMR(CDCl3) : δ(ppm) 1.06(3H, t, J = 7.5 Hz), 1.85(2H, sextet, J = 7.5 Hz), 2.36(3H, s), 3.54(2H, t, J = 7.5 Hz), 6.87-6.96(3H, m), 7.12-7.29(1H, m)

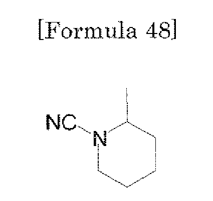

### Compound (I'-15):

Yield: 100%
1H NMR(CDCl3): δ(ppm) 1.26-1.45(4H, m), 1.58-1.71(4H, m), 1.80-1.88(1H, m), 2.97-3.07(2H, m), 3.40-3.47(1H, m)

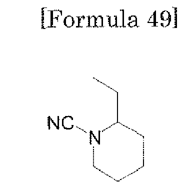

### Compound (I'-16):

Yield: 100% 1H NMR(CDCl3): δ(ppm) 0.99(3H, t, J = 7.5 Hz), 1.24-1.87(8H, m), 2.74-2.82(1H, m). 2.97-3.06(1H, m), 3.39-3.47(1H, m)

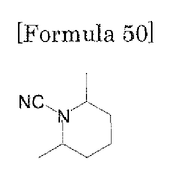

### Compound (I'-17):

Yield: 100%
1H NMR(CDCl3) : δ(ppm) 1.18-1.46(8H, m), 1.59-1.68(4H, m), 1.79-1.86(1H, m), 2.95-3.03(1H, m)

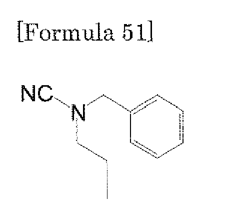

### Compound (I'-18):

Yield: 100%
1H N-MR(CDCl3) : δ(ppm) 0.95(3H, t, J = 7.4 Hz), 1.67(2H, sextet, J = 7.3 Hz), 2.90(2H, t, J = 7.4 Hz), 4.20(2H, s), 7.32-7.42(5H, m)

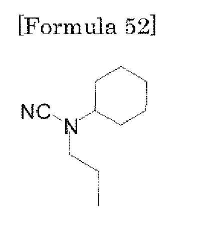

### Compound (I'-19):

Yield: 31%
1H NMR(CDCl3) : δ(ppm) 0.97(3H, t, J = 7.4 Hz), 1.13-1.49(4H, m), 1.55-1.65(2H, m), 1.67(2H, sextet, J = 7.3 Hz), 1.80-1.87(2H, m), 1.92-2.00(2H, m), 2.70(1H, tt, J = 11.4 Hz, J = 3.9 Hz), 2.97(2H, t, J = 7.4Hz)

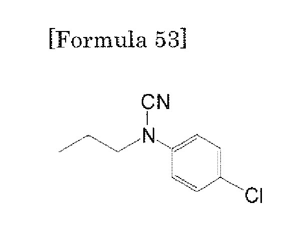

### Compound (I'-20):

Yield: 39%
1H NMR(CDCl3) : δ(ppm) 1.06(3H, t, J = 7.4 Hz), 1.85(2H, sextet, J = 7.3 Hz), 3.53(2H, t, J = 7.4 Hz), 7.05(2H, d, J = 8.7 Hz), 7.34(2H, d, J = 8.4 Hz)

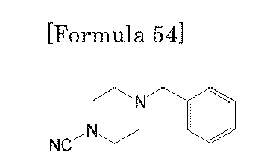

### Compound (I'-21):

Yield: 100%
1H NMR(CDCl3) : δ(ppm) 2.51(4H, t, J = 5.1 Hz), 3.25(4H, t, J = 5.0 Hz), 3.53(2H, s), 7.24-7.35(5H, m)

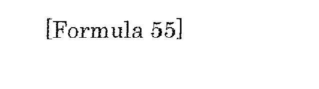

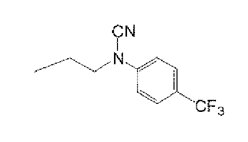

### Compound (I'-22):

Yield: 6%
1H NMR(CDCl3) : δ(ppm) 1.08(3H, t, J = 7.5 Hz), 189(2H, sextet, J = 7.3 Hz), 3.60(2H, t, J = 7.5 Hz), 7.20(2H, d, J = 8.7 Hz), 7.64(2H, d, J = 9.0 Hz)

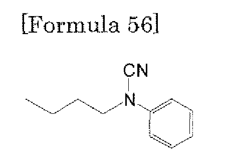

### Compound (I'-23):

Yield: 60%
1H NMR(CDCl3) : δ(ppm) 0.99(3H, t, J = 7.4 Hz), 1.48(2H, sextet, J = 7.5 Hz), 1.82(2H, quint, J = 7.4 Hz), 3.59(2H, t, J= 7.2 Hz), 7.07-7.17(3H, m), 7.35-7.40(2H, m),

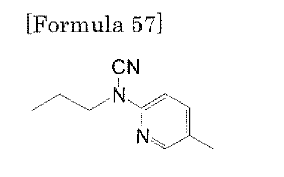

### Compound (I'-24):

Yield: 28%
1H NMR(CDCl3) : δ(ppm) 1.03(3H, t, J = 7.5 Hz), 1.83(2H, sextet, J = 7.3 Hz), 2.28(3H, s), 3.79(2H, t, J = 7.5 Hz), 7.10(1H, d, J = 8.7 Hz), 7.50(1H, dd, J = 8.4 Hz, J = 2.4 Hz), 8.12(1H, s)

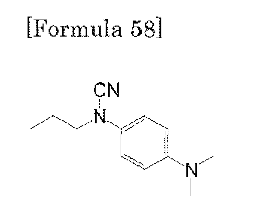

### Compound (I'-25):

Yield: 54%
1H NMR(CDCl3)50C : δ(ppm) 1.03(3H, t, J = 7.5 Hz), 1.80(2H, sextet, J = 7.2 Hz), 2.97(6H, s), 3.46(2H, t, J = 7.2 Hz), 6.73(2H, d, J = 9.0 Hz), 7.03(2H, d, J = 9.0 Hz)

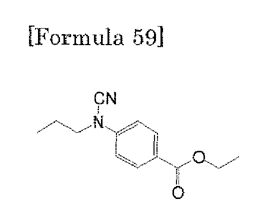

### Compound (I'-26):

Yield: 33%
1H NMR(CDCl3) : δ(ppm) 1.08(3H, t, J = 7.5 Hz), 1.39(3H, t, J = 7.2 Hz), 1.90(2H, sextet, J = 7.5 Hz), 3.61(2H, t, J = 7.5 Hz), 4.37(2H, q, J = 7.2 Hz), 7.15(2H, d, J = 8.7 Hz), 8.06(2H, d J = 8.4 Hz)

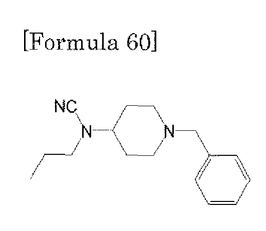

### Compound (I'-27):

Yield: 93%
1H NMR(CDCl3) : δ(ppm) 0.97(3H, t, J = 7.5 Hz), 1.28(2H, t, J = 7.5 Hz), 1.62-1.96(4H, m), 1.97-2.05(2H, m), 2.77(1H, tt, J = 11.1 Hz, J = 3.9 Hz), 2.90-3.08(4H, m), 3.50(2H, s), 7.23-7.32(5H, m)

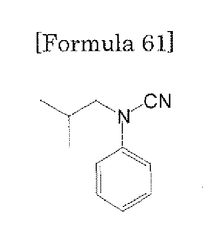

### Compound (I'-28):

Yield: 51%
1H NMR(CDCl3) : δ(ppm) 1.05(6H, d, J = 6.6 Hz), 2.20(1H, nonet, J = 6.7 Hz), 3.41(2H, d, J = 7.5 Hz), 7.07-7.14(3H, m), 7.34-7.40(2H, m)

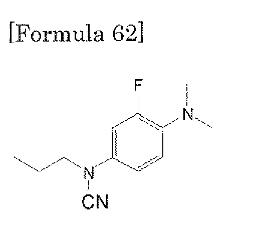

### Compound (I'-29):

Yield: 71%
1H NMR(CDCl3) : δ(ppm) 1.05(3H, t, J = 7.5 Hz), 1.83(2H, sextet, J = 7.2 Hz), 2.81(6H, s), 3.48(2H, t, J = 7.2 Hz), 6.80-6.93(3H, m)

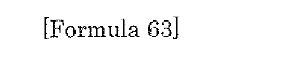

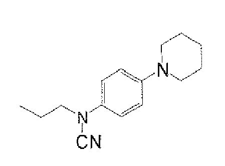

### Compound (I'-30):

Yield: 11%
1H NMR(CDCl3) : δ(ppm) 1.03(3H, t, J = 7.5 Hz), 1.53-1.60(2H, m), 1.67-1.88(6H, m), 3.10(4H, t, J= 5.4 Hz), 3.48(2H, t, J = 7.2 Hz), 6.92(2H, d, J = 9.3 Hz), 7.02(2H, d, J = 9.3 Hz)

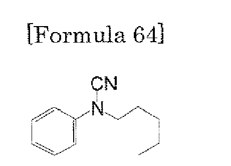

### Compound (I'-31):

Yield: 44%
1H NMR(CDCl3) : δ(ppm) 0.93(3H, t, J = 6.8 Hz), 1.32-1.50(4H, m), 1.84(2H, quintet, J = 6.9 Hz), 3.58(2H, t, J = 7.4 Hz), 7.07-7.14(3H, m), 7.38(2H, t, J = 7.5 Hz)

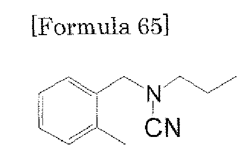

### Compound (I'-32):

Yield: 100%
1H NMR(CDCl3) : δ(ppm) 0.95(3H, t, J = 7.5 Hz), 1.69(2H, sextet, J = 7.2 Hz), 2.38(3H, s), 2.91(2H, t, J = 7.2 Hz), 4.20(2H, s), 7.19-7.30(4H, m)

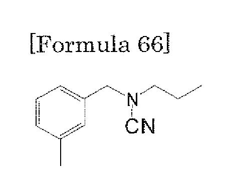

### Compound (I'-33):

Yield: 100%
1H NMR(CDCl3) : δ(ppm) 0.95(3H, t, J = 7.2 Hz), 1.67(2H, sextet, J = 7.2 Hz), 2.37(3H, s), 2.89(2H, t, J = 7.2 Hz), 4.16(2H, s), 7.11-7.17(3H, m), 7.24-7.30(1H, m)

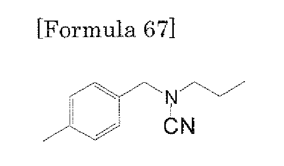

### Compound (I'-34):

Yield: 100%
1H NMR(CDCl3) : δ(ppm) 0.94(3H, t, J = 7.2 Hz), 1.66(2H, sextet, J = 7.2 Hz), 2.36(3H, s), 2.88(2H, t, J = 7.2 Hz), 4.11(2H, s), 7.17-7.28(4H, m)

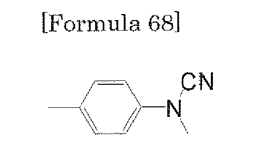

### Compound (I'-35):

Yield: 54%
1H NMR(CDCl3) : δ(ppm) 2.33(3H, s), 3.32(3H, s), 6.98(2H, d. J = 8.7 Hz), 7.18(2H, d. J = 8.7 Hz)

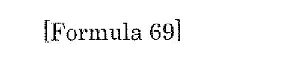

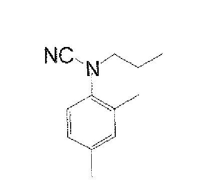

Compound (I'-36):

Yield: 41%
1H NMR(CDCl3): δ(ppm) 1.01(3H, t, J = 7.2 Hz), 1.76(2H, sextet, J = 7.2 Hz), 2.31(3H, s), 2.36(3H, s), 3.34(2H, t, J = 7.2 Hz), 6.99-7.06(2H, m), 7.11(1H, d, J = 8.1 Hz)

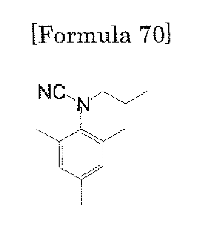

### Compound (I'-37):

Yield: 47%
1H NMR(CDCl3) : δ(ppm) 1.02(3H, t, J = 7.5 Hz), 1.83(2H, sextet, J = 7.5 Hz), 2.27(3H, s), 2.35(6H, s), 3.18(2H, t, J = 7.5 Hz), 6.90(2H, s)

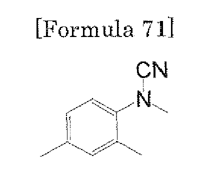

### Compound (I'-38):

Yield: 53%
1H NMR(CDCl3) : δ(ppm) 2.31(3H, s), 2.37(3H, s), 3.24(3H, s), 7.00-7.06(2H, m), 7.12(1H, d, J = 8.1 Hz)

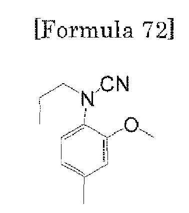

### Compound (I'-39):

Yield: 56%
1H NMR(CDCl3) : δ(ppm) 0.99(3H, t, J = 7.5 Hz), 1.70(2H, sextet, J = 7.5 Hz), 2.34(3H, s), 3.39(2H, t, J = 7.2 Hz), 3.87(3H, s), 6.72-6.78(2H, m), 7.11(1H, d, J = 8.4 Hz)

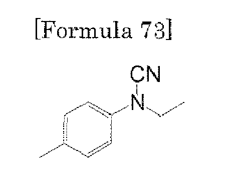

### Compound (I'-40):

Yield: 57%
1H NMR(CDCl3): δ(ppm) 1.44(3H, t, J = 7.2 Hz), 2.32(3H, s), 3.61(2H, q, J = 7.2 Hz), 7.01(2H, d, J= 8.4 Hz), 7.17(2H, d, J = 8.4 Hz)

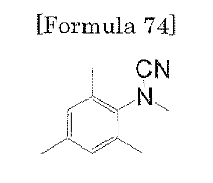

### Compound (I'-41):

Yield: 78%
1H NMR(CDCl3) : δ(ppm) 2.27(3H, s), 2.36(6H, s), 3.15(3H, s), 6.90(2H, s)

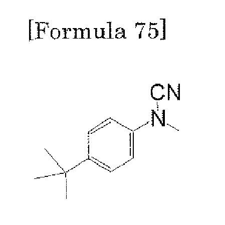

### Compound (I'-42):

Yield: 41%
1H NMR(CDCl3) : δ(ppm) 1.31(9H, s), 3.33(3H, s), 7.03(2H, d, J = 8.4 Hz), 7.39(2H, d, J = 8.4 Hz)

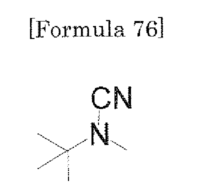

### Compound (I'-43):

Yield: 61%
1H NMR(CDCl3) : δ(ppm) 1.29(9H, s), 2.80(3H, s)

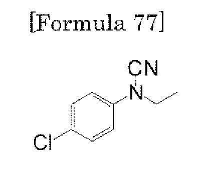

### Compound (I'-44):

Yield: 52%
1H NMR(CDCl3) : δ(ppm) 1.46(3H, t, J = 7.2 Hz), 3.61(2H, q, J = 7.2 Hz), 7.05(2H, d, J = 9.0 Hz), 7.34(2H, d, J = 9.0 Hz)

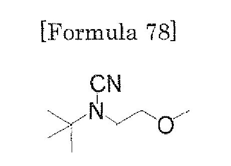

### Compound (I'-45):

Yield 94%
1H NMR(CDCl3) : δ(ppm) 1.31(9H, s), 3.15(2H, t, J = 5.7 Hz), 3.40(3H, s), 3.60(2H, t, J = 5.6 Hz)

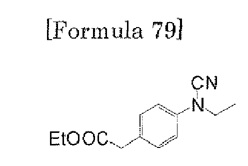

### Compound (I'-46):

Yield: 45%
1H NMR(CDCl3) : δ(ppm) 1.26(3H, t, J = 7.2 Hz), 1.45(3H, t, J = 7.2 Hz), 3.58(2H, s), 3.63(2H, q, J = 7.2 Hz), 4.15(2H, q, J = 7.2 Hz), 7.07(2H, d, J = 8.7 Hz), 7.29(2H, d J = 8.7 Hz)

### (Reference Example 3)

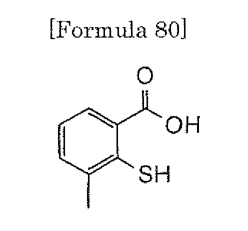

### Compound (I'-47):

To 8 ml of 6N aqueous HCl was slowly added dropwise a mixture of 2.00 g (13.2 mmole) of 3-methylanthranilic acid, 0.91 g (13.2 mmole) of sodium nitrite, 0.58 g (14.5 mmole) of sodium hydroxide, and 16 ml of water under ice-cooling, and the mixture was stirred for 30 minutes under ice-cooling. After the reaction solution was neutralize with sodium acetate, the reaction solution was added dropwise to a mixture of 6.58 g (41 mmole) of potassium O-ethyl dithiocarbonate and 20 ml of water at 80°C, and the mixture was stirred for 30 minutes. The reaction solution was returned to room temperature, and was acidified with concentrated hydrochloric acid, and the supernatant was removed by decantation. The residue was alkalified with 2N-NaOH, the insoluble was filtered, the filtrate was acidified with concentrated hydrochloric acid, and the precipitated crystal was filtered to give 1.44 g (65%) of a title compound as a yellow crystal.
1H-NMR(CDCl3): δ(ppm) 2.40 (3H, s), 6.43 (1H, d, J=8.0Hz), 7.10 (1H, t, J=7.5Hz), 7.36 (1H, d, J=7.5Hz), 8.06 (1H, d, J=7.5Hz), 12.01 (1H, br).

The following compounds were synthesized as in Reference Example 3.

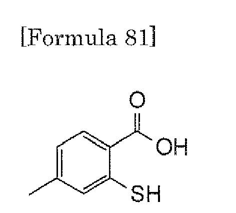

### Compound (I'-48):

Yield: 72%
1H-NMR(CDCl3): δ(ppm) 2.35 (3H, s), 6.20 (1H, br), 7.00 (1H, d, J=8.0Hz), 7.15 (1H, s), 8.03 (1H, d, J=8.0Hz), 11.90 (1H, br)

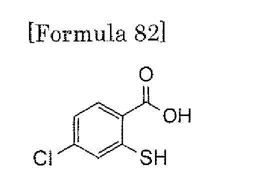

### Compound (I'-49):

Yield: 33%
1H-HMR(CDCl3): δ(ppm) 4.78 (1H, s), 7.17 (1H, dd, J=9.0Hz), 7.35 (1H, d, J=2.0Hz), 8.06 (1H, d, =9.0Hz), 12.03 (1H, br).

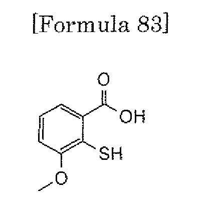

### Compound (I'-50):

Yield: 71%
1H-NMR(DMSO-d6): δ(ppm) 3.90 (3H, s), 5.62 (1H, br), 7.16-7.25 (2H, m), 7.58 (1H, dd, J=7.5, 2.0Hz), 13.2 (1H, br).

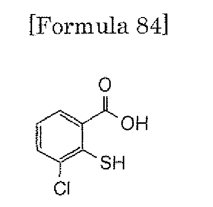

### Compound (I'-51):

Yield: 77%
1H-NMR(DMSO-d6): δ(ppm) 7.19 (1H, t, J=8.0Hz), 7.69 (1H, dd, J=8.0, 1.5Hz), 7.97 (1H, dd, J=8.0, 1.5Hz), 9.43 (1H, br) (SH : not detected)

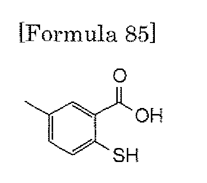

### Compound (I'-52):

Yield: 60%
1H-NMR(DMSO-d6): δ(ppm) 2.27 (3H, s), 5.24 (1H, br), 7.22 (1H, dd, J=8.0, 1.5Hz), 7.39 (1H, d, J=8.0Hz), 7.74 (1H, d, J=1.5Hz), 13.12 (1H, br)

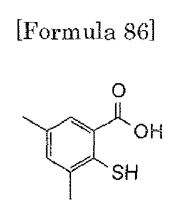

### Compound (I'-53):

Yield: 79%
1H-NMR(DMSO-d6): δ(ppm) 2.26 (3H, s), 2.33 (3H, s), 5.23 (1H, br), 7.11 (1H, s), 7.54 (1H, s), 12.09 (1H, br)

### (Reference Example 4)

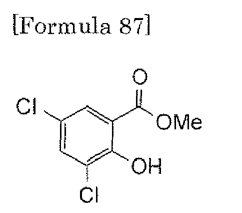

### Compound (I'-54):

A mixture of 1.5 g (7.25 mmole) of 3,5-dichloro salicylic acid, 0.71 g of concentrated sulfuric acid and methanol was heated to reflux with stirring for 10 hours. The reaction solution was concentrated under reduced pressure, and was alkalified with saturated NaHCO₃. The precipitated crystal was filtered to give 1.0 g (80%) of a title compound as a colorless crystal.
1H-NMR(CDCl3): δ(ppm) 3.96 (3H, s), 7.73 (1H, d, J=2.5Hz), 7.89 (1H, dd, J=2.5, 0.5Hz), 10.99 (1H, br).

The following compounds were synthesized as in Reference Example 4.

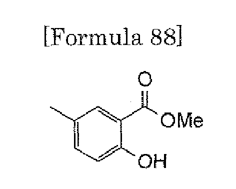

### Compound (I'-5):

Yield: 87%
1H-NMR(CDCl3): δ(ppm) 2.28 (3H, s), 3.94 (3H, s), 6.88 (1H, d, J=8.5Hz), 7.26 (1H, dd, J=8.5. 2.0Hz), 7.63 (1H, d, J=2.0Hz).

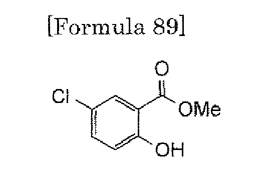

### Compound (I'-56):

Yield: 100%
1H-NMR(CDCl3): δ(ppm) 3.98 (3H, s), 6.94 (1H, d, J=9.0Hz), 7.40 (1H, dd, J=9.0, 2.5Hz), 7.81 (1H, d, J=2.5Hz).

### (Reference Example 5)

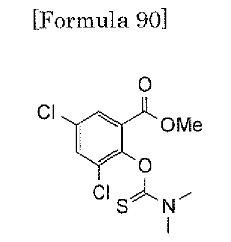

### Compound (I'-57):

A mixture of 1.0 g (4.52 mmole) of the compound (I'-54), 0.56 g (4.53 mmole) of N,N-dimethylthiocarbamoyl chloride, 0.52 g (4.53 mole) of 1,4-diazabicyclo[2.2.2]octane and 7.5 ml of N,N-dimethylformamide was stirred at room temperature for 2 days. To the reaction solution was added water, and extracted with ethyl acetate. The organic layer was washed with brine, dehydrated with anhydrous sodium sulfate, and filtered, and the solvent was concentrated under reduced pressure. The residue was purified by silicagel columnehromatography (n-hexane : ethyl acetate = 3:1) to give 0.54 g (39%) of a title compound as a yellow liquid.
1H-NMR(CDCl3): δ(ppm) 3.42 (3H, s), 3.47 (3H, s), 3.86 (3H, s), 7.63 (1H, d, J=2.5Hz), 7.90 (1H, d, J=2.5Hz).

The following compounds were synthesized as in Reference Example 5.

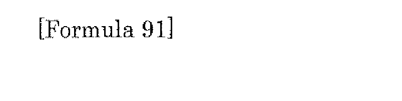

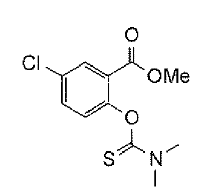

### Compound (I'-58):

Yield: 64%
1H-NMR(CDCl3): δ(ppm) 3.39 (3H, s), 3.46 (3H, s), 3.85 (3H, s), 7.07 (1H, d, J=9.0Hz), 7.52 (1H, dd, J=9.0, 2.5Hz), 7.98 (1H, d, J=2.5Hz).

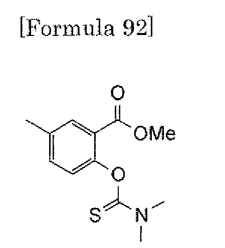

### Compound (I'-59):

Yield: 50%
1H-NMR(CDCl3): δ(ppm) 2.39 (3H, s), 3.40 (3H, s), 3.47 (3H, s), 3.83 (3H, s), 7.01(1H, d, J=8.0Hz), 7.37 (1H, ddd, J=8.0, 2.0, 0.5Hz), 7.81 (1H, d, J=2.0Hz).

### (Reference Example 6)

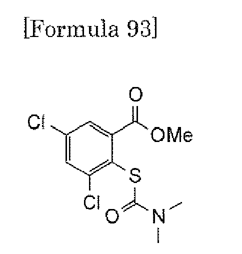

### Compound (I'-60):

The compound (I'-57) (0.50 g) was stirred at 200°C for 12 hours. The reaction solution was cooled with air, and purified by silicagel columnchromatography (n-hexane : ethyl acetate = 3 : 1) to give 0.46 g (92%) of a title compound as a pale yellow crystal.
1H-NMR(CDCl3): δ(ppm) 3.02 (3H, br), 3.15 (3H, br), 3.92 (3H, s), 7.63 (1H, d, J=2.5Hz), 7.69 (1H, d, J=2.5Hz).

The following compounds were synthesized as in Reference Example 6.

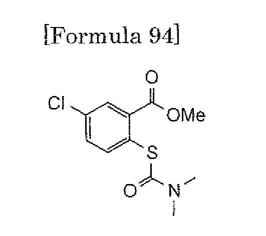

### Compound (I'-61):

Yield: 65%
1H-NMR(CDCl3): δ(pom) 3.02 (3H, s), 3.12 (3H, s), 3.89 (3H, s), 7.45 (1H, dd, J=8.5, 2.5Hz), 7.54 (1H, d, J=8.5Hz), 7.88 (1H, d, J=2.0Hz).

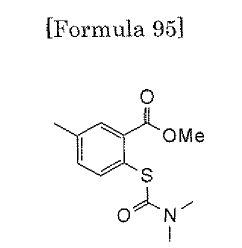

### Compound (I'-62):

Yield: 53%
1H-NMR(CDCl3): δ(ppm) 2.39 (3H, s), 3.02 (3H, br), 3.11 (3H, br), 3.88 (3H, s), 7.29 (1H, ddd, J=7.5, 2.0, 1.0Hz), 7.48 (1H, d, J=7.5Hz), 7.70 (1H, dd, J=1.5, 0.5Hz).

### (Reference Example 7)

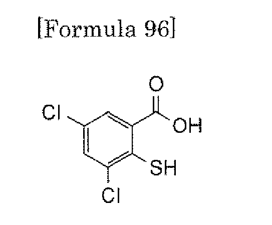

### Compound (I'-63):

A mixture of 0.40 g (1.30 mmole) of the compound (I'-61) and a 2N-aqueous NaOH was heated to reflux with stirring for 4 hours. After cooled with the air, the reaction mixture was acidified with 2N aqueous HCl, and the precipitated crystal was filtered to give 0.26 g (90%) of a title compound as a colorless crystal.
1H-NMR(CDCl3): δ(ppm) 2.40 (1H, br), 6.18 (1H, br), 7.62 (1H, d, J=2.5Hz), 8.07 (1H, d, J=2.5Hz)

The following compounds were synthesized as in Reference Example 7.

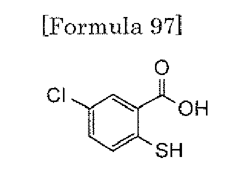

### Compound (I'-64):

Yield: 90%
1H-NMR(DMSO-d6): δ(ppm) 6.42 (1H, s), 7.45 (1H, dd, J=8.5, 2.5Hz), 7.55 (1H, d, J=8.5Hz), 7.87 (1H, d, J=2.5Hz), 12.00 (1H, br).

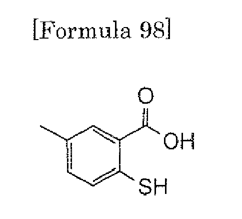

### Compound (I'-65):

Yield: 89%
1H-NMR(CDCl3): δ(ppm) 2.35 (3H, s), 4.55 (1H, s), 7.20-7.25 (2H, m), 7.95 (1H, s), 12.01 (1H, br).

The following compounds were synthesized as in Reference Example 4.

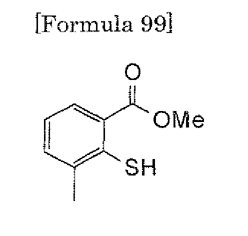

### Compound (I'-66):

Yield: 66%
1H-NMR(CDCl3): δ(ppm) 2.38 (3H, s), 3.93 (3H, s), 6.56 (1H, s), 7.05 (1H, t, J=7.5Hz), 7.29 (1H, d, J=7.5Hz), 7.89 (1H, d, J=7.5Hz).

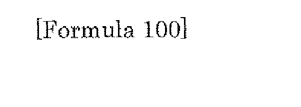

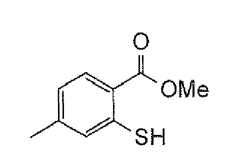

### Compound (I'-67):

Yield: 28%
1H-NMR(CDCl3): δ(ppm) 2.32 (3H, s), 3.90 (3H, s), 4.74 (1H, s), 6.96 (1H, dd, J=8.0, 1.0Hz), 7.12 1H, s), 7.91 (1H, d, J=8.0Hz).

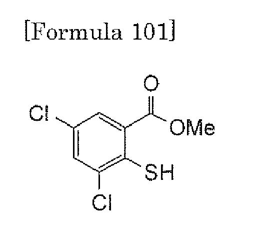

### Compound (I'-68):

Yield: 43%
1H-NMR(CDCl3): δ(ppm) 3.95 (3H, s), 6.25 (1H, s), 7.57 (1H, d, J=2.5Hz), 7.97 (1H, d, J=2.5Hz).

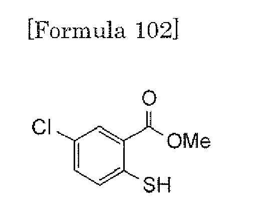

### Compound (I'-69):

Yield: 76%
1H-NMR(CDCl3): δ(ppm) 3.94 (3H, s), 6.26 (1H, s), 7.37 (1H, dd, J=8.0, 2.0Hz), 7.64 (1H, d, J=8.0Hz), 8.04 (1H, d, J=2.0Hz).

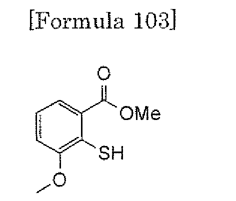

### Compound (I'-70):

Yield: 29%
1H-NMR(CDCl3): δ(ppm) 3.93 (3H, s), 3.94(3H, s), 5.31 (1H, s), 7.01 (1H, dd, J=8.0, 1.0Hz), 7.11 (1H, t, J=8.0Hz), 7.66 (1H, dd, J=8.0, 1.0Hz).

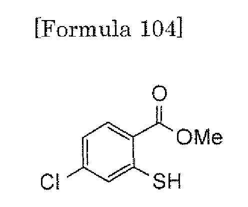

### Compound (I'-71):

Yield 66%
1H-NMR(CDCl3): δ(ppm) 3.90 (3H, s), 6.26 (1H, s), 7.09 (1H, d, J=1.0Hz), 7.54 (1H, dd, J=8.0, 1.0Hz), 7.96 1H, d, J=8.0Hz)

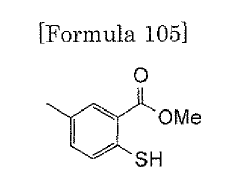

### Compound (I'-72):

Yield: 42%
1H-NMR(CDCl3): δ(ppm) 2.32 (3H, s), 3.92 (3H, s), 4.58 (1H, s), 7.14 (1H, dd, J=8.0, 1.5Hz), 7.20 (1H, d, J=8.0Hz), 7.83 (1H, d, J=1.5Hz)

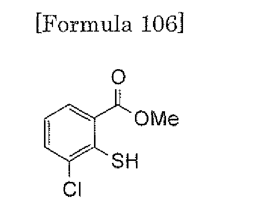

### Compound (I'-73):

Yield: 66%
1H-NMR(CDCl3): δ(ppm) 3.93 (3H, s), 6.25 (1H, s), 7.09 (1H, t, J=8.0Hz), 7.54 (1H, dd, J=8.0, 1.5Hz), 7.96 (1H, dd, J=8.0, 1.5, 1.0Hz)

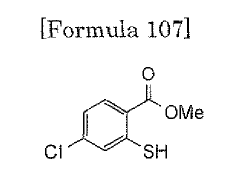

### Compound (I'-74):

Yield: 36%
1H-NMR(CDCl3): δ(ppm) 4.01 (3H, s), 6.25 (1H, s), 7.23 1H, dd, J=8.0, 2.0Hz), 7.70 (1H, d, J=2.0Hz), 8.01 (1H, d, J=8.5Hz)

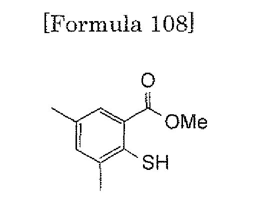

### Compound (I'-75):

Yield: 69%
1H-NMR(CDCl3): δ(ppm) 2.29 (3H, s), 2.34 (3H, s), 3.90 (3H, s), 6.29 (1H, s), 7.13 (1H, s), 7.70 (1H, s)

### [Example 4]

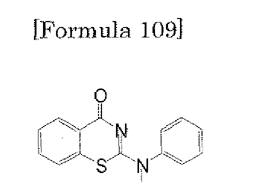

### Compound (1-4):

A mixture of 0.39 g (2.97 mmole) of the compound (I'-5), 0.50 g (2.97 mmole) of methyl thiosalicylate, 0.62 ml (4.46 mmole) of triethylamine and 5 ml of toluene was heated to reflux with stirring for 12 hours. To the reaction solution were added water and ethyl acetate, and extracted. The organic layer was washed with brine, dried over anhydrous sodium sulfate, and filtered, and the solvent was concentrated under reduced pressure. The residue was purified by silicagel columnchromatography (n-hexane : ethyl acetate = 1 : 3) to give 0.20 g (25%) of a title compound as a pale brown solid.
1H NMR(CDCl3) : δ(ppm) 3.63(3H, s), 7.14(1H, d, J = 7.2 Hz), 7.34-7.59(7H, m), 8.47(1H, d, J = 7.5 Hz)

The following compounds were synthesized as in Example 4.

### [Example 5]

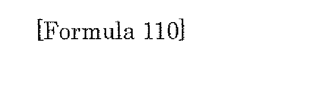

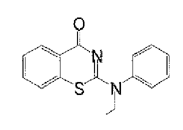

### Compound (I-5):

Yield: 53%
1H NMR(CDCl3): δ(ppm) 1.27(3H, t, J = 7.1 Hz), 4.19(2H, q, J = 7.1 Hz), 7.12(1H, d, J = 7.5 Hz), 7.31-7.34(2H, m), 7.38-7.47(2H, m), 7.53-7.58(3H, m), 8.46(1H, d, J = 6.9 Hz)

### [Example 6]

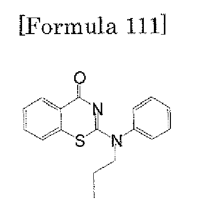

### Compound (I-6):

Yield: 3%
1H NMR(CDCl3) : δ(ppm) 0.95(3H, t, J = 7.5 Hz), 1.69(2H, sextet, J = 7.7 Hz), 4.10(2H, t, J = 7.7 Hz), 7.12(1H, d, J = 6.9 Hz), 7.31-7.34(2H, m), 7.39-7.46(2H, m), 7.52-7.56(3H, m), 8.45(1H, d, J = 6.9 Hz)

### [Example 7]

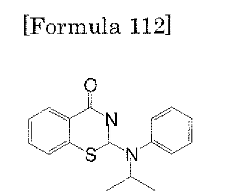

### Compound (I-7):

Yield: 22%
1H NMR(CDCl3) : δ(ppm) 1.17(6H, d, J = 6.9 Hz), 5.73(1H, septet, J = 6.7 Hz), 7.10(1H, d, J = 6.9 Hz), 7.25-7.30(2H, m), 7.36-7.49(2H, m), 7.53-7.59(3H, m), 8.45(1H, d, J = 7.2Hz)

### [Example 8]

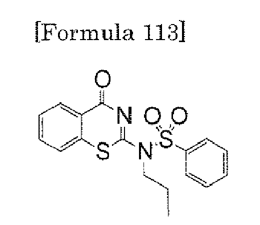

### Compound (I-8):

Yield: 60%
1H NMR(CDCl3): δ(ppm) 1.01(3H, t, J = 7.5 Hz), 1.86(2H, sextet, J = 7.5 Hz), 4.08(2H, t, J = 7.5 Hz), 7.44(1H, d, J = 7.8 Hz), 7.50-7.64(5H, m), 8.05(2H, d, J = 7.8 Hz), 8.40(1H, d, J = 7.8Hz)

### [Example 9]

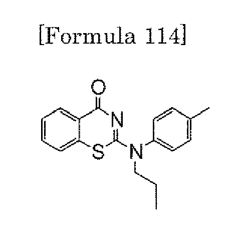

### Compound (I-9):

Yield: 23%
1H NMH(CDCl3) : δ(ppm) 0.94(3H, t, J = 7.5 Hz), 1.67(2H, sextet, J = 7.5 Hz), 2.47(3H, s), 4.07(2H, t, J = 7.5 Hz), 7.12(1H, d, J = 7.8 Hz), 7.19(2H, d, J = 7.8 Hz), 7.34(2H, d, J = 7.8 Hz), 7.39-7.46(2H, m), 8.45(1H, d, J = 7.8 Hz)

### [Example 10]

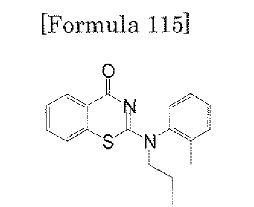

### Compound (I-10):

Yield: 54%
1H NMR(CDCl3) : δ(ppm) 0.95(3H, t, J = 7.5 Hz), 1.53-1.82(2H, m), 2.27(3H, s), 3.40-3.49(1H, m), 4.50-4.60(1H, m), 7.12(1H, d, J = 7.0 Hz), 7.20-7.47(6H, m), 8.47(1H, d, J = 7.0 Hz)

### [Example 11]

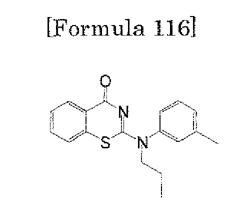

### Compound (I-11):

Yield: 25%
1H NMR(CDCl3) : δ(ppm) 0.95(3H, t, J = 7.5 Hz), 1.68(2H, sextet, J = 7.5 Hz), 2.44(3H, s), 4.08(2H, t, J = 7.5 Hz), 7.12-7.14(3H, m), 7.26-7.47(4H, m), 8.45(1H, d, J = 6.9 Hz)

### [Example 12]

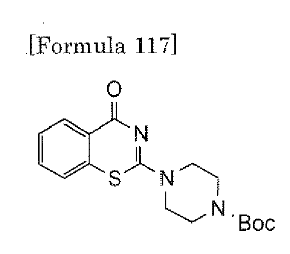

### Compound (I-12):

Yield: 50%
1H-NMR(CDCl3): δ(ppm) 1.49 (9H, s), 3.57 (4H, t, J=2.0Hz), 3.90 (4H, br), 7.35 (1H, dd, J=8.0, 1.0Hz), 7.44-7.57 (2H, m), 8.46 (1H, dd, J=7.5, 1.5Hz).

### [Example 13]

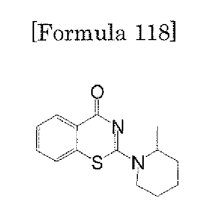

### Compound (I-13):

Yield: 42%
1H NMR(CDCl3) : δ(ppm) 1.32(3H, d, J = 7.2 Hz), 1.50-1.60(3H, m), 1.65-1.84(5H, m), 3.12(1H, t, J = 13.5 Hz), 7.32(1H, d, J = 7.5 Hz), 7.41-7.53(2H, m), 8.45(1H, d, J = 7.5Hz)

### [Example 14]

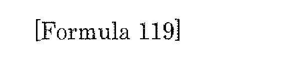

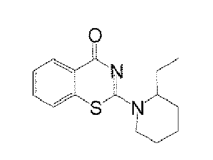

### Compound (I-14):

Yield 28%
1H NMR(CDCl3) : δ(ppm) 0.95(3H, t, J = 7.2 Hz), 1.40-1.61(5H, m), 1.64-1.95(6H, m), 7.32(1H, d, J = 8.1 Hz), 7.40-7.53(2H, m), 8.44(1H, d, J = 7.8Hz)

### [Example 15]

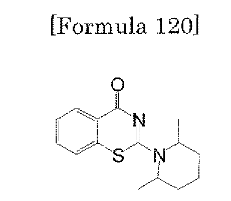

### Compound (I-15):

Yield: 82%
1H NMR(CDCl3) : δ(ppm) 1.20-1.98(14H, m), 7.32(1H, d, J = 7.8 Hz), 7.40-7.54(2H, m), 8.45(1H, d, J = 7.8Hz)

### [Example 16]

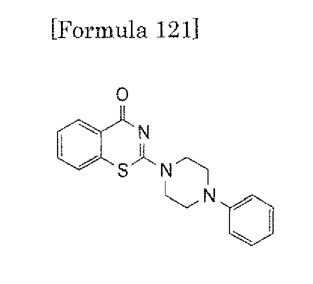

### Compound (I-16):

Yield: 10%
1H-NMR(CDCl3): δ(ppm) 3.31 (4H, t, J=5.5Hz), 4.07 (4H, br), 6.92-6.97 (3H, m), 7.27-7.38 (3H, m), 7.44-7.57 (2H, m), 8.47 (1H, dd, J=8.0, 1.5Hz).

### [Example 17]

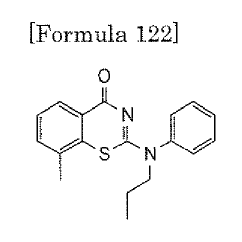

### Compound (I-17):

Yield: 13%
1H-NMR(CDCl3): δ(ppm) 0.95 (3H, t, J=7.0Hz), 1.62-1.75 (2H, m), 2.16 (3H, s), 4.10 (2H, t, J=7.5Hz), 7.30-7.34 (4H, m), 7.52-7.57 (3H, m), 8.35 (1H, t, J=5.0Hz).

### [Example 18]

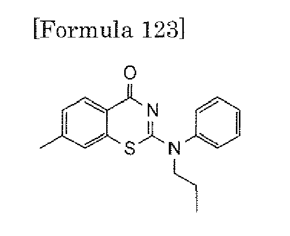

### Compound (I-18):

Yield: 10%
1H-NMR(CDCl3): δ(ppm) 0.95 (3H, t, J=7.5Hz), 1.64-1.74 (2H, m), 2.34 (3H, s), 4.06-4.13 (2H, m), 6.92 (1H, br), 7.20 (1H, dd, J=8.5, 1.0Hz), 7.30-7.33 (2H, m), 7.51-7.58 (3H, m), 8.88 (1H, d, J=8.0Hz).

### [Example 19]

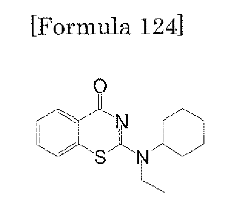

### Compound (I-19):

Yield: 82%
1H NMR(DMSO)100C : δ(ppm) 1.17-1.50(7H, m), 1.60-1.72(3H, m), 1.77-1.88(3H, m), 3.63(2H, q, J = 7.0 Hz), 4.32(1H, br), 7.44-7.62(3H, m), 8.19(1H, d, J = 7.8Hz)

### [Example 20]

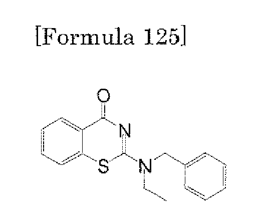

### Compound (I-20):

Yield: 22%
1H NMR(DMSO)100C : δ(ppm) 1.20(3H, t, J = 7.1 Hz), 3.68(2H, q, J = 7.1 Hz), 4.93(2H, s), 7.25-7.40(5H, m), 7.46-7.63(3H, m), 8.23(1H, d, J = 8.1Hz)

### [Example 21]

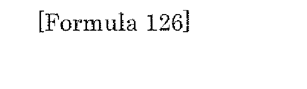

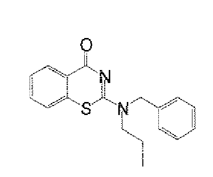

### Compound (I-21):

Yield: 79%
1H NMR(DMSO)100C : δ(ppm) 0.90(3H, t, J = 7.4 Hz), 1.67(2H, sextet, J = 7.5 Hz), 3.59(2H, t, J = 7.5 Hz), 4.94(2H, s), 7.25-7.38(5H, m), 7.46-7.63(3H, m), 8.21(1H, d, J = 7.8Hz)

### [Example 22]

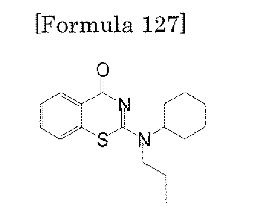

### Compound (I-22):

Yield: 27%
1H NMR(DMSO)100C : δ(ppm) 0.94(3H, t, J = 7.5 Hz), 1.15-1.49(3H, m), 1.58-1.87(9H, m), 3.49(2H, t, J = 8.1 Hz), 4.32(1H, br), 7.44-7.62(3H, m), 8.19(1H, d, J = 7.8Hz)

### [Example 23]

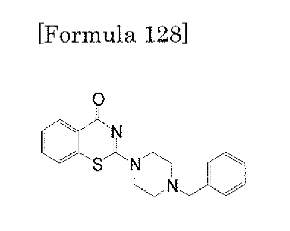

### Compound (I-23):

Yield: 81%
1H NMR(CDCl3): δ(ppm) 2.53(4H, t, J = 5.1 Hz), 3.56(2H, s), 3.81(4H, t, J = 5.3 Hz), 7.22-7.33(5H, m), 7.45-7.63(3H, m), 8.19(1H, d, J = 7.8Hz)

### [Example 24]

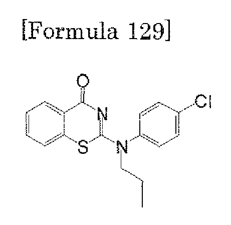

### Compound (I-24):

1H NMR(CDCl3) : δ(ppm) 0.95(3H, t, J = 7.5 Hz), 1.66(2H, sextet, J = 7.5 Hz), 4.07(2H, t, J = 7.8 Hz), 7.14(1H, d, J = 7.2 Hz), 7.25-7.30(2H, m), 7.39-7.49(2H, m), 7.52(2H, d, J = 8.7Hz), 8.45(1H, d, J = 7.5 Hz)

### [Example 25]

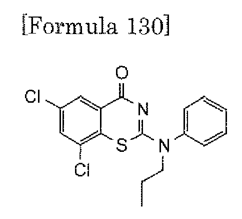

### Compound (I-25):

Yield: 34%
1H-NMR(CDCl3): δ(ppm) 0.95 (3H, t, J=7.5Hz), 1.66-1.3 (2H, m), 4.09 (2H, t, J=7.5Hz), 7.31-7.34 (2H, m), 7.52 1H, d, J=2.0Hz), 7.54-7.61 (3H, m), 8.41 1H, d, J=2.0Hz).

### [Example 26]

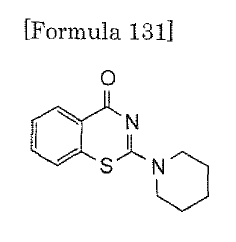

### Compound (I-26):

Yield: 56%
1H-NMR(CDCl3): δ(ppm) 1.67-1.73 (6H, m), 3.86 (4H, br), 7.32 (1H, dd, J=8.0, 1.5Hz), 7.40-7.54 (2H, m), 8.45 (1H, dd, J=7.5, 1.5Hz).

### [Example 27]

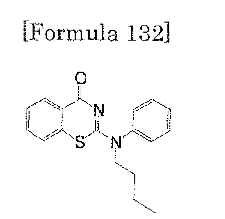

### Compound (I-27):

Yield: 42%
1H NMR(CDCl3) : δ(ppm) 0.90(3H, t, J = 7.5 Hz), 1.37(2H, sextet, J = 7.5 Hz), 1.63(2H, quintet, J = 7.5 Hz), 4.13(2H, t, J = 7.8 Hz), 7.12(1H, d, J = 7.2 Hz), 7.26-7.46(4H, m), 7.53-7.58(3H, m), 8.45(1H, d, J = 7.2Hz)

### [Example 28]

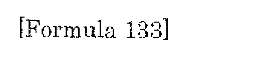

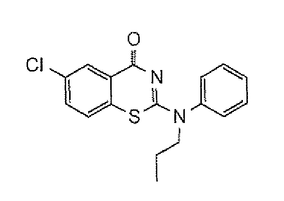

### Compound (I-28):

Yield: 4%
1H-NMR(CDCl3): δ(ppm) 0.95 (3H, t, J=7.5Hz), 1.67-1.73 (2H, m), 4.09 (2H, t, J=7.5Hz), 7.07 (1H, d, J=8.5Hz), 7.30-7.33 (2H, m), 7.41 (1H, dd, J=8.5, 2.5Hz), 7.53-7.58 (3H, m), 8.46 (1H, d, J=2.5Hz).

### [Example 29]

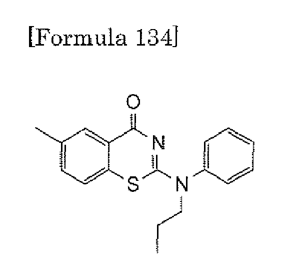

### Compound (I-29):

Yield: 31%
1H-NMR(CDCl3): δ(pom) 0.95 (3H, t, J=7.5Hz), 1.61-1.72 (2H, m), 2.40 (3H, s), 4.09 (2H, t, J=8.0Hz), 7.02 (1H, dd, J=8.5, 1.0Hz), 7.25-7.33 (3H, m), 7.52-7.55 (3H, m), 8.29 (1H, br).

### [Example 30]

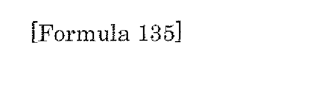

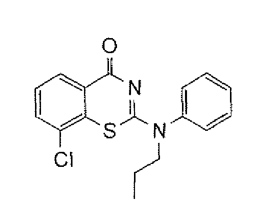

### Compound (1-30):

Yield: 56%
1H-NMR(CDCl3): δ(ppm) 0.95 (3H, t, J=7.5Hz), 1.65-1.73 (2H, m), 4.10 (2H, t, J=7.5Hz), 7.32-7.38 (3H, m), 7.49-7.59 (4H, m), 8.41 (1H, br).

### [Example31]

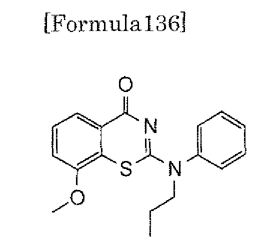

### Compound (I-31):

Yield: 19%
1H-NMR(CDCl3): δ(ppm) 0.94 (3H, t, J=7.0Hz), 1.64-1.772 (2H, m), 3.81 (3H, s), 4.09 (2H, t, J=7.5Hz), 6.97 (1H, dd, J=8.0, 1.0Hz), 7.31-7.38 (3H, m), 7.51-7.58 (3H, m), 8.09 (1H, dd, J=8.0, 1.0Hz).

### [Examples 32]

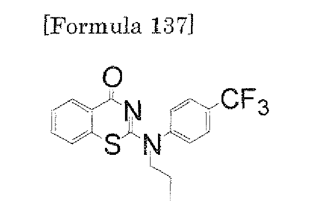

### Compound (I-32):

Yield: 50%
1H NMR(CDCl3) : δ(ppm) 0.96(3H, t, J = 7.5 Hz), 1.68(2H, sextet, J = 7.5 Hz), 4.11(2H, t, J = 7.5 Hz), 7.15(1H, d, J = 7.1 Hz), 7.41-7.50(4H, m), 7.82(2H, d, J = 8.4Hz), 8.45(1H, d, J = 7.1 Hz)

### [Example 33]

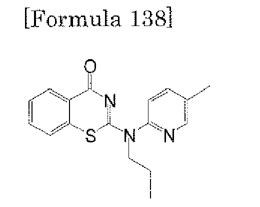

### Compound (I-33):

Yield: 14%
1H NMR(CDC13) : δ(ppm) 0.94(3H, t, J = 7.5 Hz), 1.71(2H, sextet, J = 7.5 Hz), 2.45(3H, s), 4.18(2H, t, J = 7.5 Hz), 7.16(1H, d, J = 7.5 Hz), 7.28(1H, d, J = 8.1 Hz), 7.39-7.49(2H, m), 7.69(1H, d, J = 7.8 Hz), 8.42-8.47(2H, m)

### [Example 34]

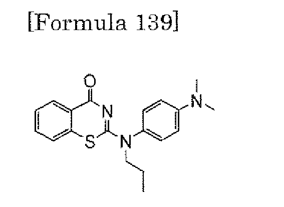

### Compound (I-34):

Yield: 21%
1H NMR(CDCl3) : δ(pom) 0.94(3H, t, J = 7.5 Hz), 1.67(2H, sextet, J = 7.5 Hz), 3.06(1H, s), 4.04(2H, t, J = 7.5 Hz), 6.77(2H, d, J = 9.0 Hz), 7.12(3H, m), 7.34-7.45(2H, m), 8.45(1H, d, J = 7.5 Hz)

### [Example 35]

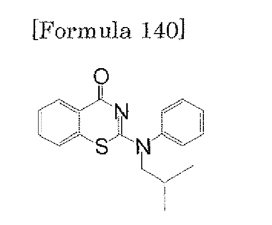

### Compound (I-35):

Yield: 73%
1H NMR(CDCl3) : δ(ppm) 0.99(6H, d, J = 6.6 Hz), 1.94(1H, nonet, J = 6.9 Hz), 4.05(2H, d, J = 7.5 Hz), 7.13(1H, d, J = 7.2 Hz), 7.33-7.45(4H, m), 7.52-7.59(3H, m), 8.45(1H, d, J = 7.2 Hz)

### [Example 36]

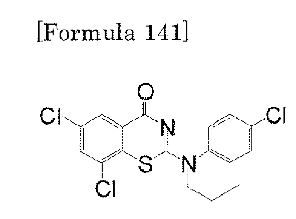

### Compound (I-36):

Yield: 77%
1H NMR(CDCl3) : δ(ppm) 0.95(3H, t, J = 7.5 Hz), 1.67(2H, sextet, J = 7.5 Hz), 4.06(2H, t, J = 7.5 Hz), 7.25-7.30(2H, m), 7.53-7.56(3H, m), 8.41(1H, d, J = 2.4 Hz)

### [Example 37]

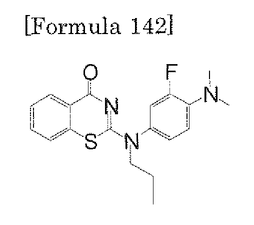

### Compound (I-37):

Yield: 65%
1H NMR(CDCl3) : δ(ppm) 0.94(3H, t, J = 7.2 Hz), 1.67(2H, sextet, J = 7.5 Hz) 2.97(6H, s), 4.03(2H, t, J = 8.0 Hz), 6.89-7.00(3H, m), 7.15(1H, d, J = 7.5 Hz), 7.38-7.48(2H, m), 8.45(1H, d, J = 7.2 Hz)

### [Example 38]

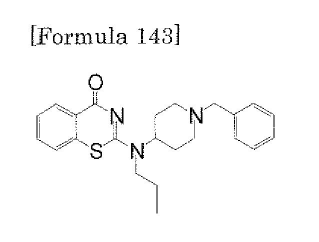

### Compound (I-38):

Yield: 11%
1H NMR(DMSO)100C : δ(ppm) 0.95(3H, t, J = 7.5 Hz), 1.62-1.79(4H, m), 1.84-1.97(2H, m), 2.13(2H, td, J = 11.7 Hz, J = 2.1 Hz), 2.90-2.95(2H, m), 3.47-3.52(4H, m), 4.23(1H, br), 7.21-7.34(5H, m), 7.44-7.62(3H, m), 8.18(1H, d, J = 7.2 Hz),

### [Example 39]

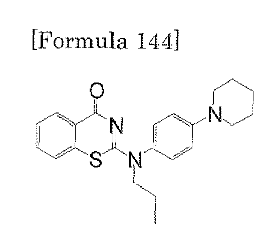

### Compound (I-39):

Yield: 76%
1H NMR(CDC13) : δ(ppm) 0.94(3H, t, J = 7.5 Hz), 1.60-1.79(8H, m), 3.29(4H, t, J = 5.4 Hz), 4.03(2H, t, J = 7.2 Hz), 6.99(2.H, d, J = 8.7 Hz), 7. 11-7.14(3H, m), 7.35-7.45(2H, m), 8.45(1H, d, J = 7.2 Hz)

### [Example 40]

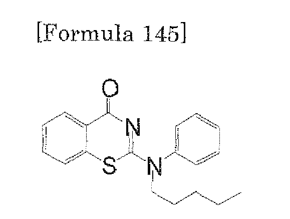

### Compound (I-40):

Yield: 58%
1H NMR(CDCl3) δ(ppm) 0.86(3H, t, J= 7.2 Hz), 1.26-1.35(4H, m), 1.55-1.70(2H, m), 4.12(2H, t, J = 7.8 Hz), 7.12(1H, d, J = 7.2 Hz), 7.30-7.34(2H, m), 7.37-7.46(2H, m), 7.52-7.57(3H, m), 8.45(1H, d, J = 6.9 Hz)

### [Example 41]

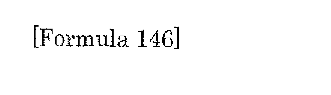

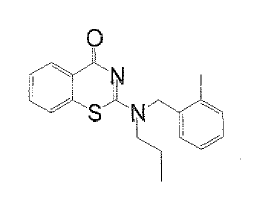

### Compound (I-41):

Yield: 78%
1H NMR(DMSO)100C : δ(ppm) 0.89(3H, t, J = 7.5 Hz), 1.68(2H, sextet, J = 7.5 Hz), 2.32(3H, s), 3.57(2H, t, J = 7.5 Hz), 4.92(2H, s), 7.06-7.10(1H, m), 7.13-7.22(3H, m), 7.46-7.63(3H, m), 8.21(1H, d. J = 8.1 Hz)

### [Example 42]

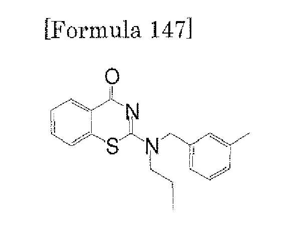

### Compound (I-42):

Yield: 85%
1H NMR(DMSO)100C : δ(ppm) 0.89(3H, t, J = 7.5 Hz), 1.68(2H, sextet, J = 7.5 Hz), 2.29(3H, s), 3.58(2H, t, J = 7.5 Hz), 4.90(2H, s), 7.07-7.15(3H, m), 7.20-7.25(1H, m), 7.46-7.63(3H, m), 8.22(1H, d. J = 7.8 Hz)

### [Example 43]

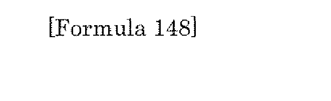

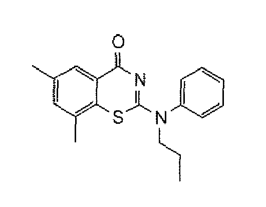

### Compound (I-43):

Yield: 8%
1H-NMR(CDCl3): δ(ppm) 0.94 (3H, t, J=7.5Hz), 1.60-1.72 (2H, m), 2.13 (3H, s), 2.37 (3H, s), 4.10 (2H, t, J=8.0Hz), 7.15 (1H, dd, J=8.0, 1.0Hz), 7.25-7.34 (2H, m), 7.51-7.61 (3H m), 8.18 (1H, dd, J=8.0, 1.0Hz).

### [Example 44]

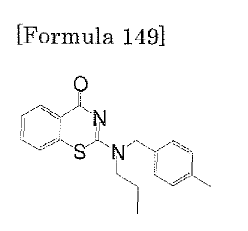

### Compound (I-44):

Yield: 93%
1H NMR(DMSO)100C : δ(ppm) 0.89(3H, t, J = 7.5 Hz), 1.67(2H, sextet, J = 7.5 Hz), 2.28(3H, s), 3.57(2H, t, J = 7.5 Hz), 4.89(2H, s), 7.14-7.23(4H, m), 7,46-7.63(3H, m), 8.21(1H, d. J = 7.8 Hz)

### [Example 45]

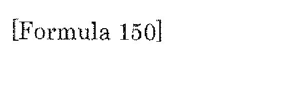

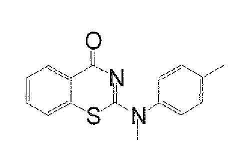

### Compound (I-45):

Yield: 92%
1H NMR(CDC13) : δ(ppm) 2.46(3H, s), 3.61(3H, s), 7.13(1H, d. J = 7.5 Hz), 7.22(2H, d. J = 8.4 Hz), 7.33-7.48(4H, m), 8.46(1H, d. J = 7.5 Hz)

### [Example 46]

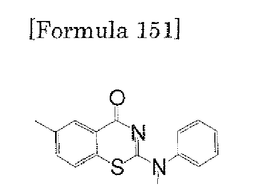

### Compound (I-46):

Yield: 90%
1H NMR(CDCl3) : δ(ppm) 2.41(3H, s), 3.63(3H, s), 7.03(1H, d. J = 8.4 Hz), 7.26-7.30(1H, m), 7.33-7.36(2H, m), 7.51-7.58(3H, m), 8.30(1H, s)

### [Example 47]

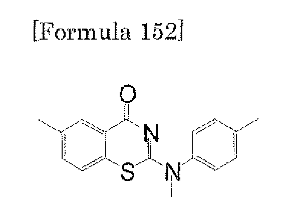

### Compound (I-47):

Yield: 54%
1H NMR(CDCl3) : δ(ppm) 2.41(3H, s), 2.46(3H, s), 3.61(3H, s), 7.03(1H, d. J = 7.8 Hz), 7.21(2H, d. J = 8.4 Hz), 7.25-7.29(1H, m), 7.34(2H, d. J = 8.1 Hz), 8.30(1H, s)

### [Example 48]

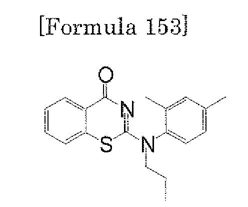

### Compound (I-48):

Yield: 70%
1H NMR(CDCl3) : δ(ppm) 0.94(3H, t, J = 7.5 Hz), 1.50-1.80(2H, m), 2.22(3H, s), 2.42(3H, s), 3.37-3.47(1H, m), 4.48-4.57(1H, m), 7.07-7.28(4H, m), 7.37-7.46(2H, m), 8.47(1H, d, J = 6.9 Hz)

### [Example 49]

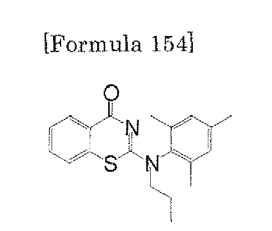

### Compound (I-49):

Yield: 72%
1H NMR(CDCl3): δ(ppm) 0.93(3H, t, J = 7.5 Hz), 1.59-1.72(2H, m), 2.21(6H, s), 2.38(3H, s), 3.91-3.97(2H, m), 7.04(2H, s), 7.12(1H, d, J = 6.9 Hz), 7.38-7.47(2H, m), 8.48(1H, d, J = 6.9 Hz)

### [Example 50]

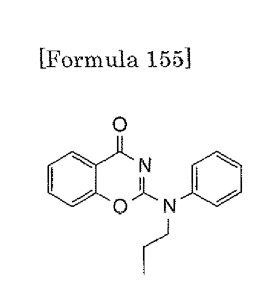

### Compound (I-50):

Yield: 25%
1H-NMR(CDCl3): δ(ppm) 0.94 (3H, t, J=7.5Hz), 1.69 (2H, sextet, J=7.5Hz), 3.98 (2H, t, J=7.5Hz), 6.91 (1H, br), 7.24-7.51 (7H, m), 8.13 (1H, dd, J=7.5, 2.0Hz).

### [Example 51]

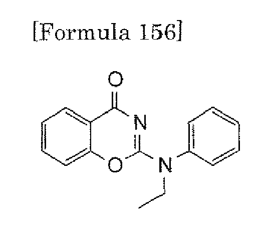

### Compound (I-51):

Yield: 58%
1H~NMR(CDCl3): δ(ppm) 1.28 (3H, t, J=7.0Hz), 4.07 (2H, q, J=7.0Hz), 6.9.1 (1H, br), 7.26-7.51 (7H, m), 8.13 (1H, dd, J=7.5, 2.0Hz).

### [Example 52]

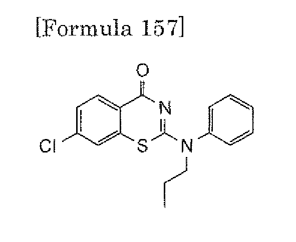

### Compound (I-52):

Yield: 10%
1H-NMR(CDCl3): δ(ppm) 0.95 (3H, t, J=7.5Hz), 1.65-1.72 (2H, m), 4.09 (2H, t, J=7.5Hz), 7.12 (1H, d, J=2.0Hz), 7.29-7.37 (3H, m), 7.52-7.58 (3H, m), 8.38 (1H, d, J=9.0Hz).

### [Example 53]

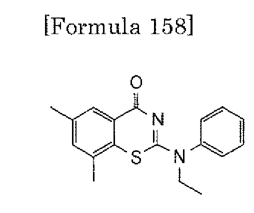

### Compound (I-53):

Yield: 10%
1H-NMR(CDCl3): δ(ppm) 0.91 (3H, t, J=7.5Hz), 2.12 (3H, s), 2.37 (3H, s), 4.01-4.12 (2H, m), 7.15 (1H, dd, J=8.0, 1.0Hz), 7.25-7.34 (2H, m), 7.51-7.61 (3H, m), 8.18 (1H, dd, J=8.0, 1.0Hz).

### [Example 54]

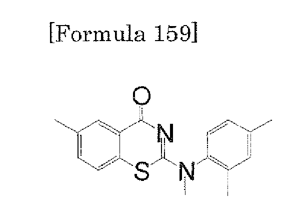

### Compound (I-54):

Yield: 74%
1H NMR(CDCl3) : δ(ppm) 2.21(3H, s), 2.40(3H, s), 2.42(3H, s), 3.54(3H, s), 7.02(1H, d. J = 8.1 Hz), 7.10-7.20(3H, m), 7.25-7.29(1H, m), 8.32(1H, s)

### [Example 55]

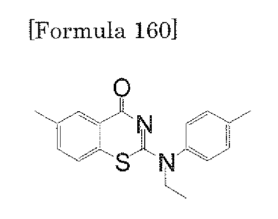

### Compound (I-55):

Yield: 27%
1H NMR(CDCl3) : δ(ppm) 1.24(3H, t, J = 7.2 Hz), 2.4-0(3H, s), 2.46(3H, s), 4.16(2H, q, J = 7.2 Hz), 7.02(1H, d, J = 7.8 Hz), 7.19(2H, d, J = 8.4 Hz), 7.24-7.30(1H, m), 7.34(2H, d, J = 8.4 Hz), 8.30(1H, s)

### [Example 56]

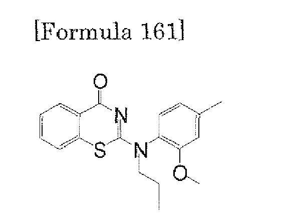

### Compound (I-56):

Yield: 55%
1H NMR(CDCl3) : δ(ppm) 0.93(3H, t, J = 7.2 Hz), 1.63(2H, sextet, J = 7.5 Hz), 2.46(3H, s), 3.50-3.62(1H, m), 3.82(3H, s), 4.36-4.45(1H, m), 6.87-6.90(2H, m), 7.09-7.13(2H, m), 7.35-7.44(2H, m), 8.46(1H, d, J = 7.2 Hz)

### [Example 57]

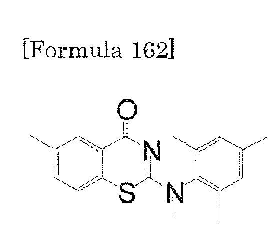

### Compound (I-57):

Yield: 46%
1H NMR(CDCl3) : δ(ppm) 2.18(6H, s), 2.37(3H, s), 2.41(3H, s), 3.49(3H, s), 7.01-7.04(3H, m), 7.26-7.29(1H, m), 8.33(1H, s)

### [Examples 58]

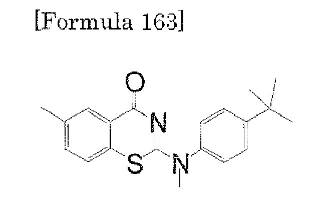

### Compound (I-58):

Yield: 47%
1H NMR(CDCl3) : δ(ppm) 1.39(9H, s), 2.41(3H, s), 3.61(3H, s), 7.05(1H, d. J = 8.1 Hz), 7.23-7.29(3H, m), 7.53(2H, d. J = 8.7 Hz), 8.30(1H, s)

### [Example 59]

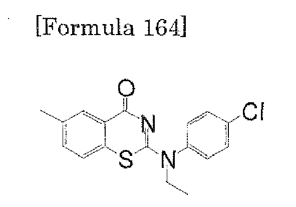

### Compound (I-59):

Yield: 32%
1H NMR(CDCl3): δ(ppm) 1.25(3H, t, J = 7.2 Hz), 2.41(3H, s), 4.16(2H, q, J = 7.2 Hz), 7.03(1H, d, J = 8.1 Hz), 7.25-7.30(3H, m), 7.52(2H, d, J = 8.4 Hz), 8.29(1H, s)

### [Example 60]

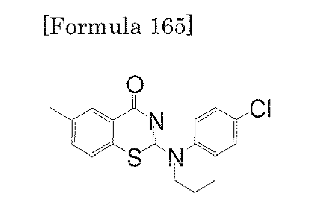

### Compound (1-60):

Yield: 34%
1H NMR(CDCl3) : δ(ppm) 0.94(3H, t, J = 7.2 Hz), 1.66(2H, sextet, J = 7.5 Hz), 2.41(3H, s), 4.06(2H, t, J = 7.8 Hz), 7.03(1H, d, J = 8.1 Hz), 7.24-7.30(3H, m), 7.51(2H, d, J = 8.7 Hz), 8.29(1H, s)

### [Example 61]

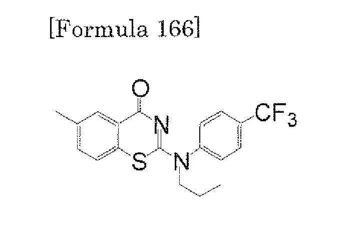

### Compound (1-61):

Yield: 38%
1H NMR(CDCl3) : δ(ppm) 0.95(3H, t, J = 7.5 Hz), 1.68(2H, sextet, J = 7.5 Hz), 2.42(3H, s), 4,10(2H, t, J = 7.8 Hz), 7.04(1H, d, J = 8.1 Hz), 7.29(1H, dd, J = 7.5 Hz, J = 1.2 Hz), 7.47(2H, d, J = 8.1 Hz), 7.81(2H, d, J = 8.1 Hz), 8.29(1H, s)

### [Example 62]

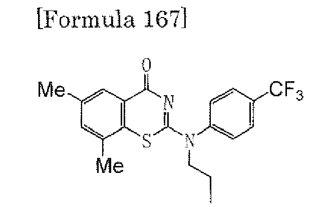

### Compound (I-62):

Yield: 46%
1H-NMR(CDCl3): δ(ppm) 0.95 (3H, t, J=7.5Hz), 1.60-1.75 (2H, m), 2.17 (3H, s), 2.38 (3H, s), 4.06-4.15 (2H, m), 7.17 (1H, s), 7.48 (2H, d, J=8Hz), 7.81 (2H, d, J=8Hz), 8.18 (1H, s).

### [Example 63]

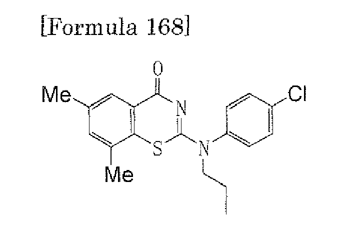

### Compound (I-63):

Yield: 47%
1H-NMR(CDCl3): δ(ppm) 0.94 (3H, t, J=7.5Hz), 1.60-1.73 (2H, m), 2.16 (3H, s), 2.37 (3H, s), 4.00-4.12 (2H, m), 7.16 (1H, s), 7.26 (2H, d, J=8.5Hz), 7.51 (2H, d, J=8.5Hz), 8.18 (1H, s).

### [Example 64]

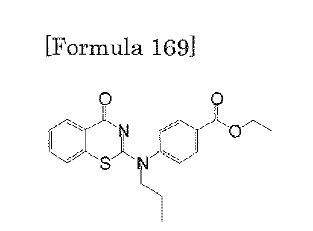

### Compound (I-64):

Yield: 100%
1H NMR(CDCl3) : δ(ppm) 0.95(3H, t, J = 7.5 Hz), 1.44(3H, t, J = 7.2 Hz), 1.68(2H, sextet, J = 7.5 Hz), 4.11(2H, t, J = 7.5 Hz), 4.44(2H, q, J = 7.2 Hz), 7.12(1H, d, J = 7.2 Hz), 7.40-7.47(4H, m), 8.22(2H, d, J = 8.4 Hz), 8.46(1H, d J = 7.5 Hz)

### [Example 65]

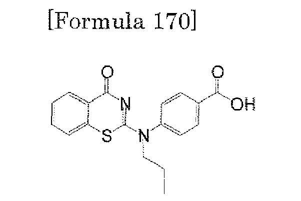

### Compound (I-65):

A mixture of 0.10 g (0.271 mmole) of the compound (I-64), 2 ml of concentrated hydrochloric acid and 4 ml of acetic acid was heated to reflux with stirring for 3.5 hours. Water and methylene chloride were added to the reaction solution, and extracted. The organic layer was washed with brine, dried over anhydrous sodium sulfate, and filtered, the solvent was concentrated under reduced pressure, and the residue was purified by silicagel columnchromatography (n-hexane : ethyl acetate = 1 : 1) to give 0.10 g (100%) of a title compound as colorless solid.
1H NMR(CDCl3) : δ(ppm) 1.01(3H, t, J = 7.5 Hz), 1.67(2H, sextet, J = 7.2 Hz), 3.16(2H, t, J = 7.2 Hz), 6.56(2H, d, J = 9.0 Hz), 7.37(1H, d, J = 8.1 Hz), 7.47(1H, t, J = 7.8 Hz), 7.65(1H, t, J = 7.8 Hz), 7.91(2H, d, J = 8.7Hz), 8.39(1H, d, J = 8.1 Hz), 8.63(1H, s(br), OH)

### [Example 66]

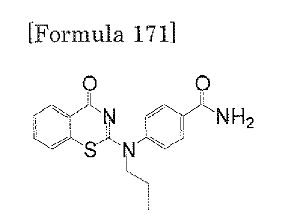

### Compound (I-66):

A mixture of 50 mg (1.47 mole) of the compound (I-65), 36 mg (2.2 mmole) of 1, 1-carbonyldiimidazole and 5 ml of tetrahydrofuran was stirred at room temperature for 4.5 hours. To the reaction solution was added 3 ml of 28% aqueous ammonia, and the mixture was stirred for 19 hours. Water and ethyl acetate were added to the reaction solution, and extracted. The organic layer was washed with brine, dried over anhydrous sodium sulfate, and filtered. The solvent was concentrated under reduced pressure, and the residue was purified by silicagel columnchromatography (ethyl acetate) to give 5 mg (10%) of a title compound as a colorless solid.
1H NMR(CDCl3) : δ(ppm) 0.95(3H, t, J = 7.5 Hz), 1.60-1.75(2H, m), 4.10(2H, t, J = 7.8 Hz), 5.79(1H, s(br), NH), 6.38(1H, s(br), NH), 7.14(1H, d, J = 7.5 Hz), 7.40-7.50(4H, m), 8.00(2H, d J = 8.4 Hz), 8.45(1H, d, J = 7.2 Hz)

### (Reference Example 8)

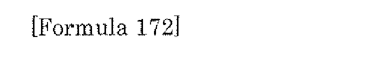

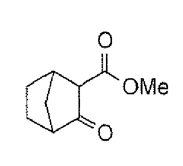

### Compound (I'-76):

To a mixture of 1.5 g (13.6 mmole) of norcamphor and 53 ml of tetrahydrofuran was added dropwise 7.0 ml (14.2 mmole) of lithium diisopropylamine at -78°C under argon atmosphere, and the mixture was stirred at the same temperature for 30 minutes. A mixture of 2.45 g (27.2 mmole) of dimethyl carbonate and 20 ml of tetrahydrofuran was added dropwise, and the mixture was stirred at room temperature for 15 hours. Water was added to the reaction solution, and extracted with ethyl acetate. The organic layer was washed with brine, dehydrated with anhydrous sodium sulfate, and filtered, and the solvent was concentrated under reduced pressure. The residue was purified by silicagel columnchromatography (n-hexane : ethyl acetate = 4 : 1) to give 0.59 g (26%) of a title compound as a yellow liquid.
1H-NMR(CDCl3): δ(ppm) 1.48-1.90 (6H, m), 2.67-2.74 (1H, m), 2.86 (1H, d, J=3.0Hz), 2.92-2.93 (1H, m), 3.73 (3H, s).

The following compounds were synthesized as in Reference Example 8.

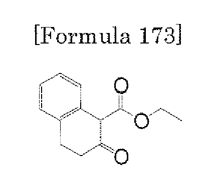

### Compound (I'-77):

Yield; 54%
1H NMR(CDCl3) : δ(ppm) 1.41(3H, t J = 7.2 Hz), 2.53(2H, t J = 7.2 Hz), 2.82(2H, t J = 7.2 Hz), 4.39(2H, q J = 7.2 Hz), 7.03-7.21(3H, m), 7.72(1H. d J = 7.8 Hz), 13.43(1H, s)

### (Reference Example 9)

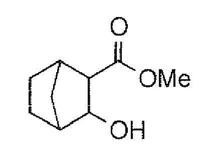

### Compound (I'-78):

To a mixture of 0.59 g (3.51 mmole) of the compound (I'-76) and 10 ml of methanol was added 0.13 g (3.51 mmole) of sodium borohydride under ice-cooling, and the mixture was stirred at room temperature for 15 hours. To the reaction solution was added 5 ml of 5% aqueous HCl, and extracted with diisopropyl ether. The organic layer was washed with brine, dehydrated with anhydrous sodium sulfate, and filtered, and the solvent was concentrated under reduced pressure to give 0.37 g (62%) of a title compound as a yellow liquid.
1H-NMR(CDCl3): δ(ppm) 1.19-1.60 (4H, m), 1.56-1.65 (2H, m), 1.83-1.90 (1H, m), 2.01-2.03 (1H, m), 2.31-2.33 (1H, m), 2.46-2.47 (1H, m), 3.69 (3H, s), 4.40-4.42 (1H, m)

The following compounds were synthesized as in Reference Example 9.

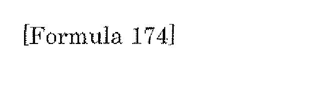

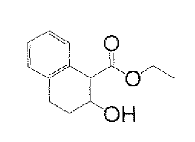

### Compound (I'-79):

Yield: 68%
1H NMR(CDCl3) : δ(ppm) 1.31(3H, t J = 7.2 Hz), 1.93-2.10(1H, m), 2.22-2.33(1H, m), 2.78-2.88(1H, m), 3.02-3.11(2H, m), 4.00(1H, d J = 4.8 Hz), 4.21-4.28(3H, m), 7.11-7.23(4H, m)

### (Reference Example 10)

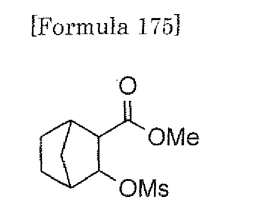

### Compound (I'-80):

To a mixture of 0.37 g (2.17 mmole) of the compound (I'-78), 0.31 g (3.04 mmole) of triethylamine and 10 ml of tetrahydrofuran was added 0.27 g (2.39 mmole) of methanesulfonyl chloride under ice-cooling, and the mixture was stirred at room temperature for 14 hours. Water was added to the reaction solution, and extracted with ethyl acetate. The organic layer was washed with brine, dehydrated with anhydrous sodium sulfate, and filtered, and the solvent was concentrated under reduced pressure to give 0.41 g (76%) of a title compound as a brown liquid.
1H-NMR(CDCl3): δ(ppm) 1.19-1.82 (6H, m), 2.36 (1H, t, J=3.0Hz), 2.53-2.55 (1H, m), 2.64-2.65 (1H, m), 3.04 (3H, s), 3.73 (3H, s), 5.18-5.21 (1H, m)

The following compounds were synthesized as in Reference Example 10.

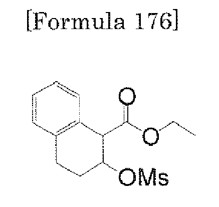

### Compound (I'-81):

Yield: 60%
1H NMR(CDCl3) : δ(ppm) 1.29(3H, t J = 7.2 Hz), 2.10-2.19(1H, m), 2.37-2.44(1H, m), 2.70-2.83(1H, m), 2.87-2.98(1H, m), 3.07(3H, s), 4.17-4.23(3H, m), 5.21-5.27(1H, m), 7.15-7.23(4H, m)

### (Reference Example 11)

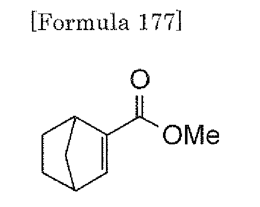

### Compound (1'-82):

A mixture of 0.41 g (1.65 mmole) of the compound (1'-80), 0.50 g (3.30 mmole) of 1,8-diazabicyclo[5.4.0]undeca-7-ene and 10 ml of toluene was heated to reflux with stirring for 2 hours. After cooled with air, water and 5% aqueous HCl were added to the reaction solution, and extracted with toluene. The organic layer was washed with brine, dehydrated with anhydrous sodium sulfate, and filtered, and the solvent was concentrated under reduced pressure to give 0.27 g (90%) of a title compound as a brown liquid.
1H-NMR(CDCl3): δ(ppm) 1.05-1.79 (6H, m), 3.00-3.04 (1H, m), 3.22-3.26 (1H, m), 3.73 (3H, s), 6.92 (1H, d, J=3.0Hz)

The following compounds were synthesized as in Reference Example 11.

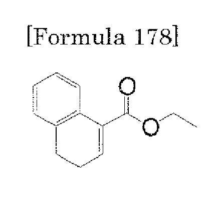

### Compound (I'-83):

Yield 77%
1H NMR(CDCl3) : δ(ppm) 1.37(3H, t J = 7.2 Hz), 2.37-2.44(2H, m), 2.77(2H, t J = 7.2 Hz), 4.32(2H, q J = 7.2 Hz), 7.13-7.29(4H, m), 7.78(1H, d, J = 7.5 Hz)

### (Reference Example 12)

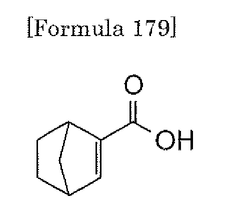

### Compound (I'-84):

A mixture of 0.27 g(1.49 mmole) of the compound (I'-82) and 4.3 ml of 2N-aqueous NaOH was heated to reflux with stirring for 8 hours. After cooled with the air, the reaction solution was acidified with 2N aqueous HCl, and extracted with diisopropyl ether. The organic layer was washed with brine, dehydrated with anhydrous sodium sulfate, and filtered, and the solvent was concentrated under reduced pressure to give 0.17 g (71%) of a title compound as a brown liquid.
1H-NMR(CDCl3): δ(ppm) 1.07-1.81 (6H, m), 3.04-3.06 (1H, m), 3.24-3.28 (1H, m), 7.08 (1H, d, J=3.0Hz), 11.59 (1H, br)

The following compounds were synthesized as in Reference Example 12.

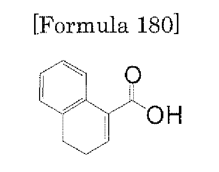

### Compound (I'-85):

Yield: 80%
1H NMR(CDCl3) : δ(ppm) 1.26(1H, s(br), OH), 2.42-2.49(2H, m), 2.79(2H, t J = 7.8 Hz), 7.14-7.29(3H, m), 7.42(1H, t, J = 7.8 Hz), 7.91(1H, d, J = 8.1 Hz)

Using the known compounds described in Tetrahedron Letter P1621-1622 (1975) and Synthesis p619-633 (2004), the following compounds were synthesized as in Reference Example 7.

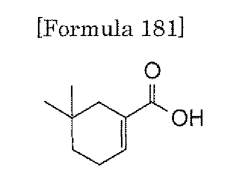

### Compound (I'-86):

Yield; 90%
1H-NMR(CDCl3): δ(ppm) 0.94 (6H, s), 1.36 (2H, t, J=6.0Hz), 2.05 (2H, q, J=2.5Hz), 2.21-2.29 (2H, m), 7.11 (1H, septet, J=2.0Hz), 11.52 (1H, br)

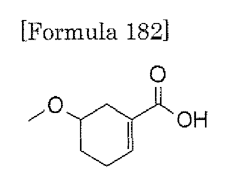

### Compound (I'-87):

Yield: 100%
1H-NMR(CDCl3): δ(ppm) 1.67-1.87 (2H, m), 2.22-2.64 (4H, m), 3.39 (3H, s), 3.55-3.60 (1H, m), 7.11-7.14 (1H, m), 11.59 (1H, br)

### (Reference Example 13)

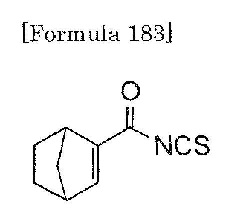

### Compound (I'-88):

To a mixture of 0.7.6 g (1.16 mmole) of the compound (I'-84) 0.5 ml of anhydrous tetrahydrofuran, 1.5 ml of anhydrous dichloromethane and three drops of anhydrous N,N-dimethylformamide was added dropwise 0.23 g (1.85 mmole) of oxalyl chloride under ice-cooling, and the mixture was heated to reflux with stirring for 3 hours. The reaction solution was concentrated under reduced pressure, and subjected to azeotropy with toluene three times. To the residue were added 1.2 ml of acetone and 0.14 g (1.39 mmole) of potassium thiocyanate, and the mixture was stirred at room temperature for 30 minutes. After the reaction solution was concentrated under reduced pressure, and the residue was purified by silicagel columnchromatography (n-hexane : ethyl acetate = 4 : 1) to give 0.08 g (40%) of a title compound as a brown liquid.
1H-NMR(CDCl3): δ(ppm) 1.06-1.29 (4H, m), 1.75-1.87 (2H, m), 3.12 (1H, br), 3.33 (1H, br), 7.20-7.22 1H, m)

Using the compound (I'-85) to the compound (I'-87) and the known compounds described in Chemical Letter 917-918 (1997), the following compounds were synthesized as in Reference Example 13.

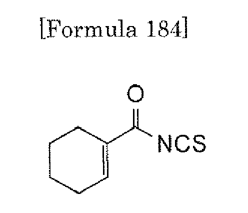

### Compound (I'-89):

Yield: 43%
1H-NMR(CDCl3): δ(ppm) 1.59-1.71 (4H, m), 2.26-2.34 (4H, m), 7.20 (1H, quint, J=1.5Hz)

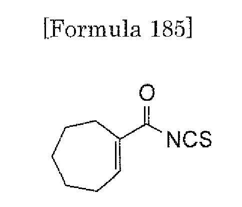

### Compound (I'-90):

Yield: 56%
1H-NMR(CDCl3): δ(ppm) 1.48-1.61 (4H, m), 1.76-1.84 (2H, m), 2.40 (2H, dd, J=11.5, 6.5Hz), 2.52 (2H, t, J=5.5Hz), 7.40 (1H, t, J=6.5Hz)

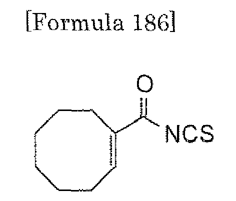

### Compound (I'-91):

Yield: 53%
1H-NMR(CDCl3): δ(ppm) 1.40-1.71 (8H, m), 2.38 (2H, td, J=8.5, 6.0Hz), 2.48 (2H, t, J=6.5Hz), 7.20 (1H, t, J=8.5Hz)

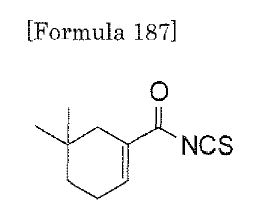

### Compound (I'-92):

Yield: 91%
1H-NMR(CDCl3): δ(ppm) 0.93 (6H, s), 2.05-2.07 (2H, m), 2.29-2.37 (2H, m), 2.21-2.29 (2H, m), 7.19 (1H, septet, J=2.0Hz)

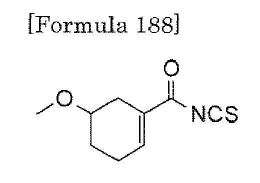

### Compound (I'-93):

Yield: 34%
1H-NMR(CDCl3): δ(ppm) 1.76-1.82 (2H, m), 2.27-2.60 (4H, m), 3.38 (3H, s), 3.55-3.63 (1H, m), 7.18-7.22 (1H, m)

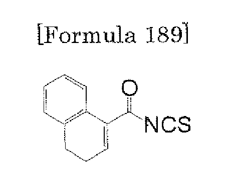

### Compound (I'-94):

Yield: 63%
1H NMR(CDCl3) : δ(ppm) 2.48-2.55(2H, m), 2.73(2H, t J = 7.8 Hz), 7.16-7.30(3H, m), 7.51(1H, d, J = 7.8 Hz), 7.92-7.97(1H, m)

A diastereomer (form A) of the present compound shown below indicates a low polar compound, and a diastereomer (form B) indicates a high polar compound.

### [Example 67]

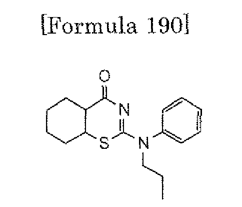

### Compound (I-67):

A mixture of 0.10 g (0.60 mmole) of the compound (I'-89), 0.081 g (0.60 mole) of N-propylaniline and 1.2 ml of 1,4-dioxane was heated to reflux with stirring for 40 minutes. The reaction solution was concentrated under reduced pressure, and the residue was purified by silicagel columnchromatography (n-hexane : ethyl acetate = 1 : 2) to give 68 mg (38%) of a diastereomer (form A) as a colorless crystal, and 81 mg (44%) of a diastereomer (form B) as a colorless crystal. Diastereomer (form A):1H-NMR(CDCl3): δ(ppm) 0.89 (3H, t, J=7.5Hz), 1.26-1.43 (4H, m), 1.55-1.62 (2H, m), 1.73-1.90 (3H, m), 2.05-2.17 (1H, m), 2.30-2.38 (1H, m), 3.10 (1H, td, J=12.0, 3.5Hz), 3.74-3.83 (1H, m), 4.04-4.14 (1H, m), 7.23-7.26 (2H, m), 7.45-7.50 (3H, m).
Diastereomer (form B):1H-NMR(CDCl3): δ(ppm) 0.89 (3H, t, J=7.5Hz), 1.35-1.41 (2H, m), 1.53-1.81 (7H, m), 1.98-2.08 (1H, m), 2.66 (1H, quintet, J=4.0Hz), 3.48-3.52 (1H, m), 3.87-4.06 (2H, m), 7.24-7.27 (2H, m), 7.45-7.51 (3H, m).

The following compounds were synthesized as in Example 67.

### [Example 68]

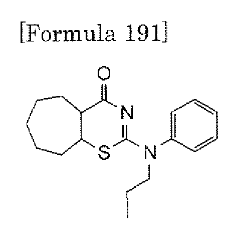

### Compound (I-68):

### Diastereomer (form A)

Yield: 15%
1H-NAER(CDCl3): δ(ppm) 0.88 (3H, t, J=7.5Hz), 1.45-1.81 (11H, m), 2.32-2.41 (2H, m), 3.18-3.26 (1H, m), 3.76 (1H, ddd, J=13.0, 9.0, 6.5Hz), 4.10 (1H, ddd, J=13.0, 9.0, 6.5Hz), 7.24-7.27 (2H, m), 7.42-7.50 (3H, m).

### [Example 69]

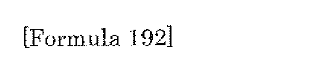

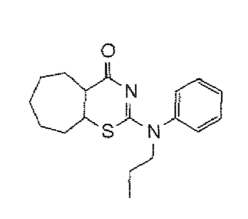

### Compound (I-69):

### Diastereomer (form B)

Yield: 72%
1H-NMR(CDCl3): δ(ppm) 0.89 (3H, t, J=7.5Hz), 1.47-2.08 (12H, m), 2.72-2.78 (1H, m), 3.65-3.70 (1H, m), 3.88-3.92 (1H, m), 3.99-4.C33 (1H. m), 7.24-7.27 (2H, m), 7.44-7.51 (3H, m).

### [Example 70]

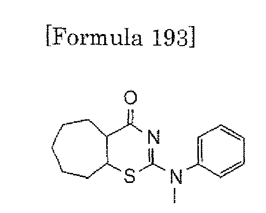

### Compound (I-70):

### Diastereomer (form A)

Yield: 49%
1H-NMR(CDCl3): δ(ppm) 1.41-1.83 (9H, m), 2.34-2.42 (2H, m), 3.22-3.30 (1H, m), 3.49 (3H, s), 7.25-7.30 (2H, m), 7.42-7.50 (3H, m).

### [Example 71]

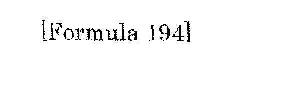

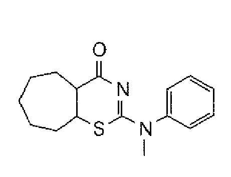

### Compound (I-71):

### Diastereomer (form B)

Yield: 27%
1H-NMR(CDCl3): δ(ppm) 1.47-2.14 (10H, m), 2.77 (1H, m), 3.51 (3H, 2), 3.68 (1H, m), 7.26-7.30 (2H, m), 7.44-7.51 (3H, m).

### [Example 72]

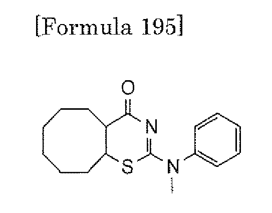

### Compound (I-72):

### Diastereomer (form A)

Yield: 2%
1H-NMR(CDCl3): δ(ppm) 1.26-1.71 (12H, m), 1.96-2.04 (1H, m), 3.46-3.60 (1H, m), 3.64 (3H, s), 7.24-7.52 (5H, m).

### [Example 73]

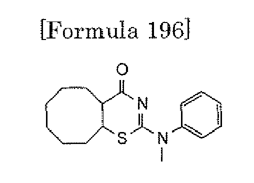

### Compound (I-73):

### Diastereomer (form B)

Yield: 8%
1H-NMR(CDCl3): δ(ppm) 1.35-1.95 (10H, m), 2.23-2.27 (1H, m), 2.43-2.53 (1H, m), 2.60-2.69 (1H, m), 3.32-3.49 (1H, m), 3.57 (3H, s), 7.27-7.50 (5H, m).

### [Example 74]

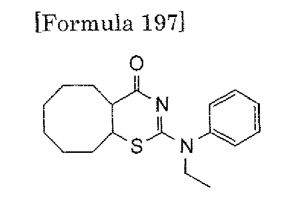

### Compound (I-74)_{:}

### Diastereomer (form A)

Yield: 4%
1H-NMR(CDCl3) δ(ppm) 1.17 (3H, t, J=7.0Hz), 1.38-2.00 (11H, m), 2.46 (1H, ddd, J=13.5, 6.5, 2.5Hz), 2.61-2.68 (1H, m), 3.35 (1H, ddd, J=13.0, 5.5, 2.0Hz), 3.89-4.01 (1H, m), 4.06-4.17 (1H, m), 7.24-7.27 (2H, m), 7.45-7.51 (3H, m).

### [Example 75]

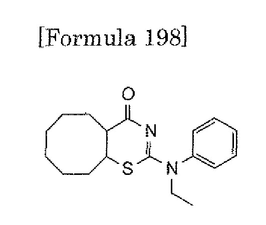

### Compound (I-75):

### Diastereomer (form B)

Yield: 38%
1H-NMR(CDCl3): δ(ppm) 1.18 (3H, t, J=7.0Hz), 1.43-1.81 (1H, m), 1.94-2.07 (1H, m), 2.92 (1H, dt, J=10.0, 3.0Hz), 3.57-3.63 (1H, m), 3.94-4.14 (2H, m), 7.23-7.29 (2H, m), 7.44-7.51 (3H, m).

### [Example 76]

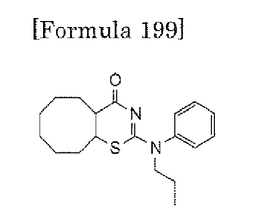

### Compound (1-76):

### Diastereomer (form A)

Yield: 9%
1H-NMR(CDCl3): δ(ppm) 0.89 (3H, t, J=7.0Hz), 1.47-1.93 (13H, m), 2.45 (1H, ddd, J=13.0, 6.5, 2.5Hz), 2.59-2.67 (1H, m), 3.34 (1H, ddd, J=13.0, 5.5, 3.0Hz), 3.73-3.83 (1H, m), 4.04-4.13 (1H, m), 7.24-7.27 (2H, m), 7.42-7.50 (3H, m).

### [Example 77]

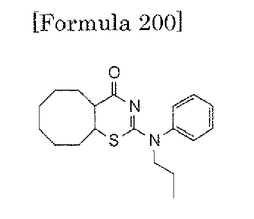

### Compound (I-77):

### Diastereomer (form B)

Yield: 91%
1H-NMR(CDCl3): δ(ppm) 0.89 (3H, t, J=7.0Hz), 1.53-1.78 (13H, m), 1.95-2.05 (1H, m), 2.92 (1H dt, J=9.0, 3.5Hz), 3.56-3.63 (1H, m), 3.79-3.89 (1H, m), 4.00-4.10 (1H, m), 7.23-7.26 (2H, m), 7.43-7.51 (3H, m).

### [Example 78]

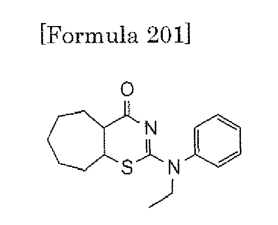

### Compound (I-78):

### Diastereomer (form A)

Yield: 76%
1H-NMR(CDCl3): δ(ppm) 1.17 (3H, t, J=7.0Hz), 1.42-1.76 (9H, m), 2.33-2.42 (2H, m), 3.19-3.27 (1H, m), 3.87-3.98 (1H, m), 4.07-4.18 (1H, m), 7.23-7.28 (2H, m), 1.44-7.9:9 (3H, m).

### [Example 79]

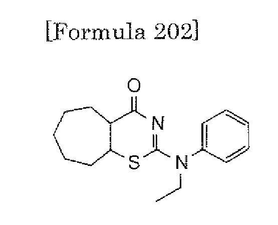

### Compound (1-79):

### Diastereomer (form B)

Yield: 23%
1H-NMR(CDCl3): δ(ppm) 1.18 (3H, t, J=7.0Hz), 1.45-2.10 (10H, m), 2.73-2.78 (1H, m), 3.65-3.70 (1H, m), 3.96-4.16 (2H, m), 7.24-7.27 (2H, m), 7.45-7.50 (3H, m).

### [Example 80]

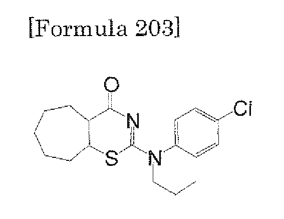

### Compound (I-80):

### Diastereomer (form A)

Yield: 66%
1H NMR(CDCl3): δ(ppm) 0.88(3H, t, J = 7.5 Hz), 1.38-1.92(1H, m), 2.31-2.42(2H, m), 3.19-3.28(1H, m), 3.68-3.78(1H, m), 4.01-4.10(1H, m), 7.19(2H, d, J = 8.7 Hz), 7.43(2H, d J = 8.7 Hz)

### [Example 81]

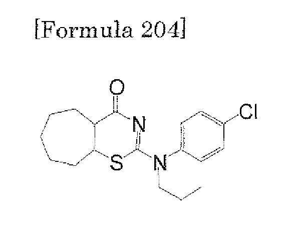

### Compound (I-81):

### Diastereomer (form B)

Yield: 29%
1H NMR(CDCl3): δ(ppm) 0.89(3H, t, J = 7.5 Hz), 1.40-1.85(10H, m), 1.92-2.10(2H, m), 2.72-2.78(1H, m), 3.60-3.74(1H, m), 3.84-4.04(2H, m), 7.19(2H, d, J = 8.7 Hz), 7.44(2H, d J = 8.7 Hz)

### [Example 82]

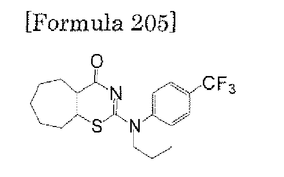

### Compound (1-82):

### Diastereomer (form A)

Yield: 66%
1H NMR(CDCl3): δ(ppm) 0.83(3H, t, J = 7.5 Hz), 1.40-1.85(11H, m), 2.33-2.44(2H, m), 3.22-3.32(1H, m), 3.71-3.82(1H, m), 4.03-4.15(1H, m), 7.40(2H, d, J = 8.1 Hz), 7.72(2H, d J = 8.1 Hz)

### [Example 83]

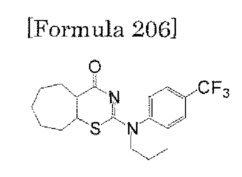

### Compound (I-83):

### Diastereomer (form B)

Yield: 29%
1H NMR(CDCl3): δ(ppm) 0.90(3H, t, J = 7.5 Hz), 1.50-2.22(12H, m), 2.74-2.79(1H, m), 3.68-3.73(1H, m), 3.85-4.07(2H, m), 7.40(2H, d, J = 8.1 Hz), 7.73(2H, d J = 8.1 Hz)

### [Example 84]

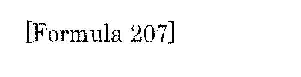

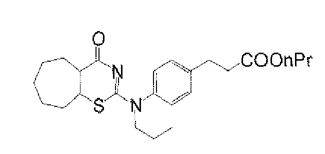

### Compound (I-84):

### Diastereomer (form A)

Yield: 65%
1H NMR(CDCl3): δ(ppm) 0.85-0.93(6H, m), 1.40-1.85(14H, m), 2.30-2.45(2H, m), 2.67(2H, t, J = 7.5 Hz), 3.01(2H, t, J = 7.5 Hz), 3.18-3.25(1H, m), 3.67-3.76(1H, m), 4.04(2H, t, J = 6.9 Hz), 7.15(2H, d, J = 8.1 Hz), 7.28(2H, d J = 8.7 Hz)

### [Example 85]

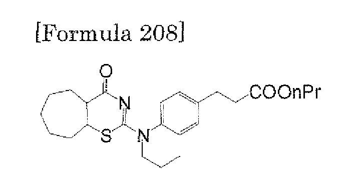

### Compound (I-85):

### Diastereomer (form B)

Yield: 35%
1H NMR(CDCl3): δ(ppm) 0.85-0.94(6H, m), 1.45-2.10(14H., m), 2.65-2.77(3H, m), 3.02(2H, t, J = 7.5 Hz), 3.64-3.69(1H, m), 3.79-3.89(1H, m), 3.96-4.07(3H, m), 7.16(2H, d, J = 8.4 Hz), 7.29(2H, d J = 8.4 Hz)

### [Example 86]

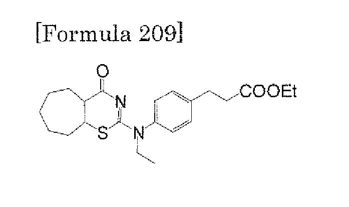

### Compound (I-86):

### Diastereomer (form A)

Yield: 65%
1H NMR(CDCl3): δ(ppm) 1.15(3H, t, J = 7.2 Hz), 1.24(3H, t, J = 7.2 Hz), 1.40-1.85(9H, m), 2.32-2.44(2H, m), 2.66(2H, t, J = 7.8 Hz), 3.0l(2H, t, J = 7.8 Hz), 3.18-3.28(1H, m), 3.85-3.95(1H, m), 4.05-4.18(3H, m), 7.15(2H, d, J = 8.4 Hz), 7.28(2H, d J = 8.4 Hz)

### [Example 87]

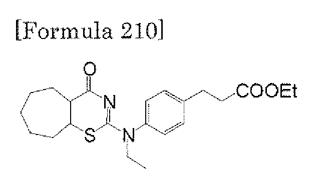

### Compound (I-87):

### Diastereomer (form B)

Yield: 35%
1H NMR(CDCl3): δ(ppm) 1H, t, J = 7.2 Hz), 1.24(3H, t, J = 7.2 Hz), 1.40-2.15(10H, m), 2.67(2H, t, J = 7.2 Hz), 2.72-2.76(1H, m), 3.02(2H, t, J = 7.8 Hz), 3.64-3.69(1H, m), 3.95-4.09(2H, m), 4.14(2H, q, J = 7.2 Hz), 7.16(2H, d, J = 8.4 Hz), 7.30(2H, d J = 8.1Hz)

### [Example 88]

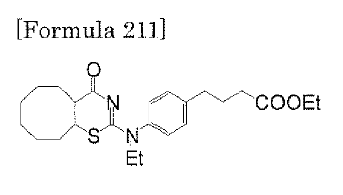

### Compound (I-88):

### Diastereomer (form A)

Yield: 46%
1H NMR(CDCl3: δ(ppm) 1.16(3H, t, J = 7.2 Hz), 1.27(3H, t, J = 7.2 Hz), 1.40-2.05(12H, m), 2.34-2.48(3H, m), 2.60-2.74(3H, m), 3.30-3.39(1H, m), 3.86-4.18(5H, m), 7.15(2H, d, J = 8.1 Hz), 7.27(2H, d J = 8.1 Hz)

### [Example 89]

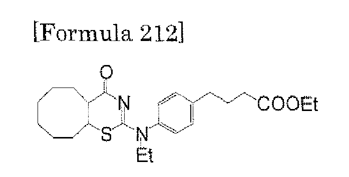

### Compound (I-89):

### Diastereomer (form B)

Yield: 37%
1H NMR(CDCl3): δ(ppm) 1.16(3H, t, J = 7.2 Hz), 1.27(3H, t, J = 7.2 Hz), 1.45-1.80(11H, m), 1.94-2.05(3H, m), 2.36(2H, t, J = 7.2 Hz), 2.71(2H, t, J = 7.2 Hz), 2.88-2.94(1H, m), 3.56-3.62(1H, m), 3.91-4.18(4H, m), 7.1.5(2H, d, J = 8.4 Hz), 7.26-7.30(2H, m)

### [Example 90]

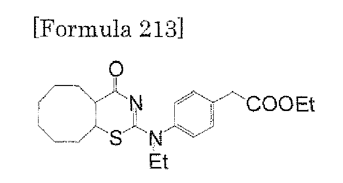

### Compound (I-90):

### Diastereomer (form A)

Yield: 51%
1H NMR(CDCl3): δ(ppm) 1.16(3H, t, J = 7.2 Hz), 1.29(3H, t, J = 7.2 Hz), 1.40-2.00(11H, m), 2.42-2.49(1H m), 2.60-2.68(1H, m), 3.31-3.39(1H, m), 3.68(2H, s), 3.87-3.98(1H, m), 4.04-4.23(3H, m), 7.20(2H, d, J = 8.1 Hz), 7.38(2H, d J = 8.4 Hz)

### [Example 91]

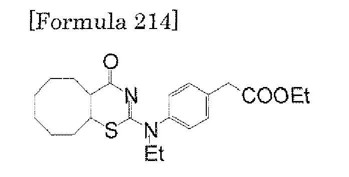

### Compound (I-91):

### Diastereomer (form B)

Yield: 41%
1H NMR(CDCl3): δ(ppm) 1.17(3H, t, J = 7.2 Hz), 1.29(3H, t, J = 7.2 Hz), 1.50-2.05(12H, m), 2.88-2.93(1H, m), 3.57-3.63(1H, m), 3.68(2H, s), 3.91-4.11(2H, m), 4.19(2H, q. J = 7.2 Hz), 7.20(2H, d, J = 8.1 Hz), 7.39(2H, d J = 7.8 Hz)

### [Example 92]

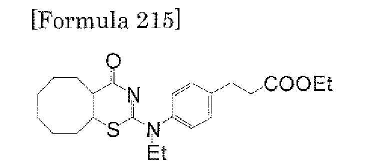

### Compound (I-92):

### Diastereomer (form A)

Yield: 50%
1H NMR(CDCl3): δ(ppm) 1.15(3H, t, J = 7.2 Hz), 1.24(3H, t, J = 7.2 Hz), 1.40-2.00(11H, m), 2.42-2.48(1H, m), 2.60-2.69(3H, m), 3.01(2H, t, J = 7.5 Hz), 3.30-3.38(1H, m), 3.86-8.91(1H, m), 4.03-4.18(3H, m), 7.16(2H, d, J = 8.1 Hz), 7.29(2H, d J = 8.1 Hz)

### [Example 93]

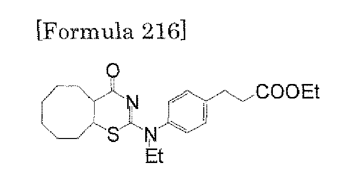

### Compound (I-93):

### Diastereomer (form B)

Yield: 42%
1H NMR(CDCl3): δ(ppm) 1.16(3H, t, J = 7.2 Hz), 1.24(3H, t, J = 7.2 Hz), 1.45-2.05(12H, m), 2.67(2H, t, J = 7.5 Hz), 2.85-2.95(1H, m), 3.02(2H, t, J = 7.5 Hz), 3.56-3.62(1H, m), 3.92-4.09(2H, m), 4.14(2H, q, J = 7.2 Hz), 7.15(2H, d, J = 8.1 Hz), 7.30(2H, d J = 8.4 Hz)

### [Example 94]

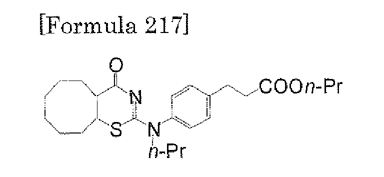

### Compound (I-94):

### Diastereomer (form A)

Yield: 19%
1H NMR(CDCl3): δ(ppm) 0.85-0.93(6H, m), 1.36-1.98(16H, m), 2.40.2.47(1H, m), 2.59-2.70(3H, m), 3.01(2H, t, J = 7.5 Hz), 3.26-3.36(1H, m), 3.70-3.81(1H, m), 4.05(2H, t, J = 6.9 Hz), 7.16(2H, d, J = 8.1 Hz), 7.26-7.30(2H, m)

### [Example 95]

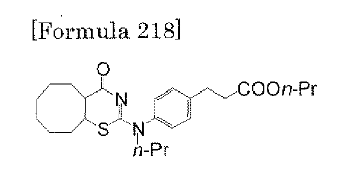

### Compound (I-95):

### Diastereomer (form B)

Yield: 52%
1H NMR(CDCl3): δ(ppm) 0.85-0.94(6H, m), 1.50-1.80(15H, m), 1.85-2.05(1H, m), 2.68(2H, t, J = 7.5 Hz), 2.87-2.93(1H, m), 3.02(2H, t, J = 7.8 Hz), 3.45-3.65(1H, m), 3.76-3.86(1H, m), 3.97-4.07(3H, m), 7.15(2H, d, J = 8.1 Hz), 7.29(2H, d J = 8.4 Hz)

### [Example 96]

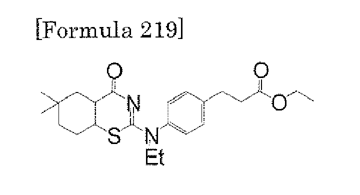

### Compound (I-96):

### Diastereomer (form A)

Yield: 84%
1H NMR(CDCl3): δ(ppm) 0.93(3H, s), 0.99(3H, s), 1.13-1.70(l1H, m), 2.02-2.08(1H, m), 2.28(1H, td, J = 12.5 Hz, J = 3.6 Hz), 2.66(2H, t, J = 7.5 Hz). 2.93-3.04(3H, m), 3.87-3.96(1H, m), 4.07-4.18(3H, m), 7. 16(2H, d, J = 8.1 Hz), 7.30(2H, d J = 8.7 Hz)

### [Example 97]

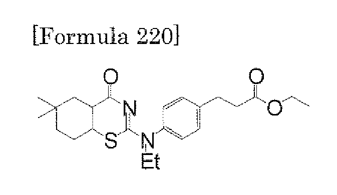

### Compound (I-97):

### Diastereomer (form B)

Yield: 16%
1H NMR(CDCl3): δ(ppm) 0.93-0.97(6H, m), 1.14-1.65(11H, m), 1.87-1.97(1H, m), 2.67(2H, t, J = 7.5 Hz), 2.78(1H, dt, J = 12.6 Hz, J = 3.9 Hz), 3.02(2H, t, J = 7.5 Hz), 3.68-3.72(1H, m), 3.97-4.18(4H, m), 7.17(2H, d, J = 8.4 Hz), 7.31(2H, d J = 8.4 Hz)

### [Example 98]

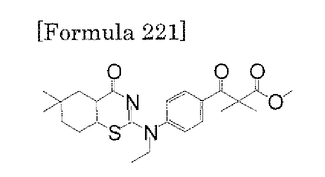

### Compound (I-98):

### Diastereomer (form A)

Yield: 6%
1H NMR(CDCl3): δ(ppm) 0.94(3H, s), 1.00(3H, s), 1.15-1.66(14H, m), 2.02-2.10(1H, m), 2.30(1H, td, J = 12.9 Hz, J = 3.9 Hz), 2.97-3.07(1H, m), 3.68(3H, s), 3.96-4.17(2H, m), 7.32(2H, d, J = 8.7 Hz), 7.89(2H, d J = 8.7 Hz)

### [Example 99]

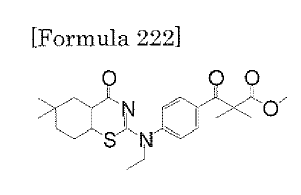

### Compound (I-99):

### Diastereomer (form B)

Yield: 58%
1H NMR(CDCl3): δ(ppm) 0.95-0.96(6H, m), 1. 16-1.64(15H, m), 2.81(1H, dt, J = 12.3 Hz, J = 3.9 Hz), 3.69(3H, s), 3.74(1H, br), 4.03-4.10(2H, m), 7.33(2H, d, J = 8.7 Hz), 7.90(2H, d J = 9.0 Hz)

### [Example 100]

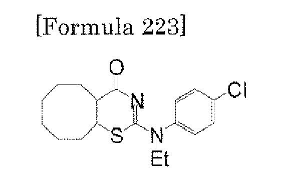

### Compound (I-100):

### Diastereomer (form A)

Yield: 40%
1H NMR(CDCl3): δ(ppm) 1.16(3H, t, J = 7.2 Hz), 1.45-2.03(11H, m), 2.41-2.49(1H, m), 2.60-2.68(1H, m), 3.32-3.40(1H, m), 3.87-4.12(2H, m), 7.20(2H, d, J = 8.4 Hz), 7.43(2H, d J = 8.4 Hz)

### [Example 101]

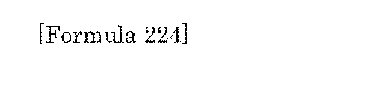

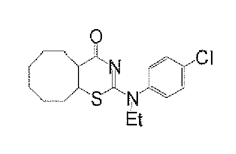

### Compound (I-101):

### Diastereomer (form B)

Yield: 36%
1H NMR(CDCl3): δ(ppm) 1.16(3H, t, J = 7.2 Hz), 1.50-2.05(12H, m), 2.88-2.93(1H, m), 3.58-3.65(1H, m), 3.92-4.13(2H, m), 7.19(2H, d, J = 8.4 Hz), 7.43(2H, d J = 8.1 Hz)

### [Example 102]

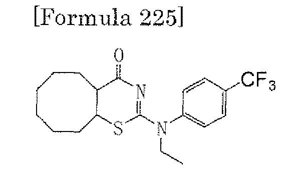

### Compound (I-102):

### Diastereomer (form A)

Yield: 33%
1H-NMR(CDCl3): δ(ppm) 1.18 (3H, t, J=7Hz), 1.37-2.04 (11H, m), 2.40-2.54 (1H, m), 2.57-2.70 (1H, m), 3.31-3.45 (1H, m), 3.90-4.20 (2H, m), 7.40 (2H, d, J=8Hz), 7.73 (2H, d, J=8Hz).

### [Example 103]

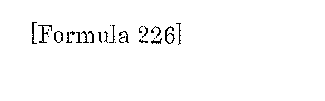

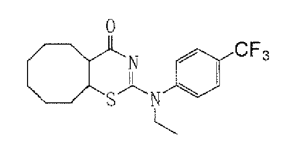

### Compound (I-103):

### Diastereomer (form B)

Yield: 36%
1H-NMR(CDCl3): δ(ppm) 1.18 (3H, t, J=7Hz), 1.49-2.04 (12H, m), 2.85-2.95 (1H, m), 3.60-3.70 (1H, m), 3.98-4.15 (2H, m), 7.39 (2H, d, J=8Hz), 7.74 (2H, d, J=8Hz).

### [Example 104]

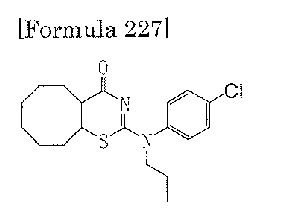

### Compound (I-104):

### Diastereomer (form A)

Yield: 37%
1H-NMR(CDCl3): δ(ppm) 0.88(3H, t, J=7.5Hz), 1.35-2.05 (13H, m), 2.39-2.50 (1H, m), 2.57-2.70 (1H, m), 3.29-3.40 (1H, m), 3.70-3.83 (1H, m), 3.95-4.10 (1H, m), 7.20 (2H, d, J=8.5Hz), 7.43 (2H, d, J=8.5Hz).

### [Example 105]

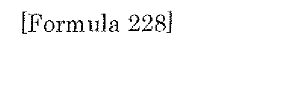

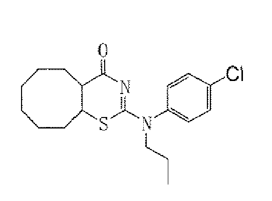

### Compound (I-105):

### Diastereomer (form B)

Yield: 43%
1H-NMR(CDCl3): δ(ppm) 0.88(3H, t, J=7.5Hz), 1.45-2.05 (14H, m), 2.86-2.96 (1H, m), 3.56-3.65 (1H, m), 3.75-3.89 (1H, m), 4.00-4.13 (1H, m), 7.19 (2H, d, J=8.5Hz), 7.44 (2H, d, J=8.5Hz).

### [Example 106]

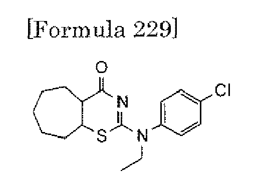

### Compound (I-106):

### Diastereomer (form A)

Yield: 66%
1H-NMR(CDCl3): δ(ppm) 1.16 (3H, t, J=7.0Hz), 1.41-1.81 (9H, m), 2.32-2.41 (2H, m), 3.20-3.28 (1H, m), 3.85-3.96 (1H, m), 4.02-4.14 (1H, m), 7.19 (2H, d, J=8.5Hz), 7.44 (2H, d, J=8.5Hz).

### [Example 107]

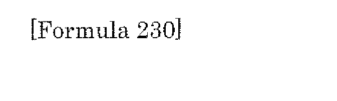

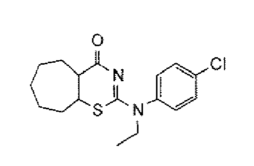

### Compound (I-107):

### Diastereomer (form B)

Yield: 24%
1H-NMR(CDCl3): δ(ppm) 1.16 (3H, t, J=7.0Hz), 1.50-2.07 (10H, m), 2.72-2.78 (1H, m), 3.66-3.71 (1H, m), 3.98-4.06 (2H, m), 7.20 (2H, d, J=8.5Hz), 7.45 (2H, d, J=8.5Hz).

### [Example 108]

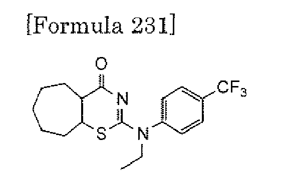

### Compound (I-108):

### Diastereomer (form A)

Yield: 10%
1H-NMR(CDCl3): δ(ppm) 1.18 (3H, t, J=7.5Hz), 1.34-1.87 (9H, m), 2.34-2.42 (2H, m), 3.23-3.31 (1H, m), 3.89-4.01 (1H, m), 4.06-4.18 (1H, m), 7.40 (2H, d, J=8.5Hz), 7.73 (2H, d, J=8.5Hz).

### [Example 109]

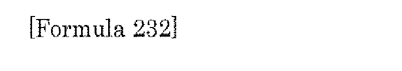

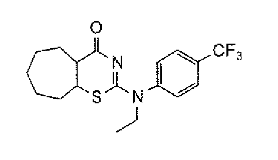

### Compound (I-109):

### Diastereomer (form B)

Yield: 72%
1H-NMR(CDCl3): δ(ppm) 1.19 (3H, t, J=7.5Hz), 1.44-2.13 (10H, m), 2.75-2.81 (1H, m), 3.68-3.73 (1H, m), 4.02-4.10 (2H, m), 7.40 (2H, d, J=8.0Hz), 7.74 (2H, d, J=8.0Hz)

### [Example 110]

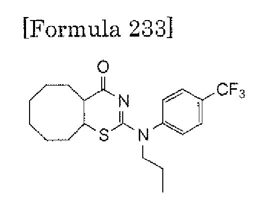

### Compound (I-110):

### Diastereomer (form A)

Yield: 31%
1H-NMR(CDCl3): δ(ppm) 0.89 (3H, t, J=7.5Hz), 1.48-1.80 (12H, m), 1.92-1.97 (1H, m), 2.46 (1H, ddd, J=13.0, 6.0, 2.0Hz), 2.59-2.67 (1H, m), 3.38 (1H, ddd, J=13.0, 5.5, 3.0Hz), 3.76-3.86 (1H, m), 4.01-4.14 (1H, m), 7.41 (2H, d, J=8.0Hz), 7.73 (2H, d, J=8.0Hz).

### [Example 111]

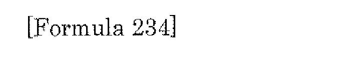

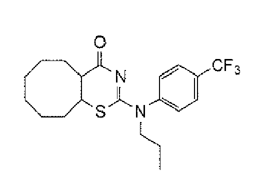

### Compound (I-111):

### Diastereomer (form B)

Yield: 32%
1H-NMR(CDCl3): δ(ppm) 0.90 (3H, t, J=7.5Hz), 1.55-1.80 (13H, m), 1.94-2.05 (1H, m), 2.89-2.94 (1H, m), 3.60-3.67 (1H, m), 3.82-3.92 (1H, m), 3.99-4.09 (1H, m), 7.40 (2H, d, J=8.5Hz), 7.74 (2H, d, J=8.5Hz).

### [Example 112]

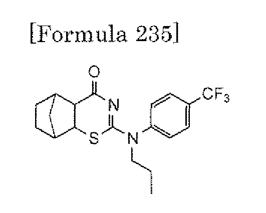

### Compound (I-112):

### Diastereomer (form A)

Yield: 23%
1H-NMR(CDCl3): δ(ppm) 0.90 (3H, t, J=7.5Hz), 1.23-1.45 (3H, m), 1.54-1.74 (5H, m), 2.19 (1H, br), 2.57 (1H, d, J=9.0Hz), 2.95 (1H, br), 3.40 (1H, d, J=9.0Hz), 3.93 (2H, t, J=8.0Hz), 7.35 (2H, d, J=9.0Hz), 7.72 (2H, d, J=8.5Hz).

### [Example 113]

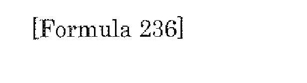

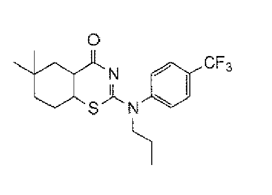

### Compound (I-113):

### Diastereomer (form A)

Yield 28%
1H-NMR(CDCl3): δ(ppm) 0.89 (3H, t, J=7.5Hz), 0.94 (3H, s), 1.00 (3H, s), 1.19 (3H, m), 1.56-1.67 (4H, m), 2.04 (1H, dt, J=1.4.0, 3.0Hz), 2.30 (1H, td, J=13.0, 4.0Hz), 3.02 (1H, td, J=13.0, 4.0Hz), 3.75-3.85 (1H, m), 4.04-4.14 (1H, m), 7.40 (2H, d, J=8.5Hz), 7.73 (2H, d, J=8.5Hz).

### [Example 114]

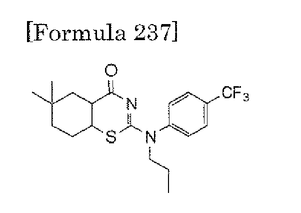

### Compound (1-114):

### Diastereomer (form B)

Yield: 47%
1H-NMR(CDCl3): δ(ppm) 0.90 (3H, t, J=7.5Hz), 0.97 (6H, s), 1.14-1.19 (1H, m), 1.42-1.66 (6H, m), 1.90-2.01 (1H, m), 2.81 (1H, dt, J=13.0, 4.0Hz), 3.75 (1H, br), 3.87-3.97 (1H, m), 4.01-4.10 (1H, m), 7.43 (2H, d, J=8.5Hz), 7.75 (2H, d, J=8.5Hz).

### [Example 115]

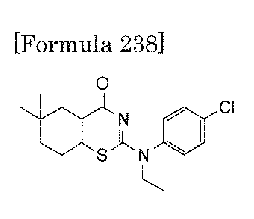

### Compound (1-115):

### Diastereomer (form A)

Yield: 35%
1H-NMR(CDCl3): δ(ppm) 0.94 (3H, s), 1.00 (3H, s), 1.16 (3H, t, J=7.0Hz), 1.20-1.45 (3H, m), 1.55-1.69 (2H, m), 2.04 (1H, dt, J=14.0, 3.0Hz), 2.29 (1H, td, J=12.0, 4.0Hz), 3.00 (1H, td, J=12.0, 4.5Hz), 3.86-3.98 (1H, m), 4.03-4.15 (1H, m), 7.19 (2H, d, J=8.5Hz), 7.44 (2H, d, J=8.5Hz).

### [Example 116]

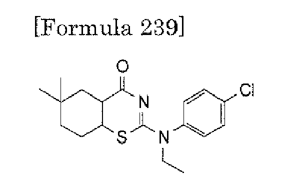

### Compound (I-116):

### Diastereomer (form B)

Yield: 17%
1H-NMR(CDCl3): δ(ppm) 0.95 (6H, s), 1.17 (3H, t, J=7.0Hz), 1.39-1.64 (5H, m), 1.88-2.45 (1H, m), 2.80 (1H, dt, J=14.0, 4.0Hz), 3.72 (1H, br), 4.04 (2H, ddd, J=14.0, 7.0, 2.5Hz), 7.21 (2H, d, J=8.5Hz), 7.45 (2H, d, J=8.5Hz).

### [Example 117]

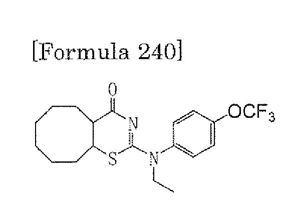

### Compound (I-117):

### Diastereomer (form A)

Yield: 24%
1H-NMR(CDCl3): δ(ppm) 1.17 (3H, t, J=7Hz), 1.40-2.05 (11H, m), 2.40-2.50 (1H, m), 2.58-2.70 (1H, m), 3.32-3.40 (1H, m), 3.87-4.15 (2H, m), 7.30 (4H, s).

### [Example 118]

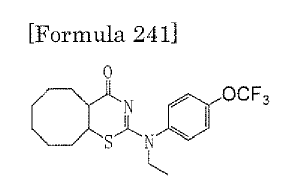

### Compound (I-118):

### Diastereomer (form B)

Yield: 12%
1H-NMR(CDCl3): δ(ppm) 1.18 (3H, t, J=7Hz), 1.45-2.05 (12H, m), 2.87-2.95 (1H, m), 3.58-3.68 (1H, m), 3.93-4.12 (2H, m), 7.30 (4H, s).

### [Example 119]

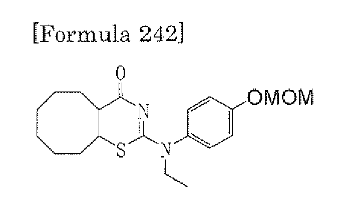

### Compound (I-119):

### Diastereomer (form A)

Yield: 64%
1H-NMR(CDCl3): δ(ppm) 1.16 (3H, t, J=7.5Hz), 1.35-2.00 (11H, m), 2.40-2.50 (1H, m), 2.58-2.70 (1H, m), 3.29-3.39 (1H, m), 3.51 (3H, s), 3.85-3.97 (1H, m), 4.00-4.15 (1H, m), 5.22 (2H, s), 7.09 (2H, d, J=9Hz), 7.16 (2H, d, J=9Hz).

### [Example 120]

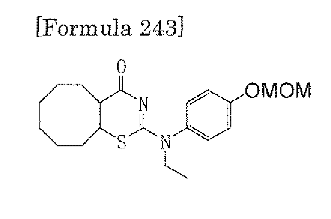

### Compound (I-120):

### Diastereomer (form B)

Yield: 23%
1H-NMR(CDCl3): δ(ppm) 1.16 (3H, t, J=7Hz), 1.40-2.05 (12H, m), 2.86-2.95 (1H, m), 3.51 (3H, s), 3.54-3.63 (1H, m), 3.89-4.08 (2H, m), 5.22 (2H, s), 7.09 (2H, d, J=9Hz), 7.16(2H, d, J=9Hz).

### [Example 121]

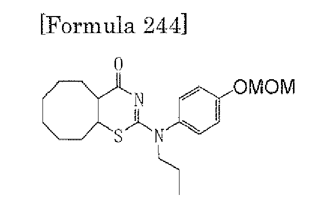

### Compound (I-121):

### Diastereomer (form A)

Yield: 58%
1H-NMR(CDCl3): δ(ppm) 0.88 (3H, t, J=7.5Hz), 1.35-2.03 (13H, m), 2.38-2.50 (1H, m), 2.57-2.70 (1H, m), 3.25-3.38 (1H, m), 3.51 (3H, s), 3.65-3.80 (1H, m), 3.97-4.16 (1H, m), 5.21 (2H, s), 7.09 (2H, d, J=9Hz), 7.15 (2H, d, J=9Hz).

### [Example 122]

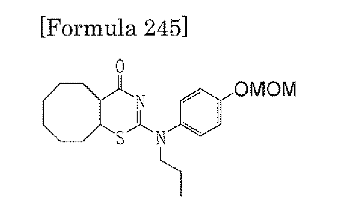

### Compound (I-122):

### Diastereomer (form B)

Yield: 38%
1H-NMR(CDCl3): δ(ppm) 0.88 (3H, t, J=7.5Hz), 1.40-2.05 (14H, m), 2.86-2.94 (1H, m), 3.51 (3H, s), 3.53-3.64 (1H, m), 3.74-3.85 (1H, m), 3.94-4.06 (1H, m), 5.22 (2H, s), 7.09 (2H, d, J=9Hz), 7.15 (2H, d, J=9Hz)

### [Example 123]

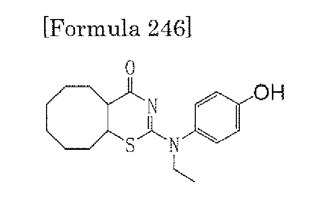

### Compound (I-123):

A mixture of 0. 18 g (0.4781 mole) of the compound (I-119), 2 ml of concentrated hydrochloric acid and 4 ml of acetic acid was stirred at room temperature for 14 hours. The reaction solution was concentrated under reduced pressure, and saturated aqueous NaHCO₃ and ethyl acetate were added to the residue, and extracted. The organic layer was washed with brine, dried over anhydrous sodium sulfate, and filtered, and the filtrate was concentrated under reduced pressure to give 0.30 g (69%) of a title compound as a colorless crystal. Diastereomer (form A)
1H-NMR(DMSO-d6): δ(ppm) 1.05 (3H, t, J=7Hz), 1.30-1.75 (8H, m), 1.80-1.95 (1H, m), 2.25-2.50 (4H, m), 3.25-3.30 (1H, m), 3.80 (2H, q, J=7Hz), 6.84 (2H, d, J=8,5Hz), 7.13 (2H, d, J=8.5Hz), 9.87 (1H, s).

The following compounds were synthesized as in Example 123.

### [Example 124]

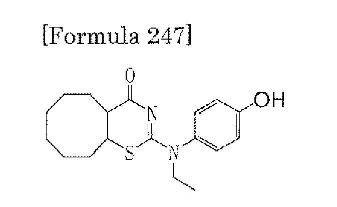

### Compound (I-124):

### Diastereomer (form B)

Yield: 75%
1H-NMR(DMSO-d6): δ(ppm) 1.05 (3H, t, J=7.5Hz), 1.30-1.95 (11H, m), 2.64-2.75 (1H, m), 3.55-3.72 (2H, m), 3.82 (2H, q, J=7.5Hz), 6.83 (2H, d, J=8.5Hz), 7.12 (2H, d, J=8.5Hz), 9.88 (1H, s).

### [Example 125]

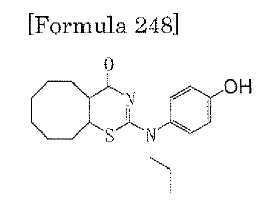

### Compound (I-125):

### Diastereomer (form A)

Yield: 69%
1H-NMR(CDCl3): δ(ppm) 0.84 (3H, t, J=7.5Hz), 1.35-2.00 (13H, m), 2.40-2.50 (1H, m), 2.55-2.68 (1H, m), 3.27-3.72 (1H, m), 3.67-3.78 (1H, m), 3.90-4.05 (1H, m), 6.55 (1H, brs), 6.98 (2H, d, J=8.5Hz), 7.08 (2H, d, J=8.5Hz).

### [Example 126]

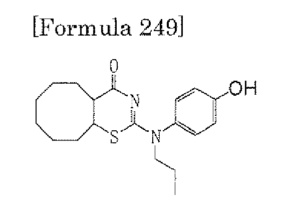

### Compound (I-126):

### Diastereomer (form B)

Yield: 75%
1H-NMR(CDCl3): δ(ppm) 0.86 (3H, t, J=7.5Hz), 1.42-2.05 (14H, m), 2.86-2.95 (1H, m), 3.50-3.63 (1H, m), 3.72-3.85 (1H, m), 3.94-4.05 (1H, m), 6.02 (1H, brs), 6.93 (2H, d, J=8.5Hz), 7.09 (2H, d, J=8.5Hz).

### [Example 127]

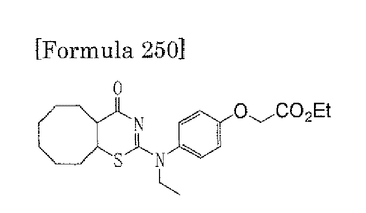

### Compound (1-127):

### Diastereomer (form A)

A mixture of 200 mg (0.6016 mmole) of the compound (I-123), 0.15 g (0.9024 mmole) of ethyl bromoacetate, 0.29 g (0.9024 mmole) of cesium carbonate and 2 ml of N,N-dimethylformamide was stirred at 50°C for 1 hour. Water and ethyl acetate were added to the reaction solution, and extracted. The organic layer was washed with brine. The organic layer was dried over anhydrous sodium sulfate, and filtered, and the solvent was concentrated under reduced pressure. The residue was purified by silicagel columnchromatography (n-hexane : ethyl acetate = 1 : 1) to give 0.24 g (95%) of a title compound as a colorless liquid.
1H-NMR(CDCl3): δ(ppm) 1.15 (3H, t, J=7Hz), 1.26 (3H, t, J=7Hz), 1.39-2.00 (11H, m), 2.39-2.50 (1H, m), 2.59-2.65 (1H, m), 3.28-3.39 (1H, m), 3.84-3.96 (1H, m), 4.00-4.10 (1H, m), 4.30 (2H, q, J=7Hz), 4.66 (2H, s), 6.96 (2H, d, J=9Hz), 7.16 (2H, d, J=9Hz).

The following compounds were synthesized as in Example 127.

### [Example 128]

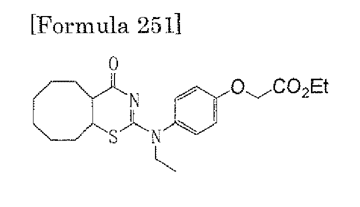

### Compound (I-128):

### Diastereomer (form B)

Yield: 79%
1H-NMR(CDCl3): δ(ppm) 1.15 (3H, t, J=7Hz), 1.26 (3H, t, J=7Hz), 1.45-1.80 (11H, m), 1.90-2.05 (1H, m), 2.85-2.94 (1H, m), 3.54-3.64 (1H, m), 3.88-4.07 (2H, m), 4.30 (2H, q, J=7Hz), 4.66 (2H, s), 6.96 (2H, d, J=9Hz), 7.17 (2H, d, J=9Hz).

### [Example 129]

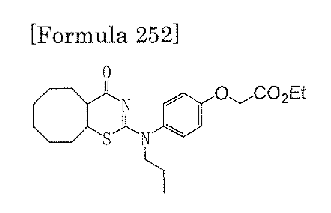

### Compound (I-129):

### Diastereomer (form A)

Yield: 100%
1H-NMR(CDCl3): δ(ppm) 0.88 (3H, t, J=7.5Hz), 1.32 (3H, t, J=7Hz), 1.39-2.00 (13H, m), 2.33-2.48 (1H, m), 2.52-2.70 (1H, m), 3.25-3.37 (1H, m), 3.77-3.80 (1H, m), 3.95-4.10 (1H, m), 4.30 (2H, q, J=7.5Hz), 4.66 (2H, s), 6.95 (2H, d, J=8.5Hz), 7.17 (2H, d, J=8.5Hz).

### [Example 130]

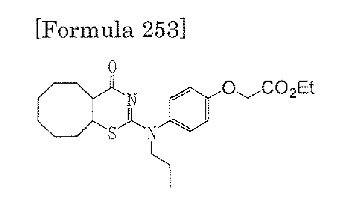

### Compound (I-130):

### Diastereomer (form B)

Yield: 65%
1H-NMR(CDCl3): δ(pom) 0.88 (3H, t, J=7.5Hz), 1.32 (3H, t, J=7Hz), 1.45-1.80 (13H, m), 1.88-2.05 (1H, m), 2.86-2.94 (1H, m), 3.53-3.64 (1H, m), 3.73-3.85 (1H, m), 3.94-4.06 (1H, m), 4.30 (2H, q, J=7.5Hz), 4.66 (2H, s), 6.96 (2H, d, J=9Hz), 7.16 (2H, d, J=9Hz).

### [Example 131]

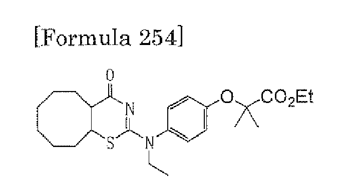

### Compound (I-131):

### Diastereomer (form A)

Yield: 70%
1H-NMR(CDCl3): δ(ppm) 1.14 (3H, t, J=7Hz), 1.24 (3H, t, J=7Hz), 1.40-2.00 (17H, m), 2.38-2.46 (1H, m), 2.57-2.70 (1H, m), 3.26-3.37 (1H, m), 3.83-3.98 (1H, m), 4.00-4.20 (1H, m), 4.25 (2H, q, J=7Hz), 6.86 (2H, d, J=9Hz), 7.09 (2H, d, J=9Hz).

### [Example 132]

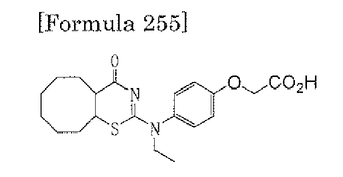

### Compound (I-132):

### Diastereomer (form A)

A mixture of 0.24 g (0.5734 mmole) of the compound (I-127), 1 ml of concentrated hydrochloric acid and 2 ml of acetic acid was stirred at room temperature for 18 hours. The reaction solution was concentrated under reduced pressure, saturated aqueous NaHCO₃ was added to the residue to adjust pH to 7, and concentrated under reduced pressure. Ethanol was added to the residue, the insoluble was filtered, and the filtrate was then concentrated under reduced pressure to give 0.17 g (76%) of a title compound as colorless amorphous substance. 1H-NMR(CDCl3): δ(ppm) 1.05 (3H, t, J=7Hz), 1.35-1.95 (11H, m), 2.25-2.50 (2H, m), 3.25-3.45 (1H, m), 3.81 (2H, q, J=7Hz), 4.13 (2H, s), 6.86 (2H, d, J=9Hz), 7.18 (2H, d, J=9Hz), 8.32 (1H, s).

The following compounds were synthesized as in Example 132.

### [Example 133]

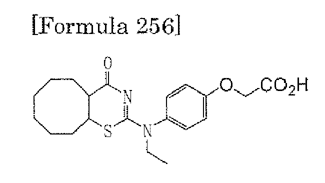

### Compound (I-133):

### Diastereomer (form B)

Yield: 41%
1H-NMR(CDCl3): δ(ppm) 1.05 (3H, t, J=7Hz), 1.35-2.00 (12H, m), 2.63-2.75 (1H, m), 3.60-370 (1H, m), 3.82 (2H, q, J=7Hz), 4.14 (2H, s), 6.87 (2H, d, J=8.5Hz), 7.17 (2H, d, J=8.5Hz), 8.33 (1H, s).

### [Example 134]

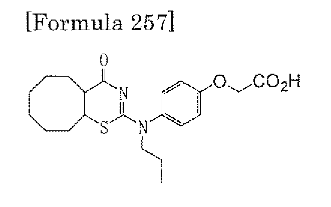

### Compound (I-134):

### Diastereomer (form A)

Yield: 96%
1H-NMR(DMSO-d6): δ(ppm) 0.82 (3H, t, J=7.5Hz), 1.33-2.00 (13H, m), 2.25-2.45 (2H, m), 3.30-3.35 (1H, m), 3.65-3.80 (2H, m), 4.11 (2H, s), 6.84 (2H, d, J=9Hz), 7.17 (2H, d, J=9Hz), 8.32 (1H, s).

### [Example 135]

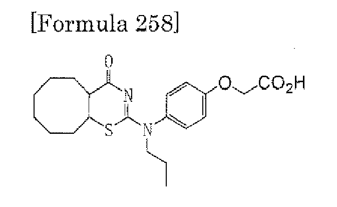

### Compound (I-135):

### Diastereomer (form B)

Yield: 100%
1H-NMR(DMSO-d6): δ(ppm) 0.82 (3H, t, J=7.5Hz), 1.35-1.95 (14H, m), 2.62-2.74 (1H, m), 3.58-3.85 (3H, m), 4.12 (2H, s), 6.85 (2H, d, J=9Hz), 7.17 (2H, d, J=9Hz), 8.33 (1H, s).

### [Example 136]

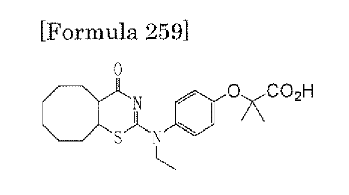

### Compound (I-136):

### Diastereomer (form A)

Yield: 51%
1H-NMR(CDCl3): δ(ppm) 1.05 (3H, t, J=7Hz), 1.35-1.96 (17H, m), 2.30-2.50 (2H, m), 3.25-3.35 (1H, m), 3.79 (2H, q, J=7Hz), 6.84 (2H, d, J=8.5Hz), 7.08 (2H, d, J=8.5Hz), 8.32 (1H, s).

### [Example 137]

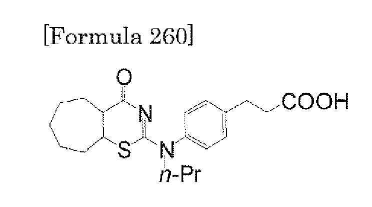

### Compound (I-137):

### Diastereomer (form A)

Yield: 100%
1H NMR(CDCl3) : δ(ppm) 0.88(3H, t, J = 7.5 Hz), 1.40-1.82(12H, m), 2.31-2.42(2H, m), 2.73(2H, t, J = 7.8 Hz), 3.03(2H, t, J = 7.8 Hz), 3.18-3.26(1H, m), 3.67-3.77(1H, m), 4.01-4.11(1H, m), 7.17(2H, d, J = 8.1 Hz), 7.30(2H, d J = 8.1. Hz)

### [Example 138]

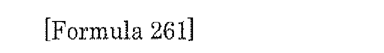

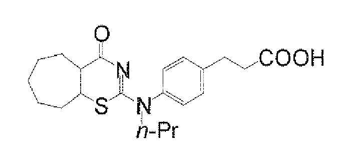

### Compound (I-138):

### Diastereomer (form B)

Yield: 100%
1H NMR(CDCl3) : δ(ppm) 0.88(3H, t, J = 7.2 Hz), 1.45-2.15(14H, m), 2.74(2H, t, J = 7.5 Hz), 3.04(2H, t, J = 7.5 Hz), 3.64-3.70(1H, m), 3.80-3.89(1H, m), 3.96-4.06(1H, m), 7.17(2H, d, J = 8.4 Hz), 7.31(2H, d J = 8.4 Hz)

### [Example 139]

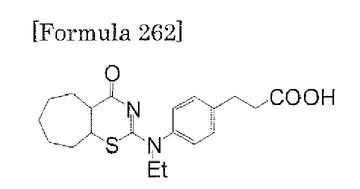

### Compound (I-139):

### Diastereomer (form A)

Yield: 76%
1H NMR(CDCl3) : δ(ppm) 1.15(3H, t, J = 7.2 Hz), 1.35-1.85(10H, m), 2.33-2.43(2H, m), 2.74(2H, t, J = 7.5 Hz), 3.03(2H, t, J = 7.5 Hz), 3.20-3.27(1H, m), 3.83-3.95(1H, m), 4.03-4.15(1H, m), 7.16(2H, d, J = 8.4 Hz), 7.30(2H, d J = 8.4 Hz)

### [Example 140]

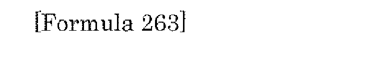

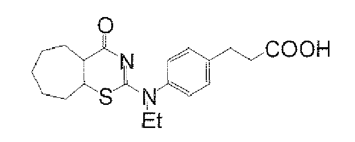

### Compound (I-140):

### Diastereomer (form B)

Yield: 90%
1H NMR(CDCl3) : δ(ppm) 1.16(3H, t, J = 7.2 Hz), 1.40-2.10(11H, m), 2.72-2.78(3H, m), 3.04(2H, t, J = 7.5 Hz), 3.64-3.70(1H, m), 3.97-4.07(1H, m), 7.17(2H, d, J = 8.4 Hz), 7.32(2H, d J = 8.4 Hz)

### [Example 141]

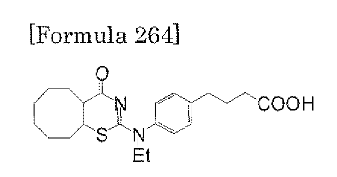

### Compound (I-141):

### Diastereomer (form A)

Yield: 86%
1H NMR(CDCl3) : δ(ppm) 1.16(3H, t, J = 7.2 Hz), 1.40-2.05(14H, m), 2.36-2.49(3H, m), 2.59-2.76(3H, m), 3.30-3.38(1H, m), 3.86-4.12(2H, m), 7.15(2H, d, J = 8.1 Hz), 7.26-7.29(2H, m)

### [Example 142]

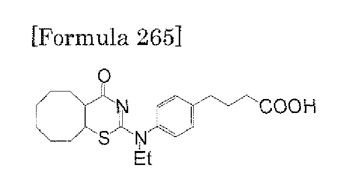

### Compound (I-142):

### Diastereomer (form B)

Yield: 83%
1H NMR(CDCl3) : δ(ppm) 1.16(3H, t, J = 7.2 Hz), 1.45-1.80(12H, m), 1.96-2.06(3H, m), 2.42(2H, t, J = 7.2 Hz), 2.74(2H, t, J = 7.5 Hz), 2.89-2.94(1H, m), 3.55-3.63(1H, m), 3.90-4.10(2H, m), 7.15(2H, d, J = 7.8 Hz), 7.26-7.30(2H, m)

### [Example 143]

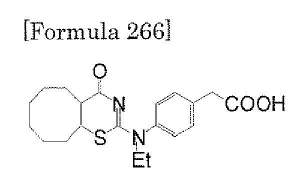

### Compound (I-143):

### Diastereomer (form A)

Yield: 91%
1H NMR(CDCl3) : δ(ppm) 1.15(3H, t, J = 7.2 Hz), 1.45-2.00(12H, m), 2.42-2.49(1H, m), 2.59-2.66(1H, m), 3.32-3.39(1H, m), 3.72(2H, s), 3.85-3.97(1H, m), 4.00-4.12(1H, m), 7.21(2H, d, J = 8.1 Hz), 7.40(2H, d J = 8.4 Hz)

### [Example 144]

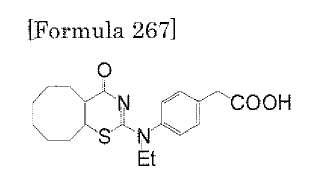

### Compound (I-144):

### Diastereomer (form B)

Yield: 100%
1H NMR(CDCl3): δ(ppm) 1.17(3H, t, J = 7.2 Hz), 1.45-2.05(13H, m), 2.88-2.94(1H, m), 3.57-3.64(1H, m), 3.74(1H, s), 3.94-4.11(2H, m), 7.22(2H, d, J = 8.4 Hz), 7.41(2H, d J = 8.1 Hz)

### [Example 145]

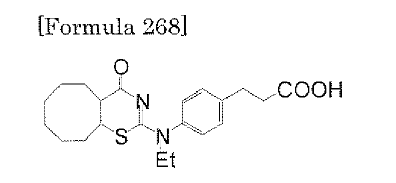

### Compound (I-145):

### Diastereomer (form A)

Yield: 83%
1H NMR(CDCl3) : δ(ppm) 1.16(3H, t, J = 7.2 Hz), 1.50-2.05(13H, m), 2.73(2H, t, J = 7.5 Hz), 2.89-2.95(1H, m), 3.03(2H, t, J = 7.5 Hz), 3.57-3.63(1H, m), 3.90-4.11(2H, m), 7.16(2H, d, J = 8.4 Hz), 7.31(2H, d J = 8.4 Hz)

### [Example 146]

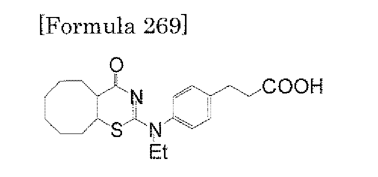

### Compound (I-146):

### Diastereomer (form B)

Yield: 87%
1H NMR(CDCl3) : δ(ppm) 1.16(3H, t, J = 7.2 Hz), 1.50-2.05(13H, m), 2.73(2H, t, J = 7.5 Hz), 2.89-2.95(1H, m), 3.03(2H, t, J = 7.5 Hz), 3.57-3.63(1H, m), 3.90-4.11(2H, m), 7.16(2H, d, J = 8.4 Hz), 7.31(2H, d J = 8.4 Hz)

### [Example 147]

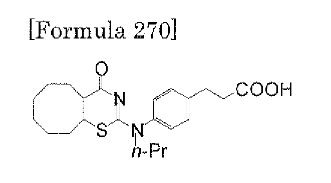

### Compound (I-147):

### Diastereomer (form A)

Yield: 67%
1H NMR(CDCl₃) : δ(ppm) 0.88(3H, t, J = 7.5 Hz), 1.40-2.05(15H, m), 2.58-2.76(3H, m), 3.03(2H, t, J = 7.5 Hz), 3.11-3.38(1H, m), 3.71-4.12(2H, m), 7.16(2H, d, J = 8.1 Hz), 7.23-7.33(2H, m)

### [Example 148]

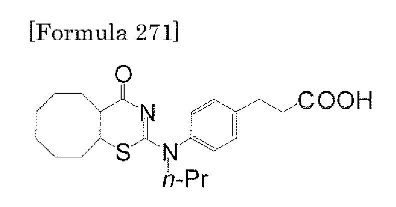

### Compound (I-148):

### Diastereomer (form B)

Yield: 100%
1H NMR(CDCl3) : δ(ppm) 0.88(3H, t, J = 7.5 Hz), 1.40-1.80(14H, m), 1.85-2.05(1H, m), 2.73(2H, t, J = 7.5 Hz), 2.80-2.94(1H, m), 3.03(2H, t, J = 7.5 Hz), 3.57-3.62(1H, m), 3.76-3.85(1H, m), 3.97-4.07(1H, m), 7.16(2H, d, J = 8.4 Hz), 7.30(2H, d J = 8.4 Hz)

### [Example 149]

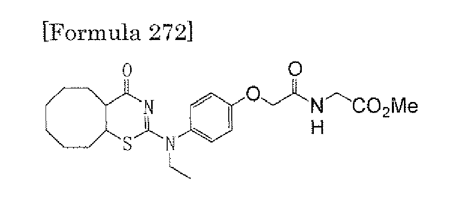

### Compound (I-149):

### Diastereomer (form A)

A mixture of 0.12 g (0.3073 mmole) of the compound (I-133), 39 mg (0.3073 mmole) of glycine methyl ester/hydrochloride, 84 mg (0.615 mmole) of HOBt, 62 mg (0.3227 mmole) of WSC/hydrochloride, 62 mg (0.615 mmole) of triethylamine and 1.2 ml of dimethylformamide was stirred at room temperature for 23 hours. Water and ethyl acetate were added to the reaction solution, and extracted. The organic layer was washed with brine, dehydrated with anhydrous sodium sulfate, and filtered, and the filtrate was concentrated under reduced pressure. The residue was purified by silicagel columnchromatography (ethyl acetate) to give 60 mg (42%) of a title compound as a colorless liquid.
1H-NMR(CDCl3): δ(ppm) 1.16 (3H, t, J=7Hz), 1.40-2.05 (11H, m), 2.40-2.50 (1H, m), 2.57-2.70 (1H, m), 3.30-3.40(1H, m), 3.80 (3H, s), 3.85-4.00 (1H, m), 4.00-4.15 (1H, m), 4.17 (2H, d, J=9Hz), 4.59 (2H, s), 7.00 (2H, d, J=9Hz), 7.09 (1H, brs), 7.21 (2H, d, J=9Hz).

The following compounds were synthesized as in Example 66.

### [Example 150]

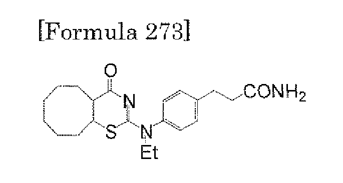

### Compound (I-150):

### Diastereomer (form A)

Yield: 21%
1H NMR(CDCl3) : δ(ppm) 1.16(3H, t, J = 7.2 Hz), 1.40-2.00(11H, m), 2.41-2.64(4H, m), 3.04(2H, t, J = 7.5 Hz), 3.30-3.38(1H, m), 3.85-3.97(1H, m), 4.02-4.16(1H, m), 5.30-5.50(2H, br, NH2), 7.16(2H, d, J = 8.4 Hz), 7.31(2H, d, J = 8.7 Hz)

### [Example 151]

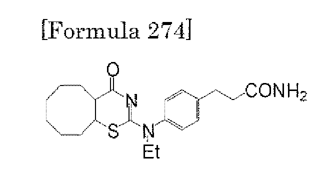

### Compound (I-151):

### Diastereomer (form B)

Yield: 96%
1H NMR(CDCl3) : δ(ppm) 1.16(3H, t, J = 7.2 Hz), 1.50-2.10(12H, m), 2.58(2H, t, J = 7.8Hz), 2.88-2.93(1H, m), 3.04(2H, t, J = 7.8 Hz), 3.55-3.62(1H, m), 3.92-4.01(2H, m), 5.86(2H, br, NH2), 7.15(2H, d, J = 8.1 Hz), 7.31(2H, d, J = 8.4 Hz)

### [Example 152]

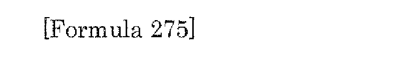

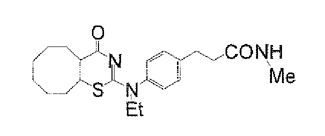

### Compound (I-152):

### Diastereomer (form B)

Yield: 50%
1H NMR(CDCl3) : δ(ppm) 1.16(3H, t, J = 7.2 Hz), 1.45-2.10(12H, m), 2.51(2H, t, J= 7.8Hz), 2.80(3H, d, J = 8.1 Hz), 2.86-2.93(1H, m), 3.04(2H, t, J = 7.8 Hz), 3.55-3.64(1H, m), 3.90-4.15(2H, m), 5.49(1H, br, NH), 7.15(2H, d, J = 8.1 Hz), 7.29(2H, d, J = 8.1 Hz)

### [Example 153]

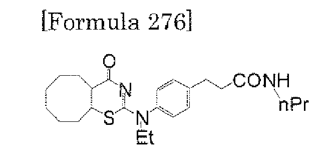

### Compound (I-153):

### Diastereomer (form B)

Yield: 19%
1H NMR(CDCl3) : δ(ppm) 0.87(3H, t, J = 7.2 Hz), 1.15(3H, t, J = 6.9 Hz), 1.41-2.05(14H, m), 2.50(2H, t, J = 7.5 Hz), 2.88-2.94(1H, m), 3.04(2H, t, J = 7.5Hz), 3.20(2H, q, J = 6.7 Hz), 3.56-3.61(1H, m), 3.90-4.08(2H, m), 5.40(1H, br, NH), 7.15(2H, d, J = 8.1 Hz), 7.30(2H, d, J = 8.4 Hz)

### [Example 154]

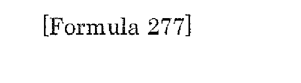

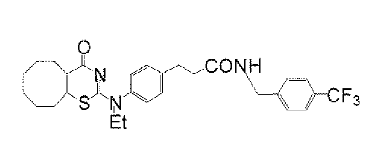

### Compound (1-154):

### Diastereomer (form B)

Yield: 15%
1H NMR(CDCl3) : δ(ppm) 1.15(3H, t, J = 7.2 Hz), 1.40-2.05(12H, m), 2.59(2H, t, J = 7.5 Hz), 2.86-2.93(1H, m), 3.07(2H, t, J = 7.5Hz), 3.55-3.62(1H, m), 3.85-4.07(2H, m), 4.49(2H, d, J = 5.7 Hz), 6.01(1H, br, NH), 7.14(2H, d, J = 8.1 Hz), 7.27-7.36(4H, m), 7.56(2H, d, J = 8.1 Hz)

### [Example 155]

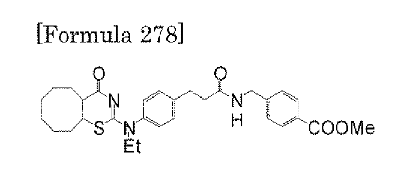

### Compound (I-155):

### Diastereomer (form B)

Yield: 74%
1H NMR(CDCl3) : δ(ppm) 1.15(3H, t, J = 7.2 Hz), 1.40-2.10(12H, m), 2.59(2H, t, J = 7.8 Hz), 2.88-2.94(1H, m), 3.08(2H, t, J = 7.8Hz), 3.55-3.61(1H, m), 3.91-4.08(5H, m), 4.49(2H, d, J = 6.0 Hz), 5.82(1H, br, NH), 7.15(2H, d, J = 8.4 Hz), 7.26-7.32(4H, m), 7.98(2H, d, J = 8.4 Hz)

### [Example 156]

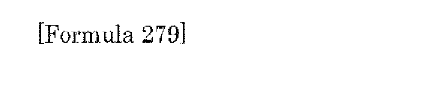

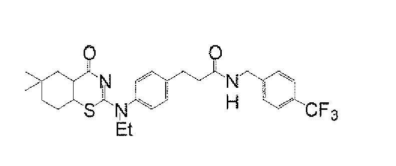

### Compound (I-156):

### Diastereomer (form B)

Yield: 19%
1H NMR(CDCl3) : δ(ppm) 0.94-0.96(6H, m), 1.10-2.05(9H, m), 2.60(2H, t, J = 6.9 Hz), 2.76(1H, dt, J = 12.6 Hz, J = 4.0 Hz), 3.07(2H, t, J = 6.9 Hz), 3.66-3.72(1H, m), 3.95-4.10(2H, m), 4.49(2H, d, J = 4.5 Hz), 6.03(1H, br, NH), 7.10-7.20(2H, m), 7.29-7.45(4H, m), 7.55-7.58(2H, m)

### (Reference Example 14)

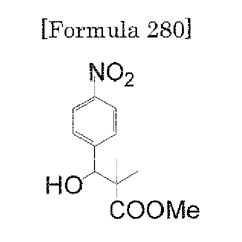

### Compound (I'-95):

To a mixture of 2.00 g (19.6 mmole) of methyl isobutanoate and 30 ml of anhydrous tetrahydrofuran was added dropwise 14.7 ml (29.4 mmole) of lithium diisopropylamine at -78°C under argon atmosphere, and the mixture was stirred for 1.5 hours. Thereafter, 2.96 g (19.6 mmole) of 4-nitrobenzaldehyde was added dropwise thereto, and the mixture was stirred at room temperature for 15.5 hours. Saturated aqueous NH₄Cl was added to the reaction solution, and extracted with ethyl acetate. The organic layer was washed with brine, dehydrated with anhydrous sodium sulfate, and filtered, and the solvent was concentrated under reduced pressure. The residue was purified by silicagel columnchromatography (n-hexane : ethyl acetate = 3 : 1) to give 3.00 g (60%) of a title compound as a pale yellow crystal.
1H NMR(CDCl3) : δ(ppm) 1.14(3H, s), 1.15(3H, s), 3.41(1H, d, J = 4.2 Hz), 3.75(3H, s), 5.01(1H, d, J = 4.2 Hz), 7.49(2H, d, J = 9.0 Hz), 8.19(2H, d, J = 8.7 Hz)

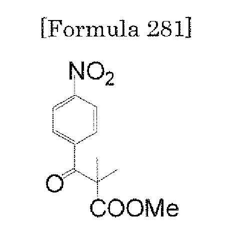

### Compound (I'-96):

A mixture of 3.00 g (11.8 mmole) of the compound (I'-95), 8.91 g (23.7 mmole) of pyridinium dichromate and 40 ml of dichloromethane was heated to reflux with stirring for 19 hours. The reaction solution was filtered through Celite, and water was added to the filtrate, and extracted with dichloromethane. The organic layer was washed with brine, dehydrated with anhydrous sodium sulfate, and filtered, and the solvent was concentrated under reduced pressure. The residue was purified by silicagel columnchromatography (n-hexane : ethyl acetate = 3 : 1) to give 2.74 g (92%) of a title compound as a pale yellow crystal.
1H NMR(CDCl3) : δ(ppm) 1.57(6H, s), 3.67(3H, s), 7.97(2H, d, J = 9.0 Hz), 8.28(2H, d, J = 9.0 Hz)

### (Reference Example 15)

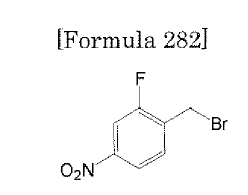

### Compound (I'-97):

A mixture of 3 g (19.33 mmole) of 2-fluoro-4-nitrotoluene, 3.6 g (20.3 mmole) of N-bromosuccinimide, 63 mg of α,α-azobisisobutyronitrile and 15 ml of carbon tetrachloride was heated to reflux with stirring for 8 hours. Water was added to the reaction solution, the mixture was extracted, and the organic layer was washed with brine. The organic layer was dehydrated with anhydrous sodium sulfate, and filtered, and the solvent was concentrated under reduced pressure. The residue was purified by silicagel columnchromatography (n-hexane) to give 3.57 g (79%) of a title compound.
1H-NMR(CDCl3): δ(ppm) 4.53 (2H, s), 7.61 (1H, t, J=8.0Hz), 7.96 (1H, dd, J=9.0, 2.0Hz), 8.04 (1H, dd, J=8.0, 2.0Hz).

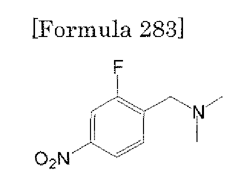

### Compound (I'-98):

A mixture of 1.5 g (6.41 mmole) of the compound (I'-97), 2.33 g (23.1 mmole) of triethylamine, 0.63 g (7.69 mmole) of dimethylamine hydrochloride and 15 ml of N,N-dimethylformamide was stirred at room temperature for 18 hours. Water and ethyl acetate were added to the reaction solution, this was extracted, and the organic layer was washed with brine. The organic layer was dehydrated with anhydrous sodium sulfate, and filtered, and the solvent was concentrated under reduced pressure to give 1.21g (95%) of a title compound as a brown liquid.
1H NMR(CDCl3) : δ(ppm) 2.29(6H, s), 3.57(2H, s), 7.62(1H, t, J = 7.8 Hz), 7.92(1H, dd, J = 9.6 Hz, J = 2.1 Hz), 8.01-8.05(1H, m)

The following compounds were synthesized as in Compound (I'-98).

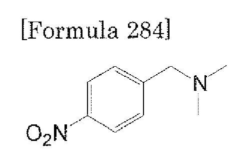

### Compound (I'-99):

Yield: 100%
1H NMR(CDCl3) : δ(ppm) 2.26(6H, s), 3.51(2H, s), 7.49(2H, d, J = 9.0 Hz), 8.18(2H, d, J = 8.7 Hz)

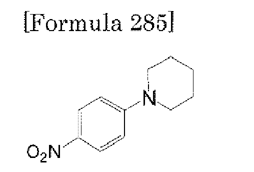

### Compound (I'-100):

Yield: 100%
1H NMR(CDCl3) : δ(ppm) 1.66-1.72(6H, m), 3.42-3.50(4H, m), 6.79(2H, d, J = 9.3 Hz), 8.10(2H, d, J = 9.6 Hz)

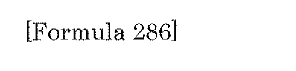

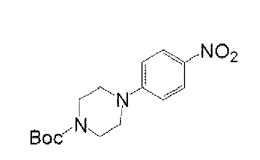

### Compound (I'-101):

Yield: 79%
1H NMR(CDCl3) : δ(ppm) 1.49(9H, s), 3.40-3.44(4H, m), 3.56-3.63(4H, m), 6.82(2H, d, J = 9.6 Hz), 8.14(2H, d, J = 9.6 Hz)

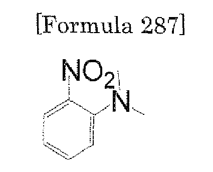

### Compound (I'-102):

Yield: 100%
1H NMR(CDCl3) : δ(ppm) 2.90(6H, s), 6.80-6.85(1H, m), 7.02(1H, dd, J = 8.4 Hz, J = 1.2 Hz), 7.38-7.43(1H, m), 7.77(1H, dd. J = 8.4 Hz, J = 1.5 Hz)

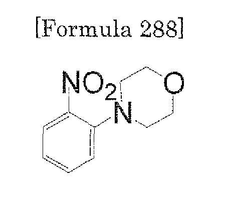

### Compound (I'-103):

Yield: 100%
1H NMR(CDCl3) : δ(ppm) 3.06(4H, t, J = 4.5 Hz), 3.85(4H, t, J = 4.5 Hz), 7.09(1H, t, J = 7.5 Hz), 7.15(1H, d, J = 8.1 Hz), 7.51(1H, t, J = 7.8 Hz), 7.77(1H, dd. J = 8.1 Hz, J = 1.5 Hz)

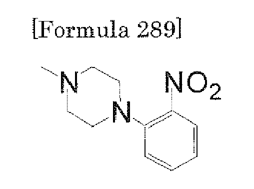

### Compound (I'-104):

Yield: 80%
1H NMR(CDCl3) : δ(ppm) 2.37(3H, s), 2.59(4H, t, J = 4.7 Hz), 3.10(4H, t, J = 4.7 Hz), 7.04(1H, t, J = 7.7 Hz), 7.15(1H, dd. J = 8.4 Hz, J = 1.2 Hz), 7.48(1H, t, J = 7.7 Hz), 7.75(1H, dd. J = 8.1 Hz, J = 1.5 Hz)

### (Reference Example 16)

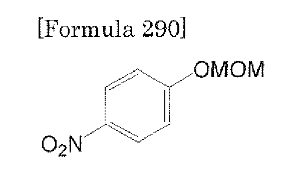

### Compound (I'-105):

To a mixture of 10 g (0.07189 mole) of 4-nitrophenol, 31.3 ml (0.1797 mole) of diisopropylethylamine and 50 ml of THF was added dropwise 13.6 ml (0.1797 mole) of 2-methoxymethoxy chloride under ice-cooling, and the mixture was stirred at room temperature for 2 hours. Water and ethyl acetate were added to the reaction solution, this was extracted, and the organic layer was then washed with brine. The organic layer was dehydrated with anhydrous sodium sulfate, and filtered, and the solvent was concentrated under reduced pressure. The residue was purified by silicagel columnchromatography (n-hexane : ethyl acetate = 3 : 1) to give 14.3 g (100%) of 4-methoxymethoxynitrobenzene as a pale yellow liquid.
1H-NMR(CDCl3) : δ(ppm) 3.50 (3H, s), 5.26 (2H, s), 7.11(2H, d, J=9Hz), 8.21 (2H, d, J=9Hz).

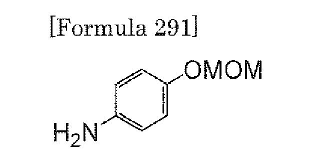

### Compound (I'-106):

A mixture of 14.3 g (0.07148 mole) of 4-methoxymethoxynitrobenzene, 0.14 g of platinum (IV) oxide and 70 ml of methanol was stirred at room temperature for 20 hours under hydrogen atmosphere. The reaction solution was filtered, and the filtrate was concentrated to give 9.05 g (82%) of 4-methoxymethoxyaniline as a red brown liquid.
1H-NMR(CDCl₃): δ(ppm) 3.46 (2H, brs), 3.47 (3H, s), 5.08 (2H, s), 6.63 (2H, d, J=9Hz), 6.87 (2H, d, J=9Hz).

The following compounds were synthesized as in Compound (I'-106).

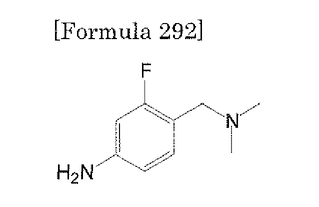

### Compound (I'-107):

Yield: 100%
1H NMR(CDCl3) : δ(ppm) 2.23(6H, s), 3.37(2H, s), 3.72(2H, s(br), NH2), 6.35-6.44(2H, m), 7.06(1H, t, J = 8.3 Hz)

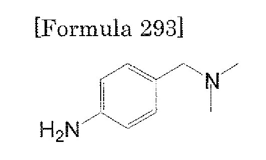

### Compound (I'-108):

Yield: 100%
1H NMR(CDCl3) : δ(ppm) 2.21(6H, s), 3.31(2H, s), 3.62(2H, s(br), NH2), 6.65(2H, d, J = 8.4 Hz), 7.08(2H, d, J = 8.1 Hz)

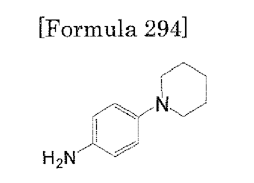

### Compound (I'-109):

Yield: 100%
1H NMR(CDCl3) : δ(ppm) 1.50-1.62(2H, m), 1.71(4H, quintet, J = 5.6 Hz), 2.98(4H, t, J = 5.4 Hz), 3.40(2H, s(br), NH2), 6.34(2H, d, J = 8.7 Hz), 6.83(2H, d, J = 8.7 Hz)

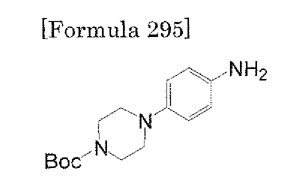

### Compound (I'-110):

Yield: 100%
1H NMR(CDCl3) : δ(ppm) 1.48(9H, s), 2.96(4H, t, J = 8.1 Hz), 3.45(3H, s(br), NH2), 3.56(4H, t, J = 8.1 Hz), 6.65(2H, d, J = 8.1 Hz), 6.81(2H, d, J = 8.1 Hz)

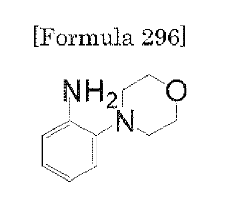

### Compound (I'-111):

Yield: 93%
1H NMR(CDCl3) : δ(ppm) 2.92(4H, t, J = 4.7 Hz), 3.85(4H, t, J = 4.7 Hz), 3.98(2H, br, NH2), 6.74-6.79(2H, m), 6.93-7.03(2H, m)

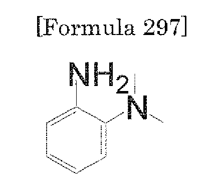

### Compound (I'-112):

Yield: 88%
1H NMR(CDCl3) : δ(ppm) 2.67(6H, s), 3.97(2H, br, NH2), 6.71-6.78(2H, m), 6.91(1H, t, J = 7.5 Hz), 7.01(1H, d, J = 7.8 Hz)

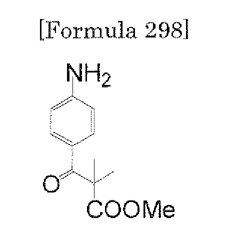

### Compound (I'-113):

Yield: 100%
1H NMR(CDCl3) : δ(ppm) 1.52(6H, s), 3.64(3H, s), 4.11(2H, s(br), NH2), 6.61(2H, d, J = 9.0 Hz), 7.70(2H, d, J = 9.0 Hz)

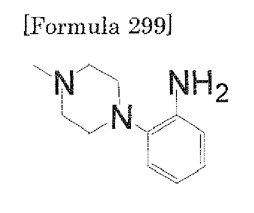

### Compound (I'-114):

Yield: 100%
1H NMR(CDCl3) : δ(ppm) 2.38(3H, s), 2.56-2.66(4H, m(br)), 2.97(4H, t, J = 4.7 Hz), 3.97(2H, s(br), NH2), 6.71-6.78(2H, m), 6.94(1H, t, J = 7.7 Hz), 7.02(1H, dd. J = 8.4 Hz, J = 1.5 Hz)

### (Reference Example 17)

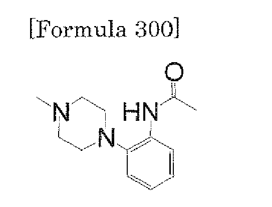

### Compound (I'-115):

To a mixture of 1.00 g (5.23 mmole) of the compound (I'-114), 0.58 g (5.75 mmole) of triethylamine and 15 ml of tetrahydrofuran was added 0.45 g (5.75 mmole) of acetyl chloride under ice-cooling, and the mixture was stirred at room temperature for 14.5 hours. Water and ethyl acetate were added to the reaction solution, this was extracted, the organic layer was washed with brine, dehydrated with anhydrous sodium sulfate, and filtered, and the solvent was concentrated under reduced pressure. The residue was purified by silicagel columnchromatography (ethyl acetate) to give 0.65 g (54%) of a title compound as a purple liquid.
1H NMR(CDCl3) : δ(ppm) 2.21(3H, s), 2.41(3H, s), 2.60-2.74(4H, br), 2.93(4H, t, J = 4.8 Hz), 7.06(1H, dd, J = 7.5 Hz, J = 1.5 Hz), 7.12-7.20(2H, m), 8.34(1H, d. J = 8.4 Hz), 8.46(1H, s(br), NH)

The following compounds were synthesized as in Reference Example 17.

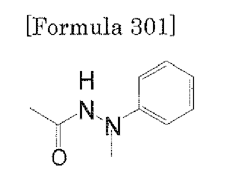

### Compound (I'-116):

Yield: 100%
1H-NMR(CDCl3): δ(ppm) 2.04, 2.07 (3H, s), 2.42 (1H, s), 3.16, 3.17 (3H, s), 6.80-6.91 (3H, m), 7.21-7.33 (2H, m)

### (Reference Example 18)

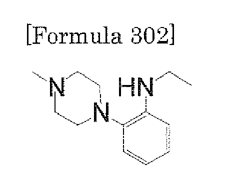

### Compound (I'-117):

To a mixture of 0.65 g (2.80 mmole) of the compound (I'-115) and 10 ml of anhydrous tetrahydrofuran was added 0.32 g (18.4 mmole) of aluminum lithium hydride under ice-cooling, and the mixture was stirred at room temperature for 24 hours. To the reaction solution was added 2.27 ml of water under ice-cooling, and the mixture was stirred at room temperature for 2 hours. The insoluble was filtered, the filtrate was washed with brine, dehydrated with anhydrous sodium sulfate, and filtered, and the solvent was concentrated under reduced pressure to give 0.14 g (23%) of a title compound as a red brown liquid.
1H NMR(CDCl3) : δ(ppm) 1.29(3H, t, J = 7.2 Hz), 2.38(3H, s), 2.50-2.68(4H, br), 2.93(4H, t, J = 4.6 Hz), 3.10-3.20(2H, m), 4.54(1H, s(br), NH), 6.61-6.70(2H, m), 7.00-7.05(2H, m)

The following compounds were synthesized as in Reference Example 18.

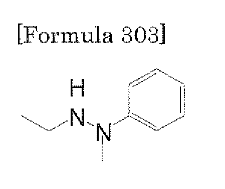

### Compound (I'-118):

Yield: 66%
1H-NMR(CDCl3): δ(ppm) 1.11 (3H, t, J=7.5Hz), 2.93 (2H, q, J=7.5Hz), 3.04 (3H, s), 6.73-6.78 (1H, m), 6.96-7.01 (2H, m), 7.19-7.24 (2H, m) (NH : not detected)

### (Reference Example 19)

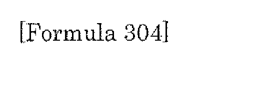

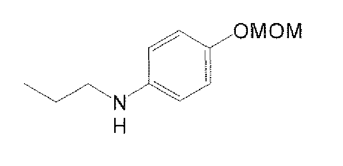

### Compound (I'-119):

A mixture of 2.0 g (0.01306 mole) of the compound (I'-106), 1.40 ml (0.014366 mole) of propyl iodide, 5.42 g (0.03918 mole) of potassium carbonate and 10 ml of N,N-dimethylformamide was stirred at room temperature for 18 hours. Water and ethyl acetate were added to the reaction solution, this was extracted, and the organic layer was washed with brine. The organic layer was dehydrated with anhydrous sodium sulfate, and filtered, and the solvent was concentrated under reduced pressure. The residue was purified by silicagel columnchromatography (n-hexane : ethyl acetate = 7 : 1) to give 1.47 g (58%) of a title compound as a pale yellow liquid.
1H-NMR(CDCl₃): δ(ppm) 0.99 (3H, t, J=7.5Hz), 1.62 (2H, sextet, J=7.5Hz), 3.04 (2H, t, J=7.5Hz), 3.34 (1H, brs), 3.48 (3H, s), 5.07 (2H, s), 6.55 (2H, d, J=9Hz), 6.90 (2H, d, J=9Hz).

The following compounds were synthesized as in Reference Example 19.

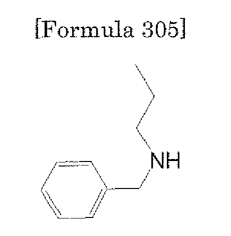

### Compound (I'-120):

Yield: 45%
1H NMR(CDCl3) : δ(ppm) 0.92(3H, t, J = 7.4 Hz), 1.54(2H, sextet, J = 7.2 Hz), 1.64(1H, s(br), NH), 2.61(2H, t, J = 7.2 Hz), 3.80(2H, s), 7.22-7.36(5H, m)

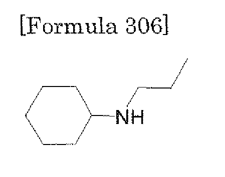

### Compound (I'-121):

Yield: 64%
1H NMR(CBCl3) : δ(ppm) 0.91(3H, t, J = 7.2 Hz), 1.03-1.90(13H, m), 2.33-2.41(1H, m), 2.58(2H, t, J = 7.4Hz)

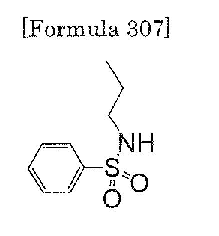

### Compound (I'-122):

Yield: 45%
1H NMR(CDCl3) : δ(ppm) 0.87(3H, t, J = 7.4 Hz), 1.49(2H, sextet, J = 7.3 Hz), 2.93(2H, q, J = 6.8 Hz), 4.35(1H, s(br), NH), 7.49-7.59(3H, m), 7.87(2H, d, J = 6.6 Hz)

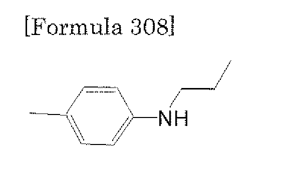

### Compound (I'-123):

Yield: 49%
1H NMR(CDCl3) : δ(ppm) 0.99(3H, t, J = 7.5 Hz), 1.63(2H, sextet, J= 7.2 Hz), 2.17(3H, s), 3.06(2H, t, J = 7.2 Hz), 3.49(1H, s(br), NH), 6.54(2H, d, J = 6.9 Hz), 6.98(2H, d, J = 7.8 Hz)

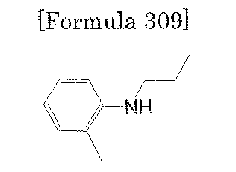

### Compound (I'-124):

Yield: 58%
1H NMR(CDCl3) : δ(ppm) 1.02(3H, t, J = 7.5 Hz), 1.69(2H, sextet, J = 7.5 Hz), 2.14(3H, s), 3.13(2H, t, J = 7.5 Hz), 3.47(1H, s(br), NH), 6.60-6.67(2H, m), 7.04-7.1.5(2H, m)

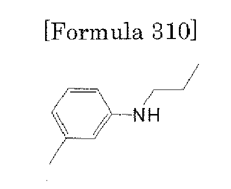

### Compound (I'-125):

Yield: 54%
1H NMR(CDCl3) : δ(ppm) 0.99(3H, t, J = 7.5 Hz), 1.63(2H, sextet, J = 7.5 Hz), 2.28(3H, s), 3.07(2H, t, J = 7.5 Hz). 3.57(1H, s(br), NH), 6.41-6.44(2H, s), 6.51(1H, d, J = 7.5 Hz), 7.06(1H, t, J = 7.5 Hz)

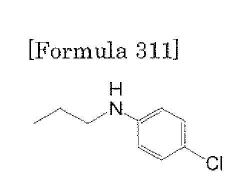

### Compound (I'-126):

Yield: 59%
1H NMR(CDCl3) δ(ppm) 0.99(3H, t, J = 7.5 Hz), 1.63(2H, sextet, J = 7.4 Hz), 3.05(2H, t, J = 7.2 Hz), 3.63(1H, s(br), NH), 6.52(2H, d, J = 8.4 Hz), 7.11(2H, d, J = 8.4 Hz)

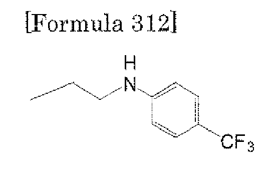

### Compound (I'-127):

Yield: 59%
1H NMR(CDCl3) : δ(ppm) 1.01(3H, t, J = 7.4 Hz), 1.65(2H, sextet, J = 7.3 Hz), 3.11(2H, q, J = 6.6 Hz), 3.98(1H, s(br), NH), 6.58(2H, d, J = 8.4 Hz), 7.39(2H, d, J = 8.4 Hz)

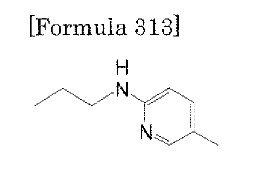

### Compound (I'-128):

Yield: 20%
1H NMR(CDCl3) : δ(ppm) 0.99(3H, t, J = 7.5 Hz), 1.63(2H, sextet, J = 7.3 Hz). 2.17(3H, s), 3.19(2H, q, J = 6.6 Hz), 4.34(1H, s(br), NH), 6.32(1H, d, J = 8.4 Hz), 7.23-7.28(1H, m), 7.90(1H, s)

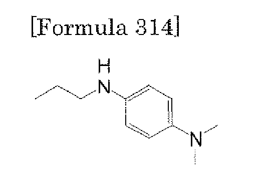

### Compound (I'-129):

Yield: 42%
1H NMR(CDCl3)50C : δ(ppm) 0.98(3H, t, J = 7.2 Hz), 1.48(1H, s(br), NH), 1.62(2H, sextet, J = 7.2 Hz), 2.81.(6H, s), 3.04(2H, t, J = 7.2 Hz), 6.58(2H, d, J = 8.7 Hz), 6.73(2H, d, J = 8.7 Hz)

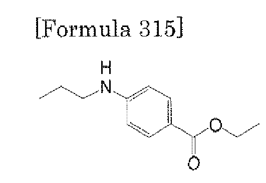

### Compound (I'-130):

Yield: 50%
1H NMR(CDCl3) : δ(ppm) 1.01(3H, t, J = 7.5 Hz), 1.36(3H, t, J = 7.2 Hz), 1.66(2H, sextet, J = 7.2 Hz), 3.13(2H, q, J = 6.6 Hz), 4.09(1H, s(br), NH), 4.31(2H, q, J = 7.2 Hz), 6.54(2H, d, J = 8.7 Hz), 7.86(2H, d J = 8.7 Hz)

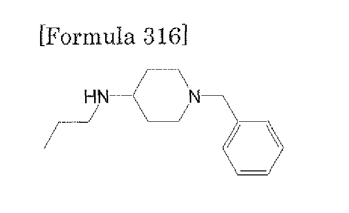

### Compound (I'-131):

Yield: 51%
1H NMR(CDCl3) : δ(ppm) 0.91(3H, t, J = 7.5 Hz), 1.31-1.79(5H, m), 1.83-1.87(2H, m), 2.01(2H, td, J = 11.7 Hz, J = 2.4 Hz), 2.44(1H, tt, J = 1.0.7 Hz, J = 3.9 Hz), 2.58(2H, t, J = 7.5 Hz), 2.83-2.86(2H, m), 3.50(2H, s), 7.22-7.32(5H, m)

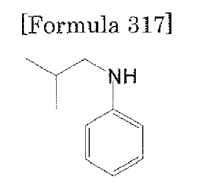

### Compound (I'-132):

Yield: 52%
1H NMR(CDCl3) : δ(ppm) 0.98(6H, d, J = 6.6 Hz), 1.89(1H, nonet, J = 6.7 Hz), 2.93(2H, d, J =6.6 Hz), 3.69(1H, s(br), NH), 6.60(2H, d, J = 7.8 Hz), 6.67(1H, t, J = 7.4 Hz), 7.16(2H, t, J = 8.0 Hz)

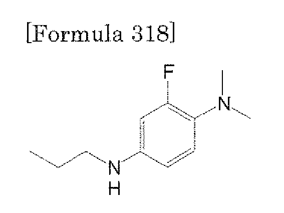

### Compound (I'-133):

Yield: 41%
1H NMR(CDCl3) : δ(ppm) 0.99(3H, t, J = 7.5 Hz), 1.62(2H, sextet, J = 7.5 Hz), 2.72(6H, s), 3.02(2H, t, J = 7.2 Hz), 3.49(1H, s(br), NH), 6.30-6.38(2H, m), 6.84(1H, t, J = 8.4 Hz)

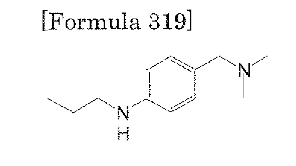

### Compound (I'-134):

Yield: 20%
1H NMR(CDCl3) : δ(ppm) 1.00(3H, t, J = 7.5 Hz), 1.64(2H, sextet, J = 7.2 Hz), 2.21(6H, s), 3.07(2H, t, J = 7.2 Hz), 3.31(2H, s), 3.60(1H, s(br), NH), 6.56(2H, d, J = 8.4 Hz), 7.09(2H, d, J = 8.7 Hz)

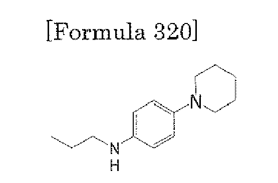

### Compound (I'-135):

Yield: 48%
1H NMR(CDCl3) : δ(ppm) 0.99(3H, t, J = 7.5 Hz), 1.50-1.75(9H, m), 2.95-3.06(6H, m), 6.58(2H, d, J = 8.7 Hz), 6.87(2H, d, J = 9.0 Hz)

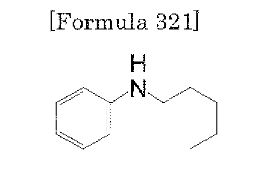

### Compound (I'-136):

Yield: 40%
1H NMR(CDCl3) : δ(ppm) 0.92(3H, t, J = 6.9 Hz), 1.35-1.41(4H, m), 1.57-1.65(2H, m), 3.10(2H, t, J = 6.9 Hz), 3.59(1H, s(br), NH), 6.60(2H, d, J = 7.5 Hz), 6.68(1H, t, J = 7.5 Hz), 7.17(2H, t, J = 7.8 Hz)

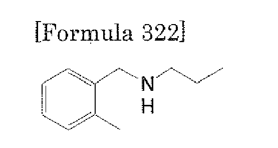

### Compound (I'-137):

Yield: 41%
1H NMR(CDCl3): δ(ppm) 0.94(3H, t, J = 7.5 Hz), 1.49-1.61(3H, m), 2.35(3H, s), 2.65(2H, t, J = 7.5 Hz), 3.77(2H, s), 7.15-7.20(2H, m), 7.26-7.31(2H, m)

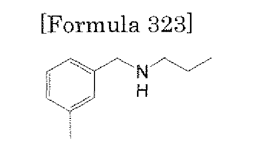

### Compound (I'-138):

Yield: 36%
1H NMR(CDCl3) : δ(ppm) 0.92(3H, t, J = 7.5 Hz), 1.48-1.60(3H, m), 2.35(3H, s), 2.60(2H, t, J = 7.2 Hz), 3.75(2H, s), 7.04-7.29(4H, m)

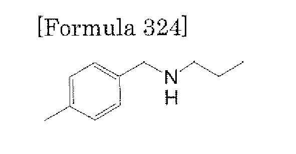

### Compound (I'-139):

Yield: 43%
1H NMR(CDCl3) : δ(ppm) 0.92(3H, t, J = 7.5 Hz), 1.47-L59(3H, m), 2.34(3H, s), 2.59(2H, t, J = 7.2 Hz), 3.75(2H, s), 7.13(2H, d, J = 8.1 Hz), 7.21.(2H, d, J = 7.8 Hz)

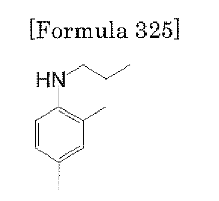

### Compound (I'-140):

Yield: 27%
1H NMR(CDCl3) : δ(ppm) 1.01(3H, t, J = 7.5 Hz), 1.67(2H, sextet, J = 7.2 Hz), 2.11(3H, s), 2.23(3H, s), 3.10(2H, t, J = 7.2 Hz), 3.34(1H, s(br), NH), 6.53(1.H, d, J = 8.1 Hz), 6.88(1H, s), 6,92(1H, d, J = 8.4 Hz)

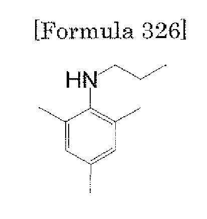

### Compound (I'-141):

Yield: 40%
1H NMR(CDCl3) : δ(ppm) 0.98(3H, t, J = 7.5 Hz), 1.53-1.66(3H, m), 2.22(3H, s), 2.52(6H, s), 2.89(2H, t, J = 7.5 Hz), 6.81(2H, s)

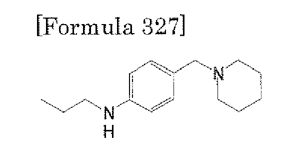

### Compound (I'-142):

Yield: 18%
1H NMR(CDCl3) : δ(ppm) 0.99(3H, t, J = 7.5 Hz), 1.35-1.69(9H, m), 2.30-2.40(4H, m), 3.07(2H, t, J = 7.2 Hz), 3.64(2H, s), 6.55(2H, d, J = 8.4 Hz), 7.09(2H, d, J = 8.4 Hz

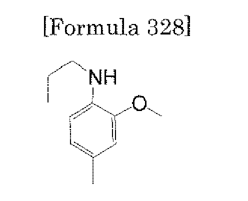

### Compound (I'-143):

Yield: 63%
1H NMR(CDCl3) : δ(ppm) 1.00(3H, t, J = 7.5 Hz), 1.66(2H, sextet, J = 7.5 Hz), 2.27(3H, s), 3.07(2H, t, J = 7.2 Hz), 3.83(3H, s), 4.03(1H, s(br), NH), 6.51(1H, d, J = 7.8 Hz), 6.60(1H, s), 6.66(1H, d, J = 7.5 Hz)

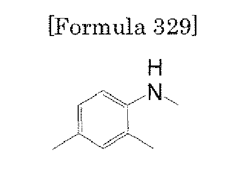

### Compound (I'-144):

Yield: 56%
1H NMR(CDCl3) : δ(ppm) 2.11(3H, s), 2.24(3H, s), 2.87(3H, s), 3.42(1H, s(br), NH), 6.53(1H, d, J= 8.1 Hz), 6.89(1H, s), 6.96(1H, d, J = 8.1 Hz)

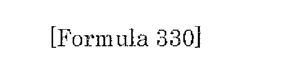

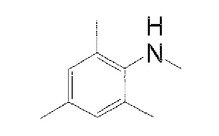

### Compound (I'-145):

Yield: 28%
1H NMR(CDCl3) : δ(ppm) 2.23(3H, s), 2.27(6H, s), 2.74(3H, s), 2.90(1H, s(br), NH), 6.83(2H, s)

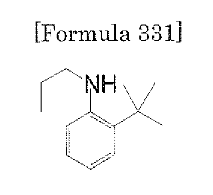

### Compound (I'-1.46):

Yield: 28%
1H NMR(CDCl3) : δ(ppm) 1.05(3H, t, J = 7.5 Hz), 1.42(9H, s), 1.72(2H, sextet, J = 7.5 Hz), 3.14(2H, q, J = 6.2 Hz), 3.85(1H, s(br). NH), 6.65-6.70(2H, m), 7.13(1H, t, J = 7.5 Hz), 7.23(1H, d, J = 7.8 Hz)

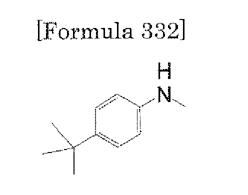

### Compound (I'-147):

Yield: 43%
1H NMR(CDCl3) : δ(ppm) 1.28(9H, s), 2.83(3H, s), 3.59(1H, s(br), NH), 6.58(2H, d, J = 8.4 Hz), 7.22(2H, d, J = 8.4 Hz)

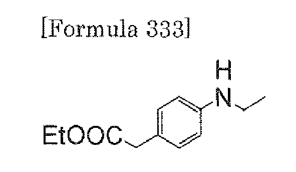

### Compound (I'-148):

Yield: 59%
1H NMR(CDCl3) : δ(ppm) 1.22-1.27(6H, m), 3.14(2H, q, J = 7.2 Hz), 3.49(2H, s), 3.52(1H, s(br), NH), 4.13(2H, q, J = 7.2 Hz), 6.56(2H, d, J = 8.4 Hz), 7.09(2H, d J = 8.4 Hz)

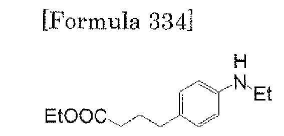

### Compound (I'-149):

Yield: 13%
1H NMR(CDCl3) : δ(ppm) 1.22-1.27(6H, m), 1.89(2H, quintet, J = 7.5 Hz), 2.30(2H, t, J = 7.5 Hz), 2.54(2H, t, J = 7.5 Hz), 3.13(2H, q, J = 7.2 Hz), 3.45(1H, s(br), NH), 4.11(2H, q, J = 7.2 Hz), 6.55(2H, d, J = 8.4 Hz), 6.98(2H, d J = 8.4 Hz)

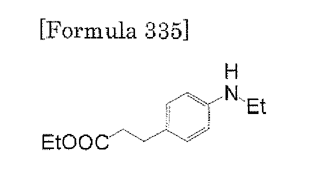

### Compound (I'-150):

Yield: 46%
1H NMH(CDCl3) : δ(ppm) 1.21-1.27(6H, m), 2.56(2H, t, J = 7.8 Hz), 2.84(2H, t, J = 7.5 Hz), 3.13(2H, q, J = 7.2 Hz), 3.45(1H, s(br), NH), 4.12(2H, q, J = 7.2 Hz), 6.54(2H, d, J = 8.4 Hz), 7.01(2H, d J = 8.4 Hz)

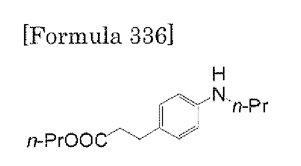

### Compound (I'-151):

Yield: 56%
1H NMR(CDCl3) : δ(ppm) 0.92(3H, t, J = 7.5 Hz), 0.99(3H, t, J = 7.5 Hz), 1.55-1.69(4H, m), 2.57(2H, t, J = 7.8 Hz), 2.84(2H, t, J = 7.2 Hz), 3.06(2H, t, J = 7.2 Hz), 3.54(1H, s(br), NH), 4.02(2H, t, J = 6.6 Hz), 6.54(2H, d, J = 8.4 Hz), 7.01(2H, d J = 8.4 Hz)

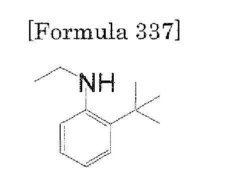

### Compound (I'-152):

Yield: 56%
1H NMR(CDCl3) : δ(ppm) 1.32.(3H, t, J = 7.2 Hz), 1.42(9H, s), 3.17-3.24(2H, m), 3.79(1H, s(br), NH), 6.66-6.71(2H, m), 7.13(1H, td, J = 7.7 Hz, J = 1.5 Hz), 7.23(1H, dd, J = 8.1 Hz, J = 1.5 Hz)

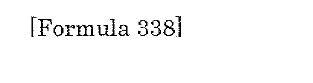

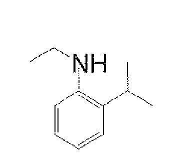

### Compound (I'-153):

Yield: 58%
1H NMR(CDCl3) : δ(ppm) 1.24-1.33(9H, m), 2.86(1H, septet, J = 6.9 Hz), 3.19(2H, q, J = 7.2 Hz), 3.55(1H, s(br), NH), 6.64(1H, d, J = 7.8 Hz), 6.72(1H, td, J = 7.5 Hz, J = 1.2 Hz), 7.08-7.16(2H, m)

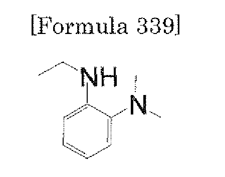

### Compound (I'-154):

Yield: 57%
1H NMR(CDCl3 : δ(ppm) 1.29(3H, t, J = 7.2 Hz), 2.64(6H, s), 3.19(2H, q, J = 7.2 Hz), 4.51(1H, br, NH), 6.60-6.69(2H, m), 6.97-7.03(2H, m)

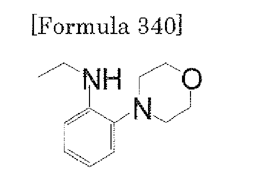

### Compound (I'-155):

Yield: 20%
1H NMR(CDCl3) : δ(ppm) 1.29(3H, t, J = 7.2 Hz), 2.89(4H, t, J = 4.5 Hz), 3.12-3.21(2H, m), 3.85(4H, t, J = 4.5 Hz), 4.58(1H, br, NH), 6.63(1H, d, J = 7.8 Hz), 6.68(1H, td, J = 7.5 Hz, J = 1.5 Hz), 6.95-7.07(2H, m)

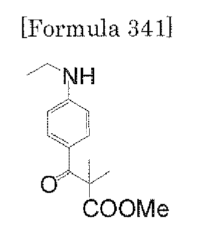

### Compound (I'-156):

Yield: 28%
1H NMR(CDCl3): δ(ppm) 1.28(3H, t, J = 7.2 Hz), 1.52(6H, s), 3.18-3.26(2H, m), 3.64(3H, s), 4.12(1H, br, NH), 6.50(2H, d, J = 9.0 Hz), 7.72(2H, d, J = 9.0 Hz)

With reference to WO2007/023882, the following compounds were synthesized.

### (Reference Example 20)

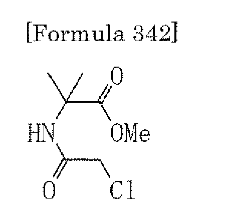

### Compound (I'-157):

To a solution of 15 g (0.1454 mole) of 2-aminoisobutyric acid in methanol (100 ml) was added dropwise 45 ml of thionyl chloride under ice-cooling, and the mixture was stirred at room temperature for 15 hours. The reaction solution was concentrated under reduced pressure to give a colorless crystal. To the crystal were added 60 ml of water and 29.7 g (0.3533 mole) of NaHCO₃, and a solution of 12.2 ml (0.1527 mole) of chloroacetyl chloride in toluene (38 ml) was added under ice-cooling, and the resulting mixture was stirred at room temperature for 18 hours. The reaction solution was separated, the organic layer was washed with brine, and dried over anhydrous sodium sulfate, and the solvent was concentrated under reduced pressure. The residue was washed with n-hexane to give 8.9 g (32%) of methyl 2-(N-chloroacetamide)-2-methylpropionate as a colorless crystal. 1H-NMR(CDCl₃): δ(ppm) 1.61 (6H, s), 3.77 (3H, s), 4.01 (2H, s), 7.17 (1H, brs).

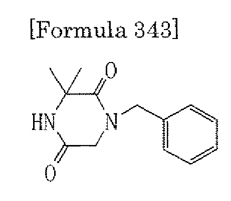

### Compound (I'-158):

A mixture of 8.9 g (0.04658 mole) of methyl 2-(N-chloroacetamide)-2-methylpropionate, 7.6 ml (0.06987 mole) of benzylamine, 9.7 ml (0.06987 mole) of triethylamine and 45 ml of methanol was heated to reflux with stirring for 10 hours. After the solvent of the reaction solution was concentrated under reduced pressure, and 45 ml of xylene was added to the residue, and the resulting mixture was heated to reflux with stirring for 20 hours. After cooled with the air, water was added, and the precipitated crystal was filtered to give 7.3 g (67%) of 1-benzyl-3,3-dimethylpiperazine-2,5-dione as a colorless crystal. 1H-NMR(CDCl3): δ(ppm) 1.53 (6H, s), 3.86 (2H, s), 4.61 (2H, s), 6.25 (1H, brs), 7.20-7.40 (5H, m).

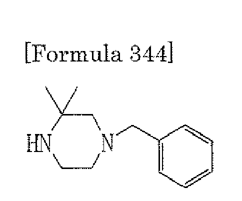

### Compound (I'-159):

To a suspension of 4.22 g (0.1111 mole) of aluminum lithium hydride in anhydrous tetrahydrofuran (70 ml) was added 7.29 g (0.03138 mole) of 1-benzyl-3,3-dimethylpiperazine-2,5-dione under ice-cooling, and the mixture was heated to reflux with stirring for 9 hours. Under ice-cooling, to the reaction solution were successively added dropwise a mixed solution of 8.5 ml of water and 17 ml of tetrahydrofuran, and 6.5 ml of a 2N-aciueous NaOH, and the mixture was stirred at room temperature for 2 hours. After aluminum hydroxide was filtered off, the solvent of the filtrate was concentrated under reduced pressure to give 5.8 g (90%) of 1-benzyl-3,3-dimethylpiperazine as a pale yellow liquid. 1H-NMR(CDCl3): δ(ppm) 1.13 (6H, s), 2.11 (2H, brs), 2.35 (2H, brs), 2.93 (2H, t, J=5Hz), 3.44 (2H, s)7.20-7.40 (5H, m).

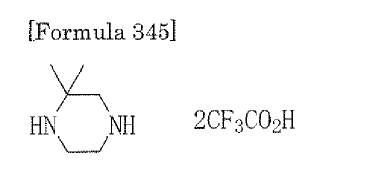

### Compound (I'-160):

A mixture of 5.25 g (0.02570 mole) of 1-benzyl-3,3-dimethylpiperazine, 0.5 g of palladium hydroxide, 25 ml of methanol, 7.8 ml (0.0771 mole) of trifluoroacetic acid and 7.8 ml (0.0771 mole) of cyclohexene was heated to reflux with stirring for 2 hours. The reaction solution was filtered, and the solvent was concentrated under reduced pressure. To the residue was added ethyl acetate, and the crystal was collected by filtration to give 6.9 g (78%) of 2,2-dimethylpiperazine-2 trifluoroacetate as a colorless crystal.
1H-NMR(DMSO-d6): δ(ppm) 1.39 (6H, s), 3.20 (2H, s), 3.21-3.30 (2H, m), 3.30-3.40 (2H, m), 9.41 (2H, s).

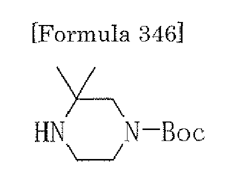

### Compound (I'-161):

To a mixture of 6.02 g (0.01760 mole) of 2,2-dimethylpiperazine-2 trifluoroacetate, 7.8 ml (0.0563 mole) of triethylamine and 30 ml of tetrahydrofuran was added dropwise 4.2 ml (0.01848 mole) of di-tsrt-butyl dicarbonate under ice-cooling, and the mixture was stirred at room temperature for 3 hours. The reaction solution was concentrated under reduced pressure, and 1N-aqueous HCl and diisopropyl ether were added, and extracted. The aqueous layer was alkalified with 2N-aqueous NaOH, and extracted with ethyl acetate. The organic layer was washed with brine, and dried over anhydrous sodium sulfate, and the solvent was concentrated under reduced pressure to give 2.6 g (69%) of 1-tert-butoxycarbonyl 3,3-dimethylpiperazine as a pale yellow liquid.
1H-NMR(CDCl3): δ(ppm) 1.11 (6H, s), 1.46 (9H, s), 2.87 (2H, t, J=5Hz), 3.16 (2H, s), 3.36 (2H, t, J=5Hz).

The following compounds were synthesized as in Reference Example 19.

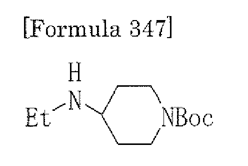

### Compound (I'-162):

Yield: 85%
1H-NMP(CDCl3): δ(ppm) 1.05 (3H, t, J=7Hz), 1.10-1.40 (2H, m), 1.45 (9H, s), 1.70-1.90 (2H, m), 2.57 (2H, q, J=7Hz), 2.60-2.80 (2H, m), 3.95-4.15 (3H, m).

The following compounds were synthesized as in Example 67. A diastereomer (form A) of the present compound shown below indicates a low polar compound, and a diastereomer (form B) indicates a high polar compound.

### [Example 157]

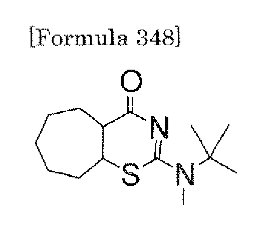

### Compound (I-157):

### Diastereomer (form A)

Yield: 7%
1H NMR(CDCl3) : δ(ppm) 1.40-1.90(18H, m), 2.21-2.29(1H, m), 2.32-2.41(1H, m), 3.09(3H, s), 3.17-3.25(1H, m)

### [Example 158]

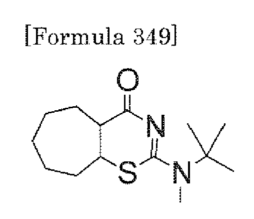

### Compound (I-158):

### Diastereomer (form B)

Yield: 34%
1H NMR(CDCl3): δ(ppm) 1.50-1.85(17H, m), 1.92-2.10(2H, m), 2.67-2.73(1H, m), 3.14(3H, s), 3.70.3.75(1H, m)

### [Example 159]

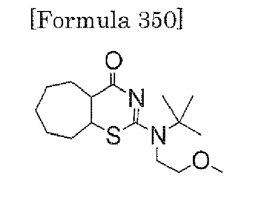

### Compound (I-159):

### Diastereomer (form A)

Yield: 7%
1H NMR(CDCl3): δ(ppm) 1.40-1.90(18H, m), 2.23-2.40(2H, m), 3.20-3.28(1H, m), 3.37(3H, s), 3.52-3.60(2H, m), 3.74(2H, t J = 6.9 Hz)

### [Example 160]

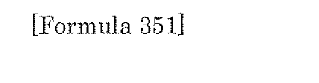

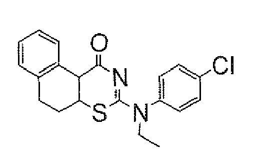

### Compound (I-160):

### Diastereomer (form A)

Yield: 16%
1H NMR(CDCl3) : δ(ppm) 1.20(3H, t, J = 7.2 Hz), 1.76-1.91(1H, m), 1.94-2.20(1H, m), 2.80-2.90(2H, m), 3.58(1H, td J = 12.5 Hz, J = 3.3 Hz), 3.74(1H, d, J = 12.6 Hz), 3.89-3.99(1H, m), 4.09-4.20(1H, m), 7.06(1H, d, J = 7.2 Hz), 7.16-7.28(4H, m), 744-7.48(3H, m)

### [Example 161]

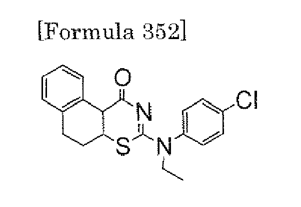

### Compound (I-161):

### Diastereomer (form B)

Yield: 80%
1H NMR(CDCl3) : δ(ppm) 1.10(3H, t, J = 7.2 Hz), 1.98-2.09(1H, m), 2.12-2.22(1H, m), 2.75-2.85(1H, m), 2.92-3.03(1H, m), 3.85-4.03(4H, m), 7.07-7.23(6H, m), 7.40(2H, d, J = 8.1 Hz)

### [Example 162]

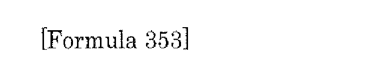

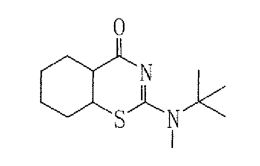

### Compound (I-162):

### Diastereomer (form A)

Yield: 5%
1H-NMR(CDCl3): δ(ppm) 1.23-1.54 (4H, m), 1.55 (9H, s), 1.74-2.08 (4H, m), 2.25-2.35 (1H, m), 3.09 (3H, s), 3.09-3.18 (1H, m).

### [Example 163]

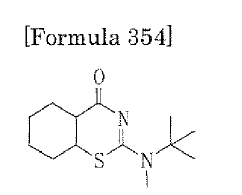

### Compound (I-163):

### Diastereomer (form B)

Yield: 19%
1H-NMR(CDCl3): δ(ppm) 1.33-1.98 (17H, m), 2.56-2.64 (1H, m), 3.15 (3H, s), 3.55-3.64 (1H, m).

### [Example 164]

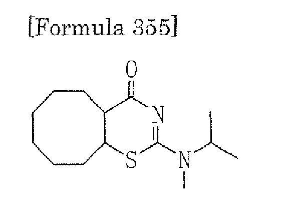

### Compound (I-164):

### Diastereomer (form A)

Yield: 36%
1H-NMR(CDCl3): δ(ppm) 1.06-1.34 (6H, m), 1.40-1.90 (10H, m), 2.02-2.16 (1H, m), 2.36-2.50 (1H, m), 2.60-2.74 (1H, m), 2.94, 3.05 (3H, s), 3.38-3.48 (1H, m), 4.20-4.35, 5.30-5.50 (1H, m).

### [Example 165]

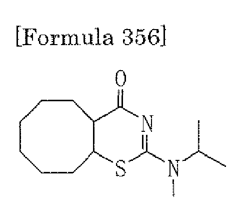

### Compound (I-165):

### Diastereomer (form B)

Yield: 35%
1H-NMR(CDCl3): δ(ppm) 1.05-1.30 (6H, m), 1.45-2.04 (12H, m), 2.85-3.10 (4H, m), 3.65-3.75 (1H, m), 4.26-4.44, 5.36-5.54 (1H, m).

### [Example 166]

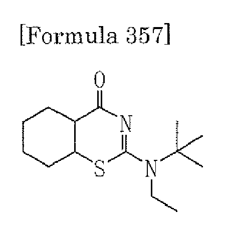

### Compound (I-166):

### Diastereomer (form B)

Yield: 11%
1H-NMR(CDCl3): δ(ppm) 1.29 (3H, t, J=7Hz), 1.30-2.00 (17H, m), 2.55-2.67 (1H, m), 3.55-3.75 (3H, m).

### [Example 167]

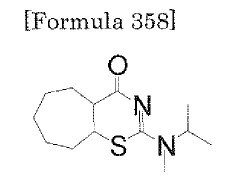

### Compound (I-167):

### Diastereomer (form A)

Yield: 11%
1H NMR(CDCl3) : δ(ppm) 1.10-1.28(6H, m), 1.42-1.88(9H, m), 2.30-2.43(2H, m), 2.92-3.04(3H, m), 3.27-3.35(1H, m), [4.20-4.32, 5.32-5.46(1H, m(rotamar))]

### [Example 168]

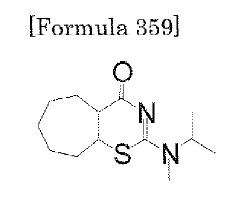

### Compound (I-168):

### Diastereomer (form B)

Yield: 75%
1H NMR(CDCl3) : δ(ppm) 1.10-1.30(6H, m), 1.54-1.90(8H, m), 2.01-2.11(2H, m), 2.73-2.78(1H, m), 2.98-3.07(3H, m), 3.74-3.79(1H, m), [4.32-4.45, 5.40-5.70(1H, m(rotamar))]

### [Example 169]

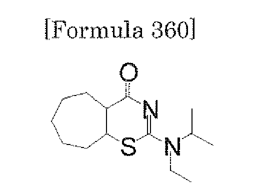

### Compound (I-169):

### Diastereomer (form A)

Yield: 12%
1H NMR(CDCl3) : δ(ppm) 1.14-2.10(18H, m), 2.32-2.44(2H, m), 3.28-3.83(3H, m), [4.38-4.30, 5.30-5.42(1H, m(rotamar))]

### [Example 170]

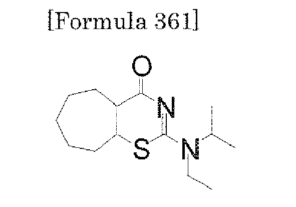

### Compound (I-170):

### Diastereomer (form B)

Yield: 74%
1H NMR(CDCl3) : δ(ppm) 1.16-1.30(9H, m), 1.50-1.90(8H, m), 1.98-2.13(2H, m), 2.73-2.78(1H, m), 3.42.3.70(2H, m), 3.75-3.80(1H, m), [4.32-4.44, 5.44-5.48(1H, m(rotamar))]

### [Example 171]

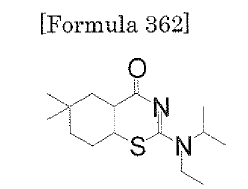

### Compound (I-171):

### Diastereomer (form A)

Yield: 11%
1H NMR(CDCl3) : δ(ppm) 0.97(3H, s), 1,01(3H, s), 1.15-1.88(14H, m), 2.02-2.11(1H, m), 2.27(1H, td, J = 12.6 Hz, J= 3.9 Hz), 3.02-3.13(1H, m), 3.40-3.81(2H, m), [4.21-4.35, 5.35-5.45(1H, m(rotamor))]

### [Example 172]

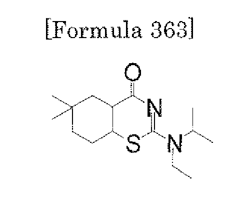

### Compound (I-172):

### Diastereomer (form B)

Yield: 80%
1H NMR(CDCl3) : δ(ppm) 0.96(3H, s), 0.98(3H, s), 1.16-1.69(14H, m), 1.96-2.09(1H, m), 2.80(1H, dt, J = 12.3, J = 4.2 Hz), 3.45-3.80(3H, m), [4.38-4.50, 5.40-5.52(1H, m(rotamar))

### [Example 173]

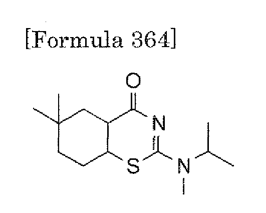

### Compound (I-173):

### Diastereomer (form A)

Yield: 26%
1H-NMR(CDCl3): δ(ppm) 0.95 (3H, s), 1.01 (3H, s), 1.14-1.87 (12H, m), 2.06 (1H, dt, J=13.0, 3.0Hz), 2.27 (1H, dt, J=12.0, 3.5Hz), 2.94, 3.05 (3H, m), 4.30, 5.44 (1H, br).

### [Example 174]

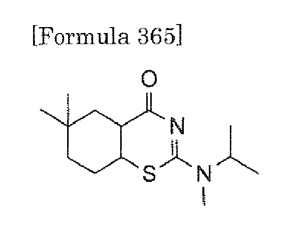

### Compound (I-174):

### Diastereomer (form B)

Yield: 56%
1H-NMR(CDCl3): δ(ppm) 0.96 (3H, s), 0.98 (3H, s), 1.12-1.76 (11H, m), 1.97-2.09 (1H, m), 2.80 (1H, dt, J=12.0, 3.5Hz), 3.01, 3.09 (3H, br), 3.78-3.81 (1H, m), 4.46, 5.52 (1H, br).

### [Example 175]

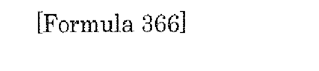

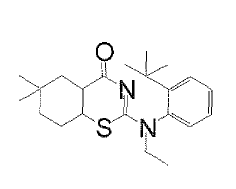

### Compound (I-175):

### Diastereomer (form A)

Yield: 16%
1H NMR(CDCl3) : δ(ppm) 0.95(3H, s), 1.00(3H, s), 1.13-1.68(17H, m), 2.05-2.36(2H, m), 2.94-3.10(2H, m), 4,65-4.81(1H, m), 7.01-7.06(1H. m), 7.22-7.28(1H, m), 7.39-7.45(1H, m), 7.59-7.62(1H, m)

### [Example 176]

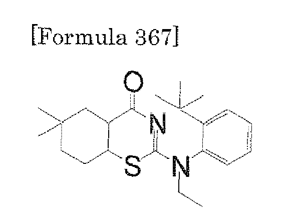

### Compound (I-176):

### Diastereomer (form B)

Yield: 73%
1H NMR(CDCl3) : δ(ppm) 0.91-0.96(6H, m), 1.12-1.67(17H, m), 1.86-1.98(1H, m), 2.76-2.82(1H, m), 2.98-3.12(1H, m), 3.69-3.78(1H, m), 4.67-4.86(1H, m), 7.03-7.07(1H, m), 7.24-7.30(1H, m), 7.40-7.47(1H, m), 7.62(1H, dd, J = 8.1 Hz, J = 1.5 Hz)

### [Example 177]

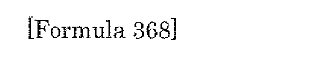

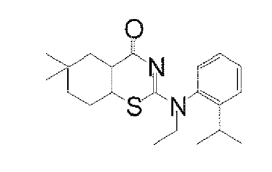

### Compound (I-177):

### Diastereomer (form A)

Yield: 17%
1H NMR(CDCl3) : δ(ppm) 0.93-1.00(6H, m), 1.13-1.65(14H, m), 2.02-2.10(1H, m), 2.20-2.36(1H, m), 2.90-3.04(2H, m), 3.16-3.37(1H, m), 4.48-4.74(1H, m), 7.10-7.14(1H, m), 7.33-7.29(1H, m), 7.42-7.49(2H, m)

### [Example 178]

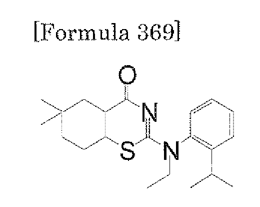

### Compound (I-178):

### Diastereomer (form B)

Yield: 83%
1H NMR(CDCl3 : δ(ppm) 0.91-0.95(6H, m), 1.10-1.68(14H, m), 1.86-1.96(1H, m), 276'2.96(2H, m), 3.24-3.35(1H, m), 3.68-3.78(1H, m), 4.56-4.72(1H, m), 7.13(1H, d, J = 8.8 Hz), 7.25-7.32(1H, m), 7.43-7.50(2H, m)

### [Example 179]

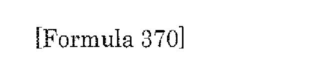

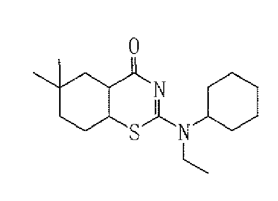

### Compound (I-179):

### Diastereomer (form A)

Yield: 8%
1H-NMR(CDCl3): δ(ppm) 0.85-1.95 (23H, m), 2.00-2.10 (1H, m), 2.26 (1H, td, J=12, 4Hz), 3.00-3.15 (1H, m), 3.35-3.50 (2H, m), 3.70-3.90, 4.90-5.10 (2H, m).

### [Example 180]

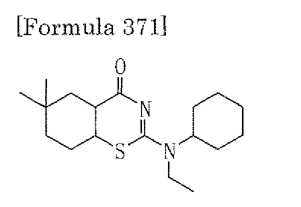

### Compound (I-180):

### Diastereomer (form B)

Yield: 57%
1H-NMR(CDCl3): δ(ppm) 0.96 (3H, s), 0.97 (3H, s), 1.10-2.10 (18H, m), 2.80 (1H, dt, J=12.5, 4Hz), 3.45-3.60 (2H, m), 3.65-3.83 (2H, m), 3.90-4.02, 5.00-5.18 (1H, m).

### [Example 181]

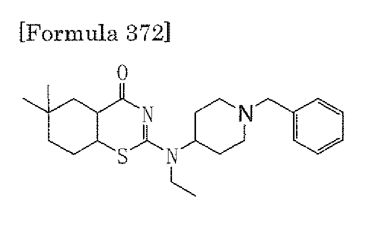

### Compound (I-181):

### Diastereomer (form A)

Yield: 19%
1H-NMR(CDCl3): δ(pom) 0.83-1.05 (9H, m), 1.15-2.35 (16H, m), 2.83-3.15 (3H, m), 3.20-3.35 (1H, m), 3.50 (2H, s), 3.65-3.85, 4.95-5.10 (1H, m), 7.20-7.35 (5H, m).

### [Example 182]

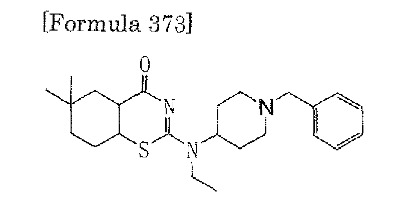

### Compound (I-182):

### Diastereomer (form B)

Yield: 61%
1H-NMR(CDCl3): δ(ppm) 0.85-1.00 (9H, m), 1.15-1.85 (14H, m), 1.95-2.20 (2H, m), 2.75-3.10 (2H, m), 3.30-3.55 (3H, m), 3.75-3.85 (1H, m), 3.95-4.05, 5.00-5.20 (1H, m), 7.30 (5H, brs).

### [Example 183]

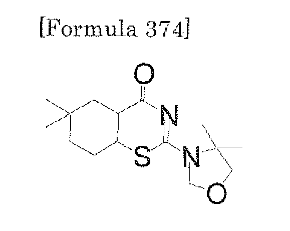

### Compound (I-183):

### Diastereomer (form A)

Yield 17%
1H NMR(CDCl3): δ(ppm) 0.97(3H, s), 1.01(3H, s), 1.20-1.90(1H, m), 2.03-2.11(1H, m), 2.28-2.38(1H, m), 3.14(1H, td, J = 12.3 Hz, J = 4.5 Hz), 3.79-3.87(2H, m), 4.92-5.34(2H, m(br))

### [Example 184]

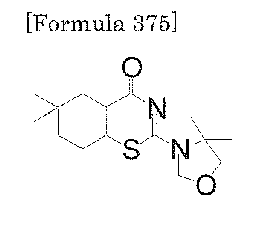

### Compound (I-184):

### Diastereomer (form B)

Yield: 83%
1H NMR(CDCl3): δ(ppm) 0.97(3H, s), 0.98(3H, s), 1.19-1.30(1H, m), 1.41-1.64(10H, m), 1.97-2.08(1H, m), 2.80(1H, dt, J = 12.1 Hz, J = 3.9 Hz), 3.80-3.90(3H, m), 5.02-5.34(2H, m(br))

### [Example 185]

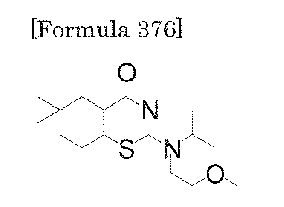

### Compound (I-185):

### Diastereomer (form A)

Yield: 16%
1H NMR(CDCl3) : δ(ppm) 0.97(3H, s), 1.02(3H, s), 1.15-1.36(9H, m), 1.45-1.53(1H, m), 1.65-1.88(3H, m), 2.00-2.10(1H, m), 2.27(1H, td, J = 12.6 Hz, J = 3.9 Hz), 3.00-3.12(1H, m), 3.33-3.83(5H, m), [4.20-4.35, 5.25-5.40(1H m(rotamar))

### [Example 186]

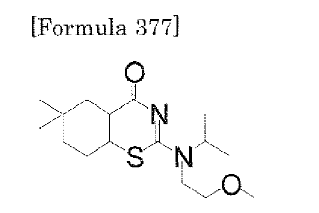

### Compound (I-186):

### Diastereomer (form B)

Yield: 83%
1H NMR(CDCl3): δ(ppm) 0.96(3H, s), 0.98(3H, s), 1.18-1.70(11H, m.), 1.97-2.13(1H, m), 2.80(1H, dt, J = 12.9 Hz, J = 3.9 Hz), 3.32-3.40(3H, m), 3.61-3.80(5H, m), [4.40-4.50, 5.30-5.50(1H, m(rotamar))]

The following compounds were synthesized as in Example 132.

### [Example 187]

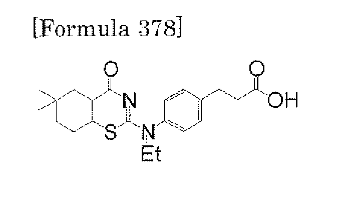

### Compound (1-187):

### Diastereomer (form A)

Yield: 80%
1H NMR(CDCl3): δ(ppm) 0.93(3H, s), 0.99(3H, s), 1.14-1.70(9H, m), 2.05(1H, dt, J= 13.5 Hz, J = 2.7 Hz), 2.29(1H, td, J = 14.5 Hz, J = 1.6 Hz), 2.73(2H, t, J = 7.8 Hz), 2.94-3.06(3H, m), 3.85-4.14(2H, m), 7. 17(2H, d, J = 8.1 Hz), 7.31(2H, d J = 8.4 Hz)

### [Example 188]

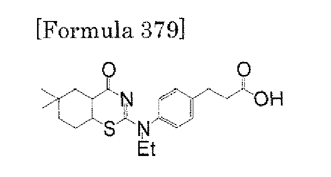

### Compound (I-188):

### Diastereomer (form B)

Yield: 100%
1H NMR(CDCl3) : δ(ppm) 0.94-0.96(6H, m), 1.17(3H, t, J = 7.2 Hz), 1.40-2.00(7H, m), 2.72-2.83(3H, m), 3.04(2H, t, J = 7.8 Hz), 3.68-3.74(1H, m), 3.97-4.10(2H, m), 7.18(2H, d, J = 8.4 Hz), 7.32(2H, d J = 8.1 Hz)

The following compounds were synthesized as in Example 67.

### [Example 189]

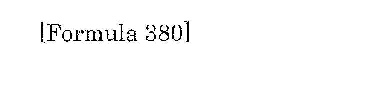

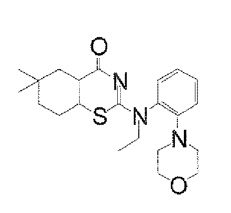

### Compound (I-189):

### Diastereomer (form A)

Yield: 66%
1H NMR(CDCl3) : δ(ppm) 0.92-1.68(13H, m), 2.03-2.19(1H, m), 2.35(1H, td, J = 2.5 Hz, J = 3.9 Hz), 2.80-3.19(6H, m), 3.36-3.55(1H, m), 3.72-3.86(4H, m), 4.60-4.82(1H, m), 7.07-7.20(3H, m), 7.37-7.44(1H, m)

### [Example 190]

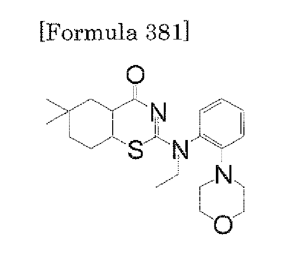

### Compound (I-190):

### Diastereomer (form B)

Yield: 13%
1H NMR(CDCl3) : δ(ppm) 0.86-1.69(14H, m), 1.88-1.99(1H, m), 2.79-2.90(3H, m), 3.11-3.24(2H, m), 3.39-3.52(1H, m), 3.67-3.87(5H, m), 4.64-4.86(1H, m), 7.09-7.20(3H, m), 7.40-7.45(1H, m)

### [Example 191]

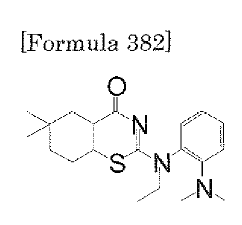

### Compound (I-191):

### Diastereomer (form A)

Yield: 59%
1H NMR(CDCl3) : δ(ppm) 0.88-1.73(13H, m), 2.01-2.23(1H, m), 2.36(1H, td, J = 12.0 Hz, J = 3.6 Hz), 2.80(3H, s), 2.82(3H, s), 2.94-3.10(1H, m), [3.21-3.42, 4.52-4.77(3H, m), rotamar] 6.88-6.95(1H, m), 6.98-7.03(1H, m), 7.08-7.13(1H, m), 7.30.7.37(1H, m)

### [Example 192]

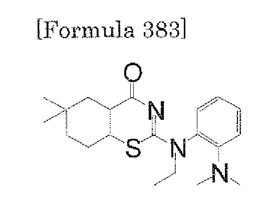

### Compound (I-192);

### Diastereomer (form B)

Yield: 35%
1H NMR(CDCl3) : δ(ppm) 0.85-1.71(14H, m), 1.83-2.05(1H, m), 2.17-2.25(1H, m), 2.80(3H, s), 2.84(3H, s), 3.20-3.38(1H, m), 3.68-3.80(1H, m), 4.56-4.90(1H, m), 6.90-7.14(3H, m), 7.25-7.38(1H, m)

### [Example 193]

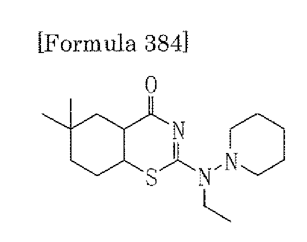

### Compound (I-193):

### Diastereomer (form A)

Yield: 62%
1H-NMR(CDCl₃): δ(ppm) 0.95 (3H, s), 1.00 (3H, s), 1.05-1.85 (14H, m), 1.95-2.05 (1H, m), 2.24 (1H, td, J=12, 4Hz), 2.65-2.95 (5H, m), 3.52 (1H, quint, J=7Hz), 3.76 (1H, quint, J=7Hz).

### [Example 194]

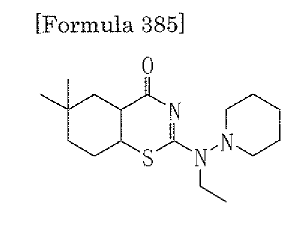

### Compound (I-194):

### Diastereomer (form B)

Yield: 8%
1H-NMR(CDCl3): δ(ppm) 0.97 (3H, s), 0.98 (3H, s), 1.05-1.80 (14H, m), 1.90-2.05 (1H, m), 2.65-2.83 (3H, m), 2.85-2.95 (2H, m), 3.53-3.80 (3H, m).

### [Example 195]

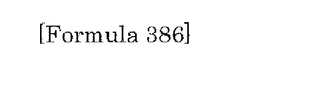

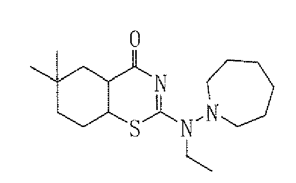

### Compound (I-195):

### Diastereomer (form A)

Yield 2%
1H-NTMR(CDCl3): δ(ppm) 0.96 (3H, s), 1.00 (3H, s), 1.19-2.06 (17H, m), 2.23 (1H, td, J=12.5, 4Hz), 2.83 (1H, td, J=12.5, 4.5Hz), 2.94-3.10 (4H, m), 3.54 (1H, quint, J=7Hz), 3.79 (1H, quint, J=7Hz).

### [Example 196]

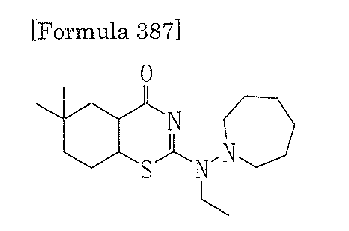

### Compound (I-196):

### Diastereomer (form B)

Yield: 5%
1H-NMR(CDCl3): δ(ppm) 0.97 (6H, s), 1.15-1.25 (1H, m), 1.31 (3H, t, J=7Hz), 1.35-2.05 (13H, m), 2.76 (1H, td, J=7, 4Hz), 2.95-3.10 (4H, m), 3.55-3.85 (3H, m).

### [Example 1.97]

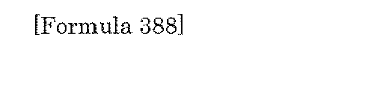

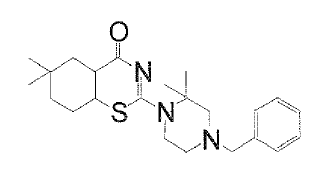

### Compound (I-197):

### Diastereomer (form B)

Yield: 17%
1H NMR(CDCl3) : δ(ppm) 0.95-0.97(6H, m), 1.17-1.66(11H, m), 1.92-2.06(1H, m), 2.17-2.38(2H, m), 2.41-2.50(1H, m), 2.66-2.81(2H, m), 3.45-3.52(2H, m), 3.57-3.65(1H, m), 3.70-3.74(1H, m), 3.80-3.87(1H, m), 7.26-7.34(5H, m)

### [Example 198]

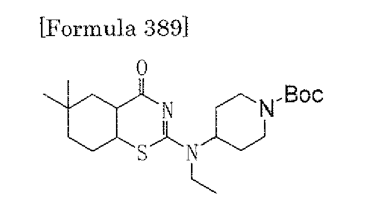

### Compound (I-198):

### Diastereomer (form B)

Yield: 44%
1H-NMR(CDCl3): δ(ppm) 0.96 (3H, s), 0.97 (3H, s), 1.5-2.10 (18H, m), 2.50-2.86 (7H, m), 3.40-3.60 (1H, m), 3.72-3.80 (1H, m), 3.95-4.45, 5.10-5.50 (4H, m).

### [Example 199]

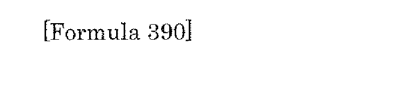

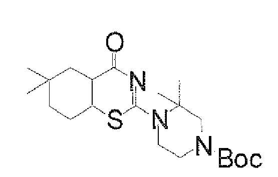

### Compound (I-199);

### Diastereomer (form A)

Yield: 23%
1H NMR(CDCl3) : δ(ppm) 0.96(3H, s), 1.01(3H, s), 1.20-1.84(20H, m), 1.99-2.06(1H, m), 2,22(1H, td, J = 12.6 Hz, J = 3.9 Hz), 3.03(1H, td, J = 12.3 Hz, J = 4.2 Hz), 3.45-3.58(4H, m), 3.74-3.94(2H, m)

### [Example 200]

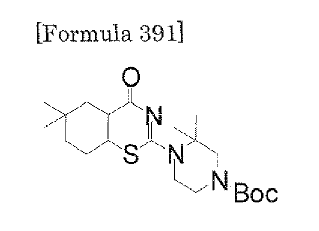

### Compound (1-200):

### Diastereomer (form B)

Yield: 30%
1H NMR(CDCl3) : δ(ppm) 0.95-0.98(6H, m), 1.20-1.68(20H, m), 1.96-2.08(1H, m), 2.77(1H, dt, J = 12.3 Hz, J = 4.2 Hz), 3.45-3.61(4H, m), 3.77-4.02(3H, m)

### [Example 201]

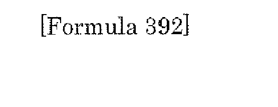

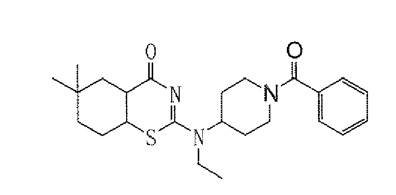

### Compound (I-201):

### Diastereomer (form B)

A mixture of 0.19 g (0.4485 mmole) of the compound (I-198), 1 ml of concentrated hydrochloric acid and 2 ml of acetic acid was stirred at room temperature for 18 hours. After the reaction solution was concentrated under reduced pressure, 2 ml of tetrahydrofuran and 0.19 ml (1.345 mmole) of triethylamine were added to the residue, 69 mg (0.4934 mmole) of benzoyl chloride was added under ice-cooling, and the mixture was stirred at room temperature for 24 hours. To the reaction solution were added water and ethyl acetate, this was extracted, and the organic layer was washed with brine. The organic layer was dried over anhydrous sodium sulfate, and filtered, the solvent was concentrated under reduced pressure, and the residue was purified by silicagel columnchromatography (only ethyl acetate) to give 0.06 g (29%) of a title compound as a colorless amorphous substance.
1H-NMR(CDCl3): δ(ppm) 0.98 (6H, s), 1.15-2.10 (14H, m), 2.76-2.86, 3.05-3.25 (3H, m), 3.40-3.60 (2H, m), 3.82 (2H, s), 4.75-4.90, 5.10-5.50 (1H, m), 7.42 (5H, s).

The following compounds were synthesized as in Example 201.

### [Example 202]

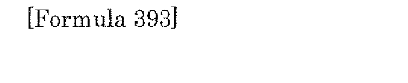

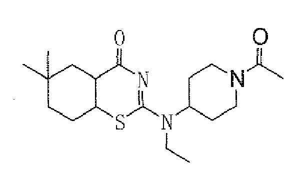

### Compound (I-202):

### Diastereomer (form B)

Yield: 37%
1H-NMR(CDCl3): δ(ppm) 0.97 (3H, s), 0.99 (3H, s), 1.35 (13H, m), 2.11 (3H, s), 2.54-2.70 (1H, m), 2.82 (1H, dt, J=J.2.5, 4Hz), 3.10-3.30 (1H, m), 3.40-3.55 (3H, m), 3.78-3.90 (1H, m), 4.68-4.80 (1H, m), 5.26-5.42 (1H, m).

### [Example 203]

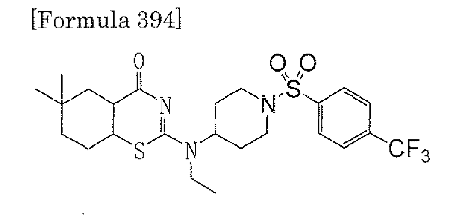

### Compound (I-203):

### Diastereomer (form B)

Yield: 18%
1H-NMR(CDCl3): δ(ppm) 0.95 (6H, s), 1.15-2.10 (1.6H, m), 2.31-2.50 (1H, m), 2.70-2.80 (1H, m), 3.43-3.50 (1H, m), 3.75-3.77, 4.95-5. 10 (1H, m), 3.81-3.93 (2H, m), 7.80-8.00 (4H, m).

### [Example 204]

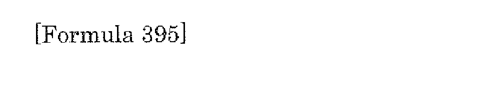

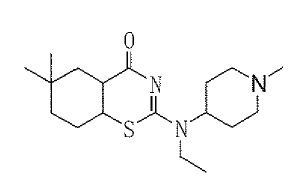

### Compound (I-204):

### Diastereomer (form B)

Yield: 12%
1H-NMR(CDCl3): δ(ppm) 0.95 (3H, s), 0.98 (3H, s), 1.14-1.87 (11H, m), 1.88-2.25 (3H, m), 2.28, 2.31 (3H, s), 2.75-3.02 (3H, m), 3.42-3.62 (3H, m), 3.65-3.82 (1H, m), 3.89-4.05, 5.00-5.19 (1H, m).

### [Example 205]

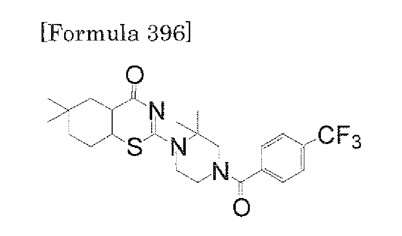

### Compound (I-205):

### Diastereomer (form A)

Yield: 84%
1H NMR(CDCl3) : δ(ppm) 0.97(3H, s), 1.02(3H, s), 1.20-1.36(2H, m), 1.45-1.85(10H, m), 1.98-2.06(1H, m), 2.24(1H, td, J = 12.6 Hz, J = 3.9 Hz), 2.97-3.14(1H, m), 3.37-4.20(1H, m), 7.53(2H, d, J = 8.1 Hz), 7.72(2H, d, J = 8.1 Hz)

### [Example 206]

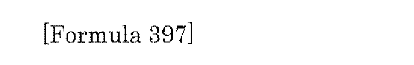

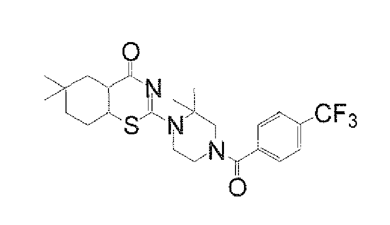

### Compound (I-206):

### Diastereomer (form B)

Yield: 47%
1H NMR(CDCl3): δ(ppm) 0.95-0.98(6H, m), 123-2.22(13H, m), 2.79(1H, dt, J = 12.6 Hz, J = 4.2 Hz), 3.37-4.16(6H, m), 7.54(2H, d, J = 8.1 Hz), 7.72(2H, d, J = 8.1 Hz)

### [Example 207]

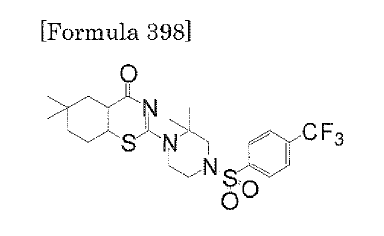

### Compound (I-207):

### Diastereomer (form A)

Yield: 74%
1H NMR(CDCl3) : δ(ppm) 0.94(3H, s), 1.00(3H, s), 1.18-1.82(11H, m), 1.97(1H, dt, J = 14.1 Hz, J = 3.3 Hz), 2.19(1H, td, J = 12.6 Hz, J = 3.9 Hz), 2.96-3.06(2H, m), 3.21-3.29(2H, m), 3,44-3.51(1H, m), 3.68-3.84(2H, m), 7.83(2H, d, J = 8.1 Hz), 7.92(2H, d, J = 8.4 Hz)

### [Example 208]

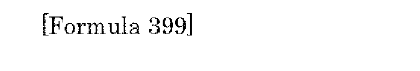

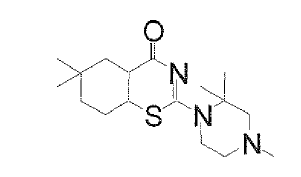

### Compound (1-208):

### Diastereomer (form A)

Yield: 54%
1H NMR(CDCl3) : δ(ppm) 0.96(3H, s), 1.01(3H, s), 1.20-1.83(10H, m), 1.94-2.36(9H, m), 2.60-2.69(1H, m), 2.97(1H, td, J = 12.3 Hz, J = 4.2 Hz), 3.47-3.56(1H, m), 3.70-3.77(1H, m)

### [Example 209]

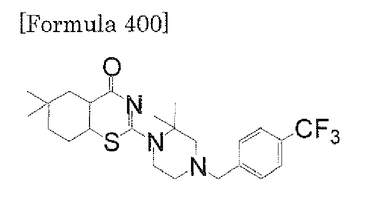

### Compound (I-209):

### Diastereomer (form A)

Yield: 64%
1H NMR(CDCl3) : δ(ppm) 0.95(3H, s), 1.01(3H, s), 1.19-1.84(11H, m), 1.96(1H, dt J = 14.4 Hz, J = 3.3 Hz), 2.14-2.26(2H, m), 2.33-2.44(2H, m), 2.67-2.75(1H, m), 2.98(1H, td, J = 12.3 Hz, J = 4.5 Hz), 3.49-3.60(3H, m), 3.76(1H, dt, J = 13.2 Hz, J = 4.4 Hz), 7.46(2H, d, J = 8.1 Hz), 7.58(2H, d, J = 8.1 Hz)

The following compounds were synthesized as in Example 67.

### [Example 210]

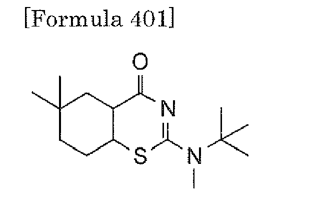

### Compound (I-210):

### Diastereomer (form B)

Yield: 10%
1H-NMR(CDCl3): δ(ppm) 0.96 (3H, s), 0.97 (3H, s), 1.17-1.25 (1H, m), 1.35-1.41 (1H, m), 1.45-1.68 (3H, m), 1.56 (9H, s), 1.94-2.07 (1H, m), 2.73-2.80 (1H, m), 3.21 (3H, s), 3.72-3.74 (1H, m).

### [Example 211]

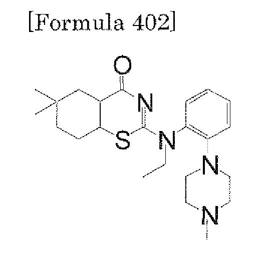

### Compound (I-211):

### Diastereomer (form B)

Yield: 38%
1H NMR(CDCl3) : δ(ppm) 0.87-1.00(6H, m), 1.09-1.20(3H, m), 1.24-1.69(3H, m), 1.88-2.41(6H, m), 2.50-2.70(4H, m), 2.77-2.86(1H, m), 2.90-2.99(2H, m), 3.20-3.50(3H, m), 3.65-3.82(1H, m), 4.64-4.85(1H, m), 7.07-7.18(3H, m), 7.38-7.44(1H, m)

### [Example 212]

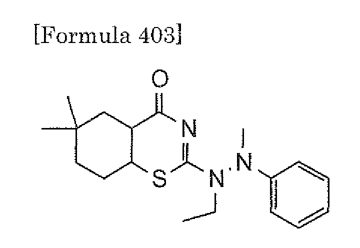

### Compound (I-212):

### Diastereomer (form A)

Yield: 10%
1H-NMR(CDCl3): δ(ppm) 0.96-1.02 (6H, m), 1.22-1.48 (5H, m), 1.66-1.72 (3H, m), 2.01-2.07 (1H, m), 2.28-2.36 (1H, m), 2.95-3.10 (1H, m), 3.19, 3.22 (3H, s), 3.28-3.59 (1H, m), 3.94-4.26 (1H, m), 6.75-6.80 (2H, m), 6.94-6.99 (1H, m), 7.28-7.35 (2H, m).

### [Example 213]

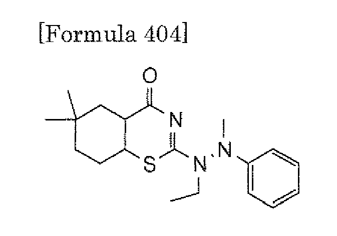

### Compound (I-213):

### Diastereomer (form B)

Yield: 88%
1H-NMR(CDCl3): δ(ppm) 0.94-0.99 (6H, m), 1.23-1.29 (4H, m), 1.49-1.65 (4H, m), 1.90-2.10 (1H, m), 2.80-2.85 (1H, m), 3.22, 3.25 (3H, s), 3.37-3.53 (1H, m), 3.70-3.75 (1H, m), 4.04-4.22 (1H, m), 6.74-6.87 (2H, m), 6.95-7.00 (1H, m), 7.29-7.36 (2H, m)

### [Example 214]

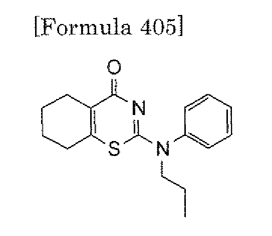

### Compound (I-214):

A mixture of 27 mg (0.089 mmole) of the compound (I-67), 22 mg (0.098 mmole) of 2,3-dichloro-5,6-dicyano-1,4-benzoquinone and 1 ml of 1,4-dioxane was heated to reflux for 2 hours. The insoluble was removed, and the filtrate was concentrated under reduced pressure. The residue was purified by silicagel columnchromatography (n-hexane : ethyl acetate = 1: 2) to give 8 mg (31%) of a title compound as a colorless crystal.
1H-NMR(CDCl3): δ(ppm) 0.91 (3H, t, J=7.5Hz), 1.56-1.78 (6H, m), 2.22-2.26 (2H, m), 2.51-2.56 (2H, m), 3.99 (2H, t, J=7.5Hz), 7.23-7.27 (2H, m), 7.46-7.54 (3H, m).

The following compounds were synthesized as in Example 214.

### [Example 215]

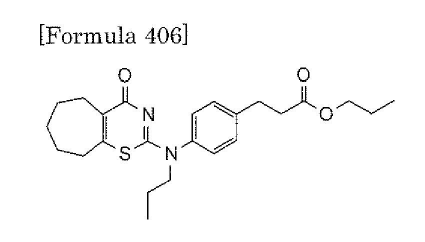

### Compound (I-215):

Yield: 54%
1H-NMR(CDCl3): δ(ppm) 0.90 (3H, t, J=7.5Hz), 0.92 (3H, t, J=7.5Hz), 1.50-1.70 (8H, m), 1.74-1.79 (2H, m), 2.37 (2H, t, J=5.5Hz), 2.69 (2H, t, J=7.5Hz), 2.89 (2H, t, J=5.5Hz), 3.03 (2H, t, J=7.5Hz), 3.96 (2H, t, J=7.5Hz), 4.05 (2H, t, J=7.5Hz), 7.17 (2H, d, J=8.5Hz), 7.33 (2H, d, J=8.5Hz).

### [Example 216]

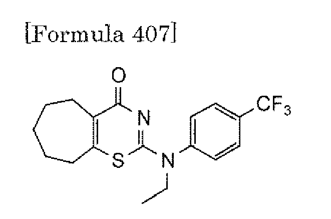

### Compound (1-216):

Yield 61%
1H-NMR(CDCl3): δ(ppm) 1.19 (3H, t, J=7.0Hz), 1.45-1.61 (4H, m), 1.70-1.85 (2H, m), 2.40 (2H, t, J=5.5Hz), 2.89 (2H, t, J=5.5Hz), 4.06 (2H, q, J=7.0Hz), 7.11 (2H, d, J=8.5Hz), 7.49 (2H, d, J=8.5Hz).

### [Example 217]

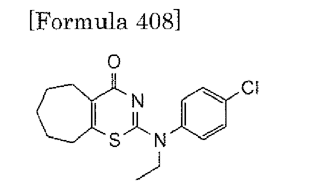

### Compound (I-217):

Yield: 92%
1H-NMR(CDCl3): δ(pom) 1.22 (3H, t, J=7.5Hz), 1.49-1.60 (4H, m), 1.76-1.84 (2H, m), 2.41 (2H, t, J=5.5Hz), 2.90 (2H, t, J=5.5Hz), 4.11 (2H, q, J=7.5Hz), 7.43 (2H, d, J=8.5Hz), 7.79 (2H, d, J=8.5Hz)

### [Example 218]

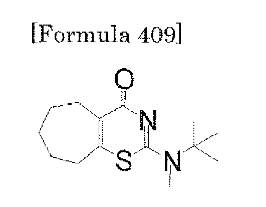

### Compound (I-218):

Yield: 58%
1H NMR(CDCl3) : δ(ppm) 1.48-1.67(13H, m), 1.79-1.86(2H, m), 2.51-2.54(2H, m), 2.88-2.91(2H, m), 3.07(3H, s)

The following compounds were synthesized as in Example 65.

### [Example 219]

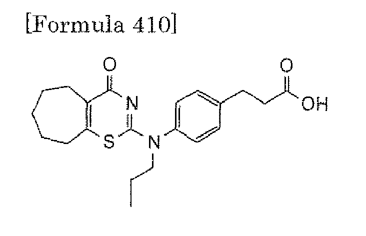

### Compound (I-219):

Yield: 55%
1H-NMR(CDCl3): δ(ppm) 0.90 (3H, t, J=7.5Hz), 1.48-1.68 (6H, m), 1.75-1.84 (2H, m), 2.40 (2H, t, J=5.0Hz), 2.75 (2H, t, J=7.5Hz), 2.89 (2H, t, J=5.0Hz), 3.05 (2H, t, J=5.0Hz), 3.94 (2H, t, J=7.5Hz), 7.17 (2H, d, J=8.0Hz), 7.36 (2H, d, J=8.0Hz), 9.14 (1H, br).

### [Example 220]

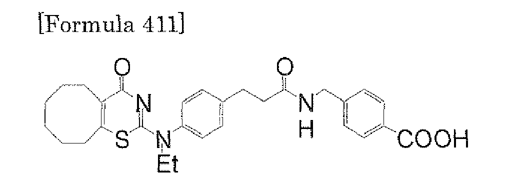

### Compound (1-220):

### Diastereomer (form B)

Yield: 75%
1H NMR(DMSO) : δ(ppm) 1.09(3H, t, J = 6.9 Hz), 1.30-1.60(7H, m), 2.43-2.73(5H, m), 2.94(2H, t, J = 7.5 Hz), 3.30-3.33(2H, m), 3.90(2H, q, J = 7.2 Hz), 4.33(2H, d, J = 6.0 Hz), 7.27-7.41(6H, m), 7.85(2H, d, J = 8.4 Hz), 8.45-8.50(1H, m), 12.9(1H, s(br), OH)

The following compounds were synthesized as in Example 67.

### [Example 221]

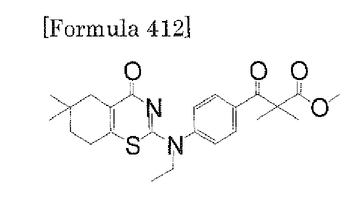

### Compound (I-221):

Yield: 42%
1H NMR(CDCl3) : δ(ppm) 0.96(6H, s), 1.19-1.29(5H, m), 1.44-1.58(8H, m), 2.25-2.32(1H, m), 2.35-2.37(1H, m), 3.70(3H, s), 4.06-4.16(2H, m), 7.34(2H, d, J = 9.0 Hz), 7.93(2H, d J = 8.7 Hz)

The following compounds were synthesized as in Example 65.

### [Example 222]

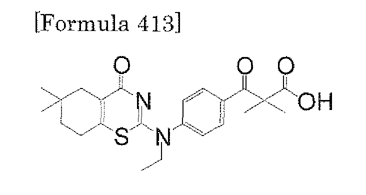

### Compound (I-222):

Yield: 65%
1H NMR(CDCl3) : δ(ppm) 0.97(6H, s), 1.20-1.29(10H, m), 1.48(2H, t, J = 6.3 Hz), 2.29-2.38(4H, m), 4.06-4.16(2H, m), 7.38(2H, d, J = 8.4 Hz), 8.23(2H, d J = 8.4 Hz)

The following compounds were synthesized as in Example 214.

### [Example 223]

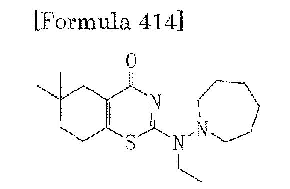

### Compound (I-223):

Yield: 20%
1H-NMR(CDCl3): δ(ppm) 0.97 (6H, s), 1.33 (3H, t, J=7Hz), 1.45-2.00 (10H, m), 2.36 (2H, s), 2.44 (2H, t, J=6.5Hz), 2.90-3.10 (4H, m), 3.70 (2H, q, J=7Hz).

### [Example 224]

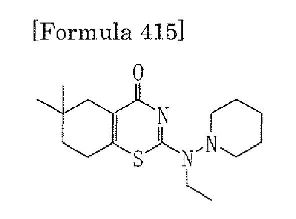

### Compound (I-224)^{:}

Yield: 19%
1H-NMR(CDCl3): δ(ppm) 0.97 (6H, s), 1.30 (3H, t, J=7Hz), 1.50 (2H, t, J=6.5Hz), 1.60-1.85 (6H, m), 2.36 (2H, s), 2.45 (2H, t, J=6.5Hz), 2.77 (2H, td, J=9, 4Hz), 2.83-2.94 (2H, m), 3.69 (2H, q, J=7Hz).

The following compounds were synthesized as in Example 66.

### [Example 225]

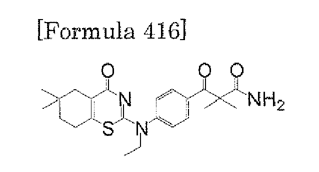

### Compound (I-225):

Yield: 48%
1H NMR(CDCl3) : δ(ppm) 0.96(6H, s), 1.22(3H, t, J = 7.2 Hz), 1.46(2H, t, J = 6.6 Hz), 1.59(6H, s), 2.26-2.36(4H, m), 4.10(2H, q, J = 6.9 Hz), 5.60-6.30(2H, br, NH2), 7.37(2H, d, J = 9.3 Hz), 7.96(2H. d J = 9.6 Hz)

### [Example 226]

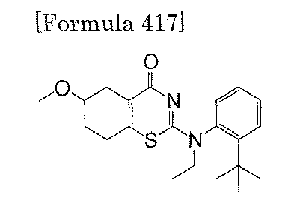

### Compound (I-226):

A mixture of 92 mg (0.35 mmole) of the compound (I'-93), 61 mg (0.35 mmole) of the compound (I'-152) and 1 ml of 1,4-dioxane was heated to reflux with stirring for 2 hours. The reaction solution was concentrated under reduced pressure, and the residue was purified by silicagel columnchromatography (n-hexane : ethyl acetate = 1 : 2). To the product were added 56 mg of 2,3-dichloro-5,0-dicyano-1,4-benzoquinone and 2 ml of 1,4-dioxane, and the mixture was heated to reflux with stirring for 3 hours. The reaction solution was concentrated under reduced pressure, and the residue was purified by silicagel columnchromatography (n-hexane : ethyl acetate = 1 : 2) to give 6 mg (7%) of a diastereomer (form A) as a colorless crystal, and 40 mg (49%) of a diastereomer (form B) as a colorless crystal.
Diastereomer (form A): 1H-NMR(CDCl3): δ(ppm) 1.23, 1.26 (3H, t, J=7.0Hz), 1.38, 1.39 (9H, s), 1.48-1.98 (3H, m), 2.38-2.68 (2H, m), 2.98-3.16 (2H, m), 3.31, 3.32 (3H, s), 3.64-3.67 (1H, m), 4.60-4.75 (1H, m), 7.02 (1H, ddd, J=7.5, 3.5, 1.5Hz), 7.21-7.27 (1H, m), 7.38-7.45 (1H, m), 7.60 (1H, dd, J=7.5, 1.5Hz)
Diastereomer (form By 1H-NMR(CDCl3): δ(ppm) 1.22, 1.26 (3H, t, J=7.0Hz), 1.39, 1.42 (9H, s), 1.48-2.05 (4H, m), 2.56-2.66 (1H, m), 2.86-3.02 (1H, m), 3.30, 3.31 (3H, s), 3.66-3.68 (1H m), 4.05-4.12 (2H, m), 7.02 (1H, ddd, J=7.5, 3.5, 1.5Hz), 7.20-7.27 (1H, m), 7.32-7.44 (1H, m), 7.59 (1H, dd, J=7.5, 1.5Hz)

The following compounds were synthesized as in Example 67.

### [Example 227]

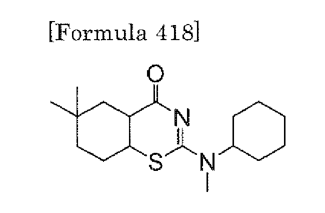

### Compound (I-227):

### Diastereomer (form A)

Yield: 59%
1H NMR(CDCl3) : δ(ppm) 0.96 (3H, s), 1.01 (3H, s), 1.05-2.40 (16H, m), 2.90-3.13 (5H, m), 3.70-3.88 4.90-5.10 (1H, m).

### [Example 228]

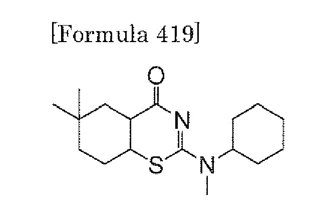

### Compound (I-228):

### Diastereomer (form B)

Yield: 25%
1H NMR(CDCl3) : δ(ppm) 0.96 (3H, s), 0.98 (3H, s), 1.15-2.10 (16H, m), 2.80 (1H, dt, J=12.5, 4Hz), 3.03 3.12 (3H, s), 3.79 (1H, s), 3.90-4.03 5.02-5.17 (1H, m).

### (Reference Example 21)

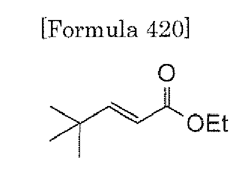

### Compound (T'-103):

To a mixture of 1.17 g (48.8 mmole) of sodium hydride and 30 ml of anhydrous tetrahydrofuran was added 9.37 g (41.8 mmole) of ethyl diethylphosphonoacetate under ice-cooling, and the mixture was stirred for 30 minutes. To the reaction solution was added a mixture of 3.00 g (34.8 mmole) of pivalaldehyde and 30 ml of anhydrous tetrahydrofuran, and the mixture was stirred at room temperature for 2 days. To the reaction solution was added saturated aqueous NH₄Cl, this was extracted with ethyl acetate, and the organic layer was washed with brine, dehydrated with anhydrous sodium sulfate, and filtered, and the solvent was concentrated under reduced pressure to give 5.58 g (100%) of a title compound as a brown liquid.
1H-NMR(CDCl3): δ(ppm) 1.08 (9H, s), 1.30 (3H, t, J=7.0Hz), 4.19 (2H, q, J=7.0Hz), 5.73 (1H, d, J=16.0Hz), 6.97 (1H, d, J=16.0Hz)

The following compounds were synthesized as in Reference Example 21.

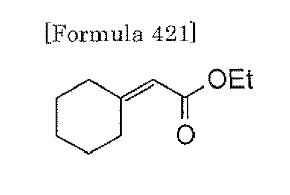

### Compound (I'-164):

Yield: 99%
1H-NMR(CDCl3): δ(ppm) 1.23-1.30 (3H, m), 1.53-1.68 (4H, m), 2.00-2.17 (4H, m), 2.80-2.93 (2H, m), 4.11-4.18 (2H, m), 5.54-5.60 (1H, m)

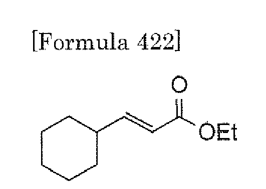

### Compound (I'-165):

Yield: 90%
1H-NMR(CDCl3): δ(ppm) 0.86-1.28 (7H, m), 1.48-1.79 (6H, m), 2.05-2.34 (3H, m), 4.05-4.16 (2H, m)

The following compounds were synthesized as in Reference Example 7.

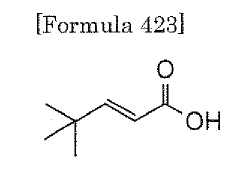

### Compound (I'-166):

Yield: 72%
1H-NMR(CDCl3): δ(ppm) 1.10 (9H, s), 5.75 (1H, d, J=15.5Hz), 7.09 (1H, d, J=15.5Hz), 10.09 (1H, br)

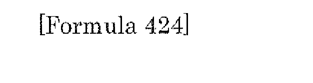

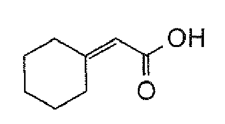

### Compound (I'-167):

Yield: 100%
1H-NMR(CDCl3): δ(ppm) 1.53-1.69 (5H, m), 2.01-2.05 (3H, m), 2.20-2.24, 2.80-2.84 (1H, m), 2.99 (1H, s), 5.60-5.63 (1H, m), 10.80 (1H, br)

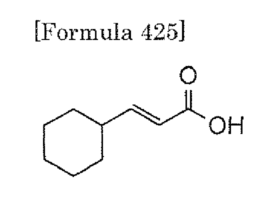

### Compound (I'-168):

Yield: 57%
1H-NMR(CDCl3): δ(ppm) 1.06-1.33 (6H, m), 1.54-1.79 (4H, m), 2.05-2.22 (1H, m), 5.76 (1H, d, J=15.0Hz), 7.03 (1H, dd, J=15.0, 6.0Hz), 12.01 (1H, br)

The following compounds were synthesized as in Reference Example 13.

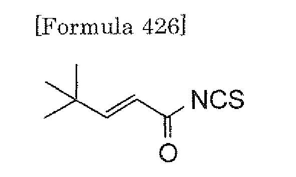

### Compound (I'-169):

Yield: 43%
1H-NMR(CDCl3): δ(ppm) 1.12 (9H, s), 5.84 (1H, d, J=15.5Hz), 7.00 (1H, d, J=15.5Hz)

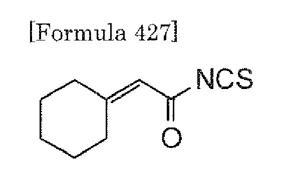

### Compound (I'-170):

Yield: 42%
1H-NMR(CDCl3): δ(pom) 1.55-1.82 (5H, m), 1.95-2.11 (3H, m), 2.23-2.27, 2.82-2.85 (1H, m), 3.22 (1H, s), 5.70-5.72 (1H, m)

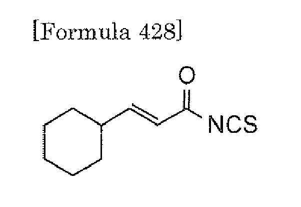

### Compound (I'-171):

Yield: 21%
1H-NMR(CDCl3): δ(ppm) 1.11-1.34 (6H, m), 1.68-1.84 (4H, m), 1.94-1.97 (1H, m), 5.88 (1H, d, J=15.0Hz), 7.06 (1H, dd, J=15.0, 6.0Hz)

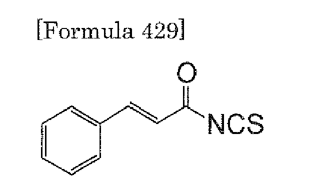

### Compound (I'-172)

Yield: 100%
1H-NMR(CDCl3): δ6.41 (1H, d, J=16.0Hz), 7.40-7.50 (3H, m), 7.54-7.60 (2H, m), 7.77 (1H, d, 16.OHz)

### [Example 229]

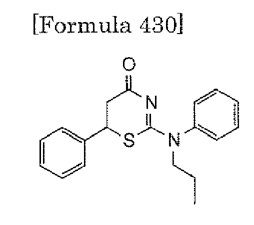

### Compound (I-229):

Yield: 75%
1H-NMR(CDCl3): δ(ppm) 0.92 (3H, t, J=7.5Hz), 1.64 (2H, q, J=7.5Hz), 2.80 (1H, dd, J=15.0, 13.OHz), 2.95 (1H, dd, J=15.0, 2.5Hz), 3.86-3.96 (1H, m), 4.05-4.16 (1H, m), 4.46 (1H, dd, J=13.5, 2.5Hz), 7.22-7.33 (7H, m), 7.40-7.46 (3H, m).

### [Example 230]

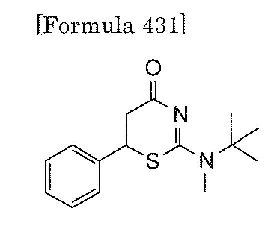

### Compound (I-230):

Yield: 12%
1H-NMR(CDCl3): δ(ppm) 1.60 (9H, s), 2.73 (1H, dd, J=15.0, 12.0Hz), 2.93 (1H, dd, J=15.0, 3.0Hz), 3.15 (3H, s), 4.50 (1H, dd, J=12.0, 3.0Hz), 7.30-7.42 (5H, m).

### [Example 231]

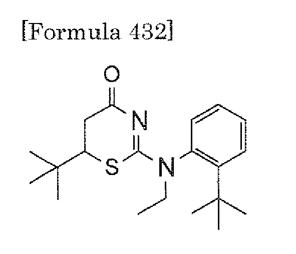

### Compound (I-231):

Yield: 100%
1H-NMR(CDCl3): δ(ppm) 0.91, 0.92 (9H, s), 1.25-1.28 (3H, m), 1.37, 1.39 (9H, s), 2.30-2.40 1H, m), 2.71-2.83 (1H, m), 3.01.-3.08 (1H, m), 3.13-3.26 (1H, m), 4.68-4.81 (1H, m), 7.00-7.05 (1H, m), 7.21-7.28 (1H, m), 7.38-7.44 (1H, m), 7.60 (1H, dd, J=8.0, 1.5Hz)

### [Example 232]

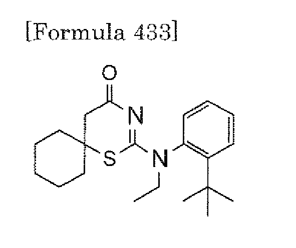

### Compound (I-232):

Yield: 31%
1H-NMR(CDCl3): δ(ppm) 0.88 (3H, t, J=7.0Hz), 1.22-1.73 (10H, m), 1.38 (9H, s), 2.53-2.65 (2H, m), 2.99-3.06 (1H, m), 4.74-4.81 (1H, m), 7.00 (1H, dd, J=7.0, 1.5Hz), 7.22-7.27 (1H, m), 7.38-7.45 (1H, m), 7.61 (1H, dd, J=7.0, 1.5Hz)

### [Example 233]

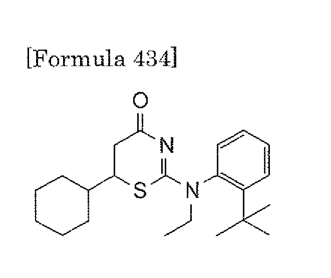

### Compound (I-233):

Yield: 47%
1H-NMR(CDCl3): δ(ppm) 0.94-1.28 (8H, m), 1.37, 1.39 (9H, s), 1.60-1.78 (6H, m), 2.35-2.59 (1H, m), 2.73 (1H, dt, J=15.0, 3.OHz), 3.00-3.21 (2H, m), 4.71-4.80 (1H, m), 7.02 (1H, ddd, J=8.0, 5.0, 1.5Hz), 7.22-7.27 (1H, m), 7.42 (1H, t, J=7.5Hz), 7.61 (1H, dd, J=8.0, 1.5Hz).

The following compound was obtained as in Example 214.

### [Example 234]

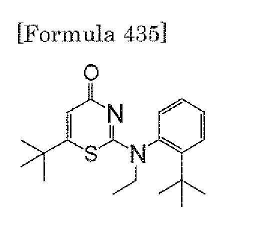

### Compound (I-234):

Yield: 100%
1H-NMR(CDCl3): δ1.18 (9H, s), 1.32 (3H, t, J=7.0Hz), 1.37 (9H, s), 3.16-3.28 (1H, m), 4.60-4.72 (1H, m), 6.41 (1H, s), 7.15 (1H, dd, J=8.0, 1.5Hz), 7.38 (1H, dt, J=8.0, 1.5Hz), 7.53 (1H, dt, J=8.0, 1.5Hz), 7.77 (1H, dd, J=8.0, 1.5Hz)

According to twenty-ninth step to thirty-seventh step described above, for example, the following compounds can be also synthesized.

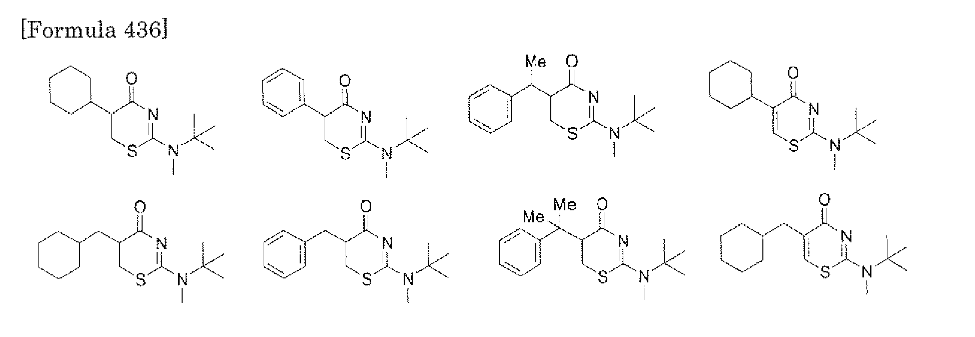

### (Test Example 1)

### Evaluation method of 11β-HSD1 inhibitor (Evaluation of compound against human 11β-HSD1)

After preincubating an inhibitor in a reaction solution consisting of 50 mM sodium phosphate buffer (pH 7.6), bovine serum albumin (1 mg/mL), NADPH (0.42 mg/mL), glucose-6-phosphate (1.26 mg/mL), glucose-6-phosphate dehydrogenase and an enzyme at room temperature for 30 minutes, cortisone (5 µM) which is a substrate was added (total amount 10 µL). After reacting at 37°C for 2 hours, an XL-665-labeled cortisol solution (5 µL), and a Cryptate-labeled anti-cortisol antibody solution (5 µL) were added, a reaction was performed at room temperature for 2 hours, and fluorescence intensity (HTRF method) was measured. A cortisol concentration was calculated from a standard curve prepared using a known concentration of cortisol for each assay.
Taking a concentration of cortisol generated in the absence of the inhibitor as a control value, 50% inhibitory concentration (IC₅₀ value) of the inhibitor against 11β-HSD1 was calculated from an inhibition curve plotting inhibition rate against the control value at each concentration of an inhibitor.

### (Test Example 2)

### Evaluation method of 11β-HSD1 inhibitor (Evaluation of compound against mouse 11β-HSD1)

After preincubating an inhibitor in a reaction solution consisting of 50 mM sodium phosphate buffer (pH 7.6), bovine serum albumin (1 mg/mL), NADPH (0.42 mg/mL), glucose-6-phosphate (1.26 mg/mL), glucose-6-phosphate dehydrogenase, and an enzyme at room temperature for 30 minutes, 11-dehydrocorticosterone (2 µM) which is a substrate was added (total amount 10 µL). After reacting at 37°C for 2 hours, an XL-665-labeled cortisol solution (5 µL), and a Cryptate-labeled anti-cortisol antibody solution (5 µL) were added, a reaction was performed at room temperature for 2 hours, and fluorescence intensity (HTRF method) was measured. A corticosterone concentration was calculated from a standard curve prepared using a known concentration of corticosterone for each assay.
Taking a concentration of corticosterone generated in the absence of the inhibitor as a control value, 50% inhibitory concentration (IC₅₀ value) of the inhibitor against 11β-HSD1 was calculated from an inhibition curve plotting inhibition rate against the control value at each concentration of the inhibitor.

Results of Test Examples 1 and 2 are shown below.
Compound (I-158): human IC₅₀ = 0.19 µM, mouse IC₅₀ = 1.49 µM
Compound (I-195): human IC₅₀ = 0.011 µM, mouse IC ₅₀ = 1.64 µM
Compound (I-175): human IC₅₀ = 0.032 µM, mouse IC ₅₀ = 0.82 µM

### (Test Example 3)

Material and method of oral absorption of 11β-HSDt inhibitor against diabetes
(1) Animal: male C57BL/6J Jcl mice at the age of 6 weeks were purchased from CLEA Japan, Inc., and used for the experiment at the age of 7 weeks after preliminary rearing for 1 week.
(2) Rearing conditions: Mice are fed in the following environment: temperature 23±2°C. humidity 55±10%, in a cycle of 8:00 to 20:00 in light and 20:00 to 8:00 in dark. During preliminary rearing, and a test term, the mice were allowed to liberally intake solid feed (CE-2, CLEA Japan, Inc.) and sterilized tap water.
(3) Identification of individual and cage: Individual number was written with oil ink on the tail of a mouse to achieve identification. A cage was attached with a label describing a name of a person in charge of the test, data of arrival, a strain, a sex, and a name of a supplier, and the mice were fed by 20 mice/cage during preliminary rearing. After start of the experiment, mice were fed in 3 mice/cage.
(4) Setting of dose and grouping: The following groups were set according to dose amounts of oral administration and intravenous administration.

| | |
|---|---|
| Oral administration | 20 mg/kg (n=3) |
| Intravenous administration | 5 mg/kg (n=3) |

(5) Preparation of dosing liquid: A preparation method is shown below. A suspension was prepared using 0.5% methylcellulose (1500cP) as a vehicle for oral administration. A solubilized solution was prepared using N,N-dimethylacetamide/polyethylene glycol 400(=1/2) as a vehicle for intravenous administration.
(6) Administration method: As to oral administration, the dosing suspension was administered compulsorily into the stomach by means of an oral sonde at a rate of 10 mL/kg. As to intravenous administration, the dosing solution was administered into caudal vein at a rate of 2.5 mL/kg by means of a glass syringe.
(7) End point: Blood was collected from heart by time-point blood sampling, and a drug concentration in plasma was measured by using HPLC or LC/MS/MS.
(8) Statistical analysis: As to a plasma concentration profile, an area under a plasma concentration-time curve (AUC) was calculated by using non-linear minimum program WinNonlin (registered trade name), and bioavailability was calculated from AUC values of the oral administration group and the intravenous administration group.

### Formulation Examples

The following Formulation Examples 1 to 8 are merely examples, and are not intended to limit the scope of the present invention. The term "active ingredient" means the present compound, its tautomer, prodrug thereof, pharmaceutically acceptable salt thereof, or hydrate thereof.

### (Formulation Example 1)

A hard gelatin capsule is prepared by using the following ingredients:

| | Dose |
|---|---|
| | (mg/capsule) |
| Active ingredient | 250 |
| Starch (dry) | 200 |
| Magnesium stearate | 10 |
| Total | 460 mg |

### (Formulation Example 2)

A tablet is prepared by using the following ingredients:

| | Dose |
|---|---|
| | (mg/tablet) |
| Active ingredient | 250 |
| Cellulose (microcrystal) | 400 |
| Silicon dioxide (fumed) | 10 |
| Stearic acid | 5 |
| Total | 665 mg |

The ingredients are mixed, and compressed to form tables each weighing 665 mg.

### (Formulation Example 3)

An aerosol solution containing the following ingredients is prepared:

| | Weight |
|---|---|
| Active ingredient | 0.25 |
| Ethanol | 25.75 |
| Propellant 22 (chlorodifluoromethane) | 74.00 |
| Total | 100.00 |

The active ingredient and ethanol are mixed, and the mixture is added to part of propellant 22, cooled to -30°C, and transferred to a packing machine. Then, a necessary amount is supplied to a stainless steel container, and diluted with the remaining propellant. A bubble unit is attached to the container.

### (Formulation Example 4)

A tablet containing 60 mg of the active ingredient is prepared in the following manner:

| | |
|---|---|
| Active ingredient | 60 mg |
| Starch | 45 mg |
| Microcrystalline cellulose | 35 mg |
| Polyvinylpyrrolidone (10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No.45 mesh U.S. sieve and mixed thoroughly. An aqueous solution containing polyvinylpyrrolidone is mixed with obtained powder and then the mixture is passed through a No. 4 mesh U.S. sieve. Granules obtained in this manner are dried at 50°C and passed through a No.18 mesh U.S. sieve. The sodium carboxymethyl starch, magnesium stearate and talc that are passed through a No.60 mesh U.S. sieve in advance, are added to the granules, mixed, and then compressed by a tableting machine to obtain tablets each weighing 150 mg.

### (Formulation Example 5)

A capsule containing 80 mg of the active ingredient is prepared in the following manner:

| | |
|---|---|
| Active ingredient | 80 mg |
| Starch | 59 mg |
| Microcrystalline cellulose | 59 mg |
| Magnesium stearate | 2 mg |
| Total | 200 mg |

The active ingredient, starch, cellulose, and magnesium stearate are mixed, and passed through a No.45 mesh U.S. sieve, and filled into a hard gelatin capsule in 200 mg quantities.

### (Formulation Example 6)

Suppository containing 225 mg of the active ingredient is prepared in the following manner:

| | |
|---|---|
| Active ingredient | 225 mg |
| Saturated fatty acid glyceride | 2000 mg |
| Total | 2225 mg |

The active ingredient is passed through a No.60 mesh U.S. sieve, and suspended in saturated fatty acid glyceride that is melted by heating least necessarily in advance. Then, the resultant mixture is put into an apparent 2g mold, and cooled.

### (Formulation Example 7)

A suspension containing 50 mg of the active ingredient is prepared in the following manner:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Pigment | q.v. |
| Purified water to total | 5 mL |

The active ingredient is passed through a No.45 mesh U.S. sieve, and mixed with sodium carboxymethyl cellulose and syrup to form a smooth paste. The benzoic acid solution and the flavor diluted with part of water are added, and stirred. Then a sufficient amount of water is added to achieve required volume.

### (Formulation Example 8)

An intravenous formulation is prepared in the following manner:

| | |
|---|---|
| Active ingredient | 100 mg |
| Saturated fatty acid glyceride | 1000 mL |

The solution of the above ingredients is intravenously administered to a patient usually at a speed of 1 mL per minute.

### [Industrial Applicability]

As is apparent from the above test examples, the compounds according to the present invention show inhibitory activity on 11β-hydroxysteroid dehydrogenase type 1. Therefore, the compounds according to the present invention are very useful as therapeutic agents for diabetes.

## Claims

1. A pharmaceutical composition having inhibitory activity on 11β-hydroxysteroid dehydrogenase type I comprising a compound represented by the formula (I): 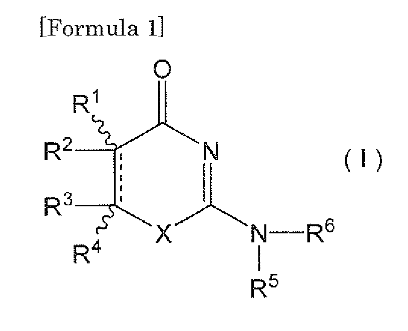 its pharmaceutically acceptable salt, or a solvate thereof,
wherein
X is O or S,
a broken line and a wavy line represent the presence or the absence of a bond,
(i) when a broken line represents the presence of a bond, a wavy line represents the absence of a bond,
R² and R³ are each independently hydrogen, halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R² and R³ may be taken together with the adjacent carbon atom to which they are attached to form an optionally substituted nonaromatic hydrocarbon ring or an optionally substituted aromatic hydrocarbon ring
(ii) when a broken line represents the absence of a bond, a wavy line represents the presence of a bond,
R¹ and R⁴ are each independently hydrogen, halogen, hydroxy, optionally substituted alkyloxy, optionally substituted alkyl, optionally substituted cycloalkyl or optionally substituted aryl,
R² and R³ are each independently hydrogen, halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or
R¹ and R², R² and R³ or R³ and R⁴ may be taken together with the adjacent carbon atom to which they are attached to form an optionally substituted nonaromatic hydrocarbon ring,
R⁵ and R⁶ are each independently hydrogen, optionally substituted alkyl, optionally substituted alkenyl, optionally substituted alkynyl, optionally substituted aryl, optionally substituted arylalkyl, optionally substituted cycloalkyl, optionally substituted cycloalkylalkyl, optionally substituted cycloalkenyl, optionally substituted cycloalkenylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkylsulfonyl or optionally substituted arylsulfonyl, or R⁵ and R⁶ may be taken together with the adjacent nitrogen atom to which they are attached to form an optionally substituted ring.

2. The pharmaceutical composition having inhibitory activity on 11β-hydroxysteroid dehydrogenase type I comprising the compound according to claim 1, its pharmaceutically acceptable salt, or a solvate thereof, wherein
(i) a broken line represents the presence of a bond, a wavy line represents the absence of a bond, and R² and R³ are taken together with the adjacent carbon atom to which they are attached to form an optionally substituted nonaromatic hydrocarbon ring or an optionally substituted aromatic hydrocarbon ring, or
(ii) a broken line represents the absence of a bond, a wavy line represents the presence of a bond, and R² and R³ are taken together with the adjacent carbon atom to which they are attached to form an optionally substituted nonaromatic hydrocarbon ring.

3. The pharmaceutical composition having inhibitory activity on 11β-hydroxysteroid dehydrogenase type I comprising the compound according to claim 2, its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted nonaromatic hydrocarbon ring.

4. The pharmaceutical composition having inhibitory activity on 11β-hydroxysteroid dehydrogenase type I comprising the compound according to claim 2, its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted aromatic hydrocarbon ring.

5. The pharmaceutical composition having inhibitory activity on 11β-hydroxysteroid dehydrogenase type I comprising the compound according to claim 2, its pharmaceutically acceptable salt, or a solvate thereof, wherein a broken line represents the absence of a bond, a wavy line represents the presence of a bond, and R² and R³ are taken together with the adjacent carbon atom to which they are attached to form an optionally substituted nonaromatic hydrocarbon ring.

6. The pharmaceutical composition having inhibitory activity on 11β-hydroxysteroid dehydrogenase type I comprising the compound according to any one of claims 2 to 5, its pharmaceutically acceptable salt, or a solvate thereof, wherein X is S.

7. The pharmaceutical composition having inhibitory activity on 11β-hydroxysteroid dehydrogenase type I comprising the compound according to claim 1, its pharmaceutically acceptable salt, or a solvate thereof, wherein a broken line represents the absence of a bond, a wavy line represents the presence of a bond, R¹ and R² are each hydrogen, R³ and R⁴ are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R³ and R⁴ may be taken together with the adjacent carbon atom to which they are attached to form an optionally substituted nonaromatic hydrocarbon ring, or
a broken line represents the absence of a bond, a wavy line represents the presence of a bond, R³ and R⁴ are each hydrogen, R¹ and R² are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R¹ and R² may be taken together with the adjacent carbon atom to which they are attached to form an optionally substituted nonaromatic hydrocarbon ring.

8. The pharmaceutical composition having inhibitory activity on 11β-hydroxysteroid dehydrogenase type I comprising the compound according to claim 7, its pharmaceutically acceptable salt, or a solvate thereof, wherein a broken line represents the absence of a bond, a wavy line represents the presence of a bond, R¹ and R² are each hydrogen, R³ and R⁴ are each independently halogen, cyano, hydroxy, carboxy, optionally substituted alkyl, optionally substituted alkenyl, optionally substituted alkynyl, optionally substituted cycloalkyl, optionally substituted cycloalkylalkyl, optionally substituted cycloalkenyl, optionally substituted aryl, optionally substituted arylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkyloxy, optionally substituted alkyloxycarbonyl, optionally substituted alkylthio, optionally substituted arylthio, optionally substituted alkylsulfonyl or optionally substituted arylsulfonyl, or R³ and R⁴ may be taken together with the adjacent carbon atom to which they are attached to form an optionally substituted nonaromatic hydrocarbon ring.

9. A compound represented by the formula (I): 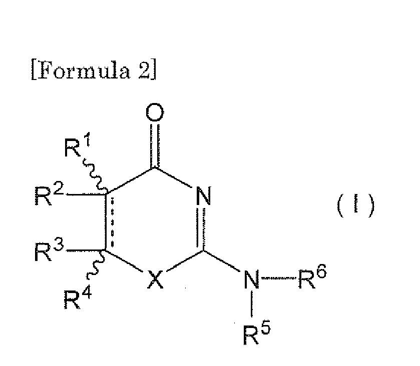 , its pharmaceutically acceptable salt, or a solvate thereof,
wherein
X is O or S,
a broken line and a wavy line represent the presence or the absence of a bond,
(i) when a broken line represents the presence of a bond, a wavy line represents the absence of a bond,
R² and R³ are taken together with the adjacent carbon atom to which they are attached to form an optionally substituted nonaromatic hydrocarbon ring,
(ii) when a broken line represents the absence of a bond, a wavy line represents the presence of a bond,
R¹ and R⁴ are each independently hydrogen, halogen, hydroxy, optionally substituted alkyloxy or optionally substituted alkyl, R² and R³ are taken together with the adjacent carbon atom to which they are attached to form an optionally substituted nonaromatic hydrocarbon ring,
R⁵ and R⁶ are each independently hydrogen, optionally substituted alkyl, optionally substituted alkenyl, optionally substituted alkynyl, optionally substituted aryl, optionally substituted arylalkyl, optionally substituted cycloalkyl, optionally substituted cycloalkylalkyl, optionally substituted cycloalkenyl, optionally substituted cycloalkenylalkyl, optionally substituted heteroaryl, optionally substituted heteroarylalkyl, optionally substituted heterocycle, optionally substituted heterocyclealkyl, optionally substituted amino, optionally substituted acyl, optionally substituted carbamoyl, optionally substituted alkylsulfonyl or optionally substituted arylsulfonyl, or R⁵ and R⁶ may be taken together with the adjacent nitrogen atom to which they are attached to form an optionally substituted ring,
provided that, the following compounds are excluded:

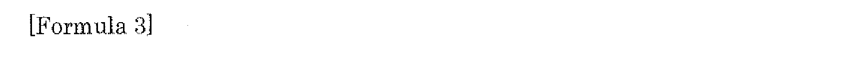

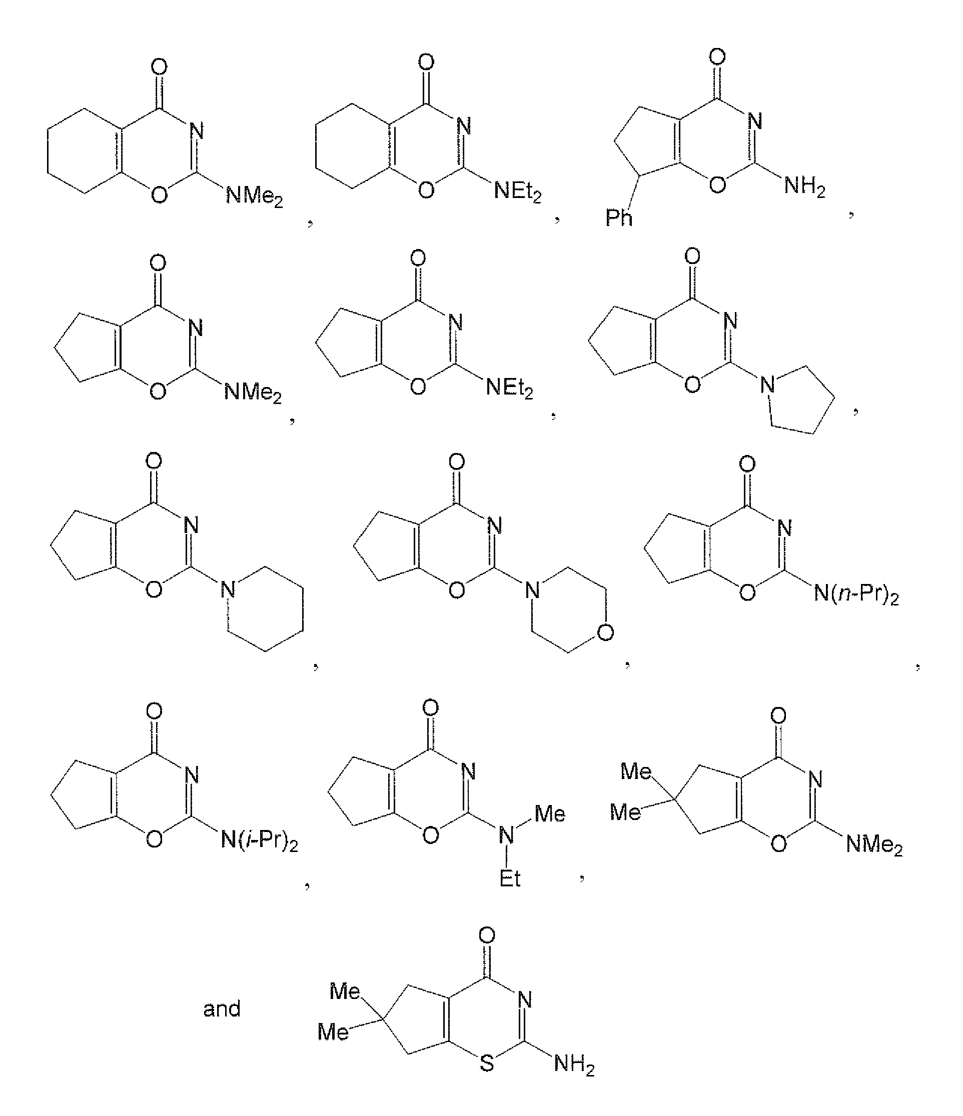

10. The compound according to claim 9, its pharmaceutically acceptable salt, or a solvate thereof, wherein a broken line represents the presence of a bond, a wavy line represents the absence of a bond, and R² and R³ are taken together with the adjacent carbon atom to which they are attached to form an optionally substituted nonaromatic hydrocarbon ring.

11. The compound according to claim 9, its pharmaceutically acceptable salt, or a solvate thereof, wherein a broken line represents the absence of a bond, a wavy line represents the presence of a bond, and R² and R³ are taken together with the adjacent carbon atom to which they are attached to form an optionally substituted nonaromatic hydrocarbon ring.

12. The compound according to claim 9 or 11, its pharmaceutically acceptable salt, or a solvate thereof, wherein R¹ and R⁴ are each hydrogen.

13. The compound according to any one of claims 9 to 12, its pharmaceutically acceptable salt, or a solvate thereof, wherein X is S.

14. The compound according to any one of claims 9 to 13, its pharmaceutically acceptable salt, or a solvate thereof, wherein R⁵ and R⁶ are each independently optionally substituted alkyl, optionally substituted aryl, optionally substituted cycloaLkyl, optionally substituted heterocycle, optionally substituted amino, or R⁵ and R⁶ may be taken together with the adjacent nitrogen atom to which they are attached to form an optionally substituted ring.

15. A pharmaceutical composition comprising the compound according to any one of claims 9 to 14, its pharmaceutically acceptable salt, or a solvate thereof.

16. The pharmaceutical composition according to any one of claims 1 to 8, or 15, for treatment and/or prevention of diabetes.

17. A method for preventing or treating diabetes, comprising administering the compound according to any one of claims 1 to 16, its pharmaceutically acceptable salt, or a solvate thereof.

18. A use of the compound according to any one of claims 1 to 16, its pharmaceutically acceptable salt, or a solvate thereof for manufacturing a medicament of treatment and/or prevention of diabetes.
